# EUROPEAN PATENT APPLICATION

(11) **EP 4 166 555 A1**
(43) Date of publication of application: **19.04.2023**
(21) Application number: 21822473.1
(22) Date of filing: 11.06.2021
(51) Int. Cl.: C07D 471/04, A61K 31/519, A61P 35/00

(54) **PYRIDINE-PYRIMIDINE DERIVATIVE, PREPARATION METHOD THEREFOR AND PHARMACEUTICAL USE THEREOF**

(30) Priority: 11.06.2020 CN 202010529071; 28.09.2020 CN 202011042186; 02.12.2020 CN 202011400233; 11.05.2021 CN 202110509569
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); FENG, Binqiang, Shanghai 200245 (CN); BAI, Dongdong, Shanghai 200245 (CN); HE, Feng, Shanghai 200245 (CN); TAO, Weikang, Shanghai 200245 (CN)
(74) Representative: Viganò, Elena
(86) International application number: PCT/CN2021/099552
(87) International publication number: WO 2021/249519

(57) **Abstract**

A pyridine-pyrimidine derivative, a preparation method therefor and a pharmaceutical use thereof. In particular, the present disclosure relates to pyridine-pyrimidine derivative as shown in general formula (I), a preparation method therefor, a pharmaceutical composition containing the derivative, and a use thereof as a therapeutic agent, especially a use thereof as an SOS1 inhibitor and a use thereof in preparation of drugs for treating diseases, conditions or disorders improved by inhibiting SOS1.

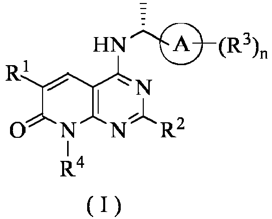

## Description

### TECHNICAL FIELD

The present disclosure belongs to the field of pharmaceutics, and relates to a pyridone-pyrimidine derivative of general formula (I), a preparation method therefor, a pharmaceutical composition containing the derivative, and use thereof as a therapeutic agent, in particular use thereof as an SOS1 inhibitor and use thereof in preparing a medicament for the treatment of a disease, condition or disorder improved by inhibiting SOS1.

### BACKGROUND

RAS is one of the oncogenes with the highest mutation rate in tumors, and about 30% of human malignancies are associated with mutations of the RAS gene. The RAS family includes KRAS, NRAS and HRAS, and KRAS mutations are the most common and account for approximately 85%. After being activated, KRAS regulates multiple functions such as cell proliferation, survival, migration and metabolism through a plurality of downstream signaling pathways represented by RAF-MEK-ERK, PI3K-AKT-mTOR and TIAM1-RAc. After mutation of KRAS gene, the protein is continuously activated, resulting in continuous activation of downstream signaling pathways and thereby promoting tumorigenesis.

KRAS protein has long been considered as an undruggable drug target because it lacks conventional small molecule binding sites on its surface and it is extremely difficult to inhibit due to its ultrahigh affinity for guanylic acid. However, based on the importance and prevalence of abnormal KRAS activation in cancer progression, KRAS has been and remains a target of high interest for drug development. The current drug development concept for inhibiting KRAS pathway mainly includes the following aspects.

The small molecule covalent inhibitor developed for KRAS G12C can irreversibly lock the G12C mutant in an inactive state. To date, clinical phase I data from Amgen and Mirati have shown promising results. However, the KRAS G12C mutation is only one of many KRAS mutations, and there still lack effective drugs for other important mutants such as G12V, G12D, G12S, G12A and G13V/D.

Search for other sites on KRAS that can target more mutants: mainly for sites binding to downstream effector molecules/sites associated with activation of protein molecules, all of which are currently under research in the preclinical stage, with IC₅₀ for activity inhibition commonly at the micromolar level;
inhibition on signaling proteins downstream of KRAS: for example, development of inhibitors against RAF, MEK, ERK, etc., which do not work well when used alone clinically at present;
inhibition on pathways upstream of KRAS: such as inhibitors against SHP2; modification and localization to KRAS: such as farnesyl transferase, which blocks the membrane localization of KRAS to achieve the effect of inhibiting its action; and knock-down of the expression of KRAS by the method of RNAi.

In general, there is currently a lack of broad-spectrum KRAS inhibitors that are effective against a variety of mutations, in addition to KRAS G12C inhibitors. In contrast, blocking the binding of activating molecules of KRAS to KRAS, such as small molecule inhibitors that selectively inhibit SOS1, i.e., guanine nucleotide exchange factor (GEF), can block the activation of KRAS by interfering with the RAS-SOS1 interaction, thereby achieving broad-spectrum inhibition of KRAS activity.

The KARS protein is a small GTPase that transforms intracellularly between an inactivated state (binding to guanosine diphosphate (GDP)) and an activated state (binding to guanosine triphosphate (GTP)). This transformation is regulated by guanine nucleotide exchange factors (GEFs) and GTPase activatoing proteins (GAPs). There are three major groups of GEFs for KRAS, namely SOS (sevenless son) 1&2, Ras-GRF and Ras-GRP, of which the latter two are expressed only in neurons and leukocytes, while only SOS is widely expressed in various tissues and is thought to play a dominant role in the activation of RAS. Since SOS1 is more highly expressed than SOS2 and more active than SOS2, current research on SOS is mainly focused on SOS1. The specific activation pathways of SOS1 for the KRAS protein are as follows: after activation of membrane surface receptors by upstream signals (such as growth factors), SOS1 is activated through SHP2-Grb2, binds to KRAS, and catalyzes the dissociation of KRAS and GDP by causing a series of conformational changes, and then KRAS binds to GTP to form active KRAS-GTP.

The existing published patents related to SOS1 include WO2018172250A1, WO2019055540A1, WO2019122129A1, US10501421B1, WO2018115380A1 and WO2019201848A1.

### SUMMARY

The present disclosure is intended to provide a compound of general formula (I) or a tautomer, mesomer, racemate, enantiomer or diastereomer thereof or a mixture thereof, or a pharmaceutically acceptable salt thereof, 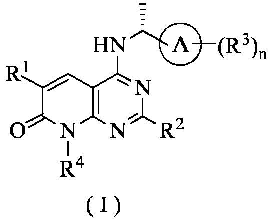 wherein:
ring A is aryl or heteroaryl;
R¹ is selected from the group consisting of hydrogen, alkyl, alkoxy, haloalkyl, haloalkoxy, hydroxyalkyl, cycloalkyloxy, heterocyclyloxy, alkenyl, alkynyl, hydroxy, cyano, amino, -NR⁵R⁶, nitro, cycloalkyl, heterocyclyl, aryloxy, heteroaryloxy, aryl and
heteroaryl, wherein the alkyl, alkoxy, cycloalkyloxy, heterocyclyloxy, cycloalkyl, heterocyclyl, aryloxy, heteroaryloxy, aryl and heteroaryl are each independently and optionally substituted with one or more substituents selected from the group consisting of halogen, alkyl, alkoxy, haloalkoxy, hydroxy, amino, oxo, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
R² is selected from the group consisting of hydrogen, halogen, alkyl, haloalkyl, alkoxy, haloalkoxy, hydroxyalkyl, cyano, amino and cycloalkyl;
R³ are identical or different and are each independently selected from the group consisting of hydrogen, halogen, alkyl, alkoxy, haloalkyl, haloalkoxy, hydroxyalkyl, alkenyl, alkynyl, hydroxy, cyano, amino, -(CH₂)ᵣNR⁵R⁶, cycloalkyl, heterocyclyl, aryl and heteroaryl, wherein the alkyl, haloalkyl, cycloalkyl, heterocyclyl, aryl and heteroaryl are each independently and optionally substituted with one or more substituents selected from the group consisting of halogen, alkyl, alkoxy, haloalkyl, haloalkoxy, hydroxy, nitro, amino, -(CH₂)ₛNR⁹R¹⁰, cyano, cycloalkyl, heterocyclyl, aryl and heteroaryl;
R⁴ is selected from the group consisting of hydrogen, alkyl and cycloalkyl, wherein the alkyl and cycloalkyl are each independently and optionally substituted with one or more substituents selected from the group consisting of halogen, alkyl, alkoxy, haloalkyl, haloalkoxy, hydroxy, nitro, amino, cyano, cycloalkyl, heterocyclyl, aryl and heteroaryl; R⁵ and R⁶ are identical or different and are each independently selected from the group consisting of hydrogen, alkyl, haloalkyl, hydroxyalkyl, hydroxy, amino, cycloalkyl and heterocyclyl;
R⁷ is selected from the group consisting of hydrogen, alkyl, haloalkyl, hydroxyalkyl, cycloalkyl and heterocyclyl;
R⁸ are identical or different and are each independently selected from the group consisting of hydrogen, alkyl, haloalkyl, hydroxyalkyl, hydroxy, amino, cycloalkyl and heterocyclyl, wherein the alkyl, haloalkyl, cycloalkyl and heterocyclyl are each independently and optionally substituted with one or more substituents selected from the group consisting of halogen, alkyl, alkoxy, haloalkyl, haloalkoxy, hydroxy, nitro, amino, cycano, cycloalkyl, heterocyclyl, aryl and heteroaryl;
R⁹ and R¹⁰ are identical or different and are each independently selected from the group consisting of hydrogen, alkyl, haloalkyl, hydroxyalkyl, hydroxy, amino, cycloalkyl and heterocyclyl;
n is 1, 2, 3 or 4;
p is 0, 1, 2 or 3;
q is 0, 1, 2 or 3;
r is 0, 1, 2 or 3; and
s is 0, 1, 2 or 3.

The present disclosure is intended to provide a compound of general formula (I) or a tautomer, mesomer, racemate, enantiomer or diastereomer thereof or a mixture thereof, or a pharmaceutically acceptable salt thereof: 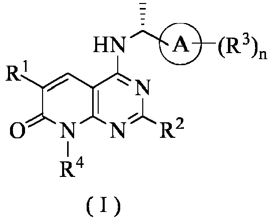 wherein:
ring A is aryl or heteroaryl;
R¹ is selected from the group consisting of hydrogen, alkyl, alkoxy, haloalkyl, haloalkoxy, hydroxyalkyl, cycloalkyloxy, heterocyclyloxy, alkenyl, alkynyl, hydroxy, cyano, amino, -NR⁵R⁶, nitro, cycloalkyl, heterocyclyl, aryl and heteroaryl, wherein the alkyl, alkoxy, cycloalkyloxy, heterocyclyloxy, cycloalkyl, heterocyclyl, aryl and
heteroaryl are each independently and optionally substituted with one or more substituents selected from the group consisting of halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
R² is selected from the group consisting of hydrogen, halogen, alkyl, haloalkyl, alkoxy, haloalkoxy, hydroxyalkyl, cyano, amino and cycloalkyl;
R³ are identical or different and are each independently selected from the group consisting of hydrogen, halogen, alkyl, alkoxy, haloalkyl, haloalkoxy, hydroxyalkyl, alkenyl, alkynyl, hydroxy, cyano, amino, -(CH₂)ᵣNR⁵R⁶, cycloalkyl, heterocyclyl, aryl and heteroaryl, wherein the alkyl, haloalkyl, cycloalkyl, heterocyclyl, aryl and heteroaryl are each independently and optionally substituted with one or more substituents selected from the group consisting of halogen, alkyl, alkoxy, haloalkyl, haloalkoxy, hydroxy, nitro, amino, -(CH₂)ₛNR⁹R¹⁰, cyano, cycloalkyl, heterocyclyl, aryl and heteroaryl;
R⁴ is selected from the group consisting of hydrogen, alkyl and cycloalkyl;
R⁵ and R⁶ are identical or different and are each independently selected from the group consisting of hydrogen, alkyl, haloalkyl, hydroxyalkyl, hydroxy, amino, cycloalkyl and heterocyclyl;
R⁷ is selected from the group consisting of hydrogen, alkyl, haloalkyl, hydroxyalkyl, cycloalkyl and heterocyclyl;
R⁸ is selected from the group consisting of hydrogen, alkyl, haloalkyl, hydroxyalkyl, hydroxy, amino, cycloalkyl and heterocyclyl;
R⁹ and R¹⁰ are identical or different and are each independently selected from the group consisting of hydrogen, alkyl, haloalkyl, hydroxyalkyl, hydroxy, amino, cycloalkyl and heterocyclyl;
n is 1, 2, 3 or 4;
p is 0, 1, 2 or 3;
q is 0, 1, 2 or 3;
r is 0, 1, 2 or 3; and
s is 0, 1, 2 or 3.

In some preferred embodiments of the present disclosure, provided is a compound of general formula (I) or a tautomer, mesomer, racemate, enantiomer or diastereomer thereof or a mixture thereof, or a pharmaceutically acceptable salt thereof, wherein ring A is aryl or heteroaryl, wherein preferably, the aryl or heteroaryl is optionally fused to cycloalkyl or heterocyclyl.

In some preferred embodiments of the present disclosure, provided is a compound of general formula (I) or a tautomer, mesomer, racemate, enantiomer or diastereomer thereof or a mixture thereof, or a pharmaceutically acceptable salt thereof, wherein ring A is 6- to 10-membered aryl, preferably phenyl or

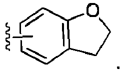

In some preferred embodiments of the present disclosure, provided is a compound of general formula (I) or a tautomer, mesomer, racemate, enantiomer or diastereomer thereof or a mixture thereof, or a pharmaceutically acceptable salt thereof, wherein ring A is phenyl.

In some preferred embodiments of the present disclosure, provided is a compound of general formula (I) or a tautomer, mesomer, racemate, enantiomer or diastereomer thereof or a mixture thereof, or a pharmaceutically acceptable salt thereof, which is a compound of formula (II) or a tautomer, mesomer, racemate, enantiomer or diastereomer thereof or a mixture thereof, or a pharmaceutically acceptable salt thereof, 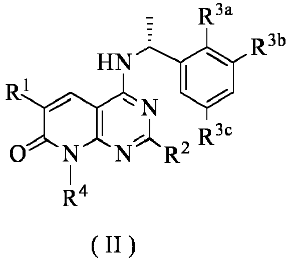 wherein:
R^{3a}, R^{3b} and R^{3c} are identical or different and are each independently selected from the group consisting of hydrogen, halogen, alkyl, alkoxy, haloalkyl, haloalkoxy, hydroxyalkyl, alkenyl, alkynyl, hydroxy, cyano, amino, -(CH₂)ᵣNR⁵R⁶, cycloalkyl, heterocyclyl, aryl and heteroaryl, wherein the alkyl, haloalkyl, cycloalkyl, heterocyclyl, aryl and heteroaryl are each independently and optionally substituted with one or more substituents selected from the group consisting of halogen, alkyl, alkoxy, haloalkyl, haloalkoxy, hydroxy, nitro, amino, -(CH₂)ₛNR⁹R¹⁰, cyano, cycloalkyl, heterocyclyl, aryl and heteroaryl; preferably, R^{3a}, R^{3b} and R^{3c} are identical or different and are each independently selected from the group consisting of hydrogen, halogen, C₁₋₆ alkyl, C₁₋₆ haloalkyl, hydroxy, cyano and amino, wherein the C₁₋₆ alkyl and C₁₋₆haloalkyl are optionally substituted with one or more substituents selected from the group consisting of halogen, hydroxy and C₁₋₆ alkoxy;
R¹, R², R⁴, R⁵, R⁶, R⁹, R¹⁰, s and r are as defined in the compound of general formula (I).

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R⁴ is hydrogen or C₁₋₆ alkyl; preferably, R⁴ is hydrogen. In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R¹ is selected from the group consisting of C₁₋₆ alkyl, C₁₋₆ alkoxy, C₁₋₆ haloalkyl, C₁₋₆ haloalkoxy, C₁₋₆hydroxyalkyl, 3- to 10-membered cycloalkyloxy, 3- to 10-membered heterocyclyloxy, 3- to 10-membered cycloalkyl, 3-to 10-membered heterocyclyl, 6- to 10-membered aryl, and 5- to 10-membered heteroaryl, wherein the C₁₋₆ alkyl, C₁₋₆alkoxy, 3- to 10-membered cycloalkyloxy, 3- to 10-membered heterocyclyloxy, 3- to 10-membered cycloalkyl, 3- to 10-membered heterocyclyl, 6- to 10-membered aryl, and 5- to 10-membered heteroaryl are optionally substituted with one or more substituents selected from the group consisting of halogen, C₁₋₆ alkyl, C₁₋₆ alkoxy, C₁₋₆haloalkoxy, hydroxy, amino, cyano, -C(O)(CH₂)_{q}OR⁷, -NHC(O)R⁸, -C(O)R⁸, -NR⁹R¹⁰, and -C(O)(CH₂)ₚNR⁹R¹⁰; R⁷ to R¹⁰, q and p are as defined in the compound of formula (I).

In some preferred embodiments of the present disclosure, provided is a compound of general formula (I) or general formula (II) or a tautomer, mesomer, racemate, enantiomer or diastereomer thereof or a mixture thereof, or a pharmaceutically
acceptable salt thereof, wherein R¹ is 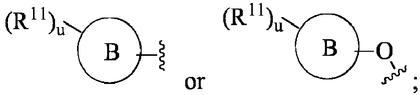 ring B is selected from the group consisting of cycloalkyl, heterocyclyl, aryl, and heteroaryl;
preferably, R¹ is selected from the group consisting of 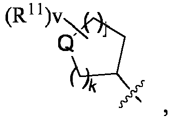 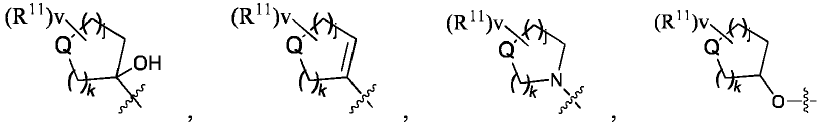 and 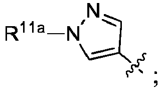 Q is selected from the group consisting of oxygen, sulfur, 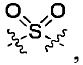 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
u is 0, 1, 2, 3, 4 or 5;
v is 0, 1, 2 or 3;
R⁷ to R¹⁰, p and q are as defined in general formula (I).

In some preferred embodiments of the present disclosure, a compound of general formula (I) or general formula (II) or a tautomer, mesomer, racemate, enantiomer or diastereomer thereof or a mixture thereof, or a pharmaceutically acceptable salt thereof, wherein R¹ is selected from the group consisting of C₁₋₆ alkoxy, 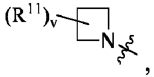 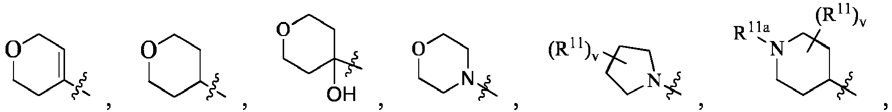 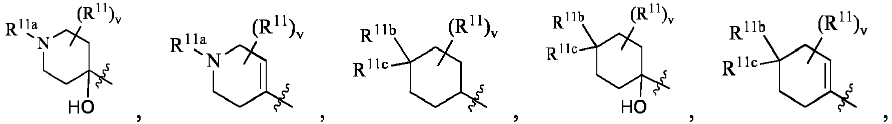 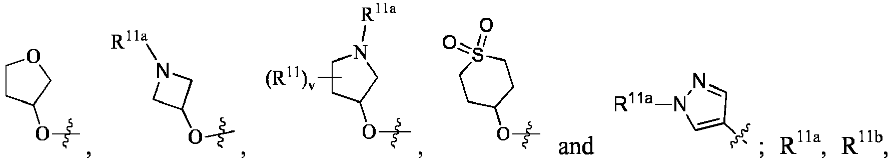
R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
v is 0, 1, 2 or 3;
R⁷ to R¹⁰, p and q are as defined in general formula (I).

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R¹ is selected from the group consisting of C₁₋₆ alkoxy, 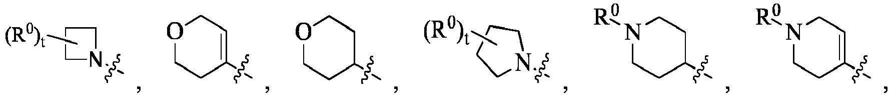 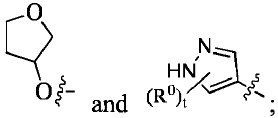 R⁰ are identical or different and are each independently selected from the group consisting of halogen, C₁₋₆alkyl, C₁₋₆alkoxy, C₁₋₆haloalkoxy, hydroxy, amino, cyano, -C(O)(CH₂)_{q}OR⁷, -NHC(O)R⁸, -C(O)R⁸, -NR⁹R¹⁰ and -C(O)(CH₂)ₚNR⁹R¹⁰; t is 0, 1, 2, 3 or 4; and R⁷ to R¹⁰, q and p are as defined in the compound of formula (I).

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R² is C₁₋₆alkyl, preferably, methyl.

In some preferred embodiments of the present disclosure, provided is a compound of general formula (I) or a tautomer, mesomer, racemate, enantiomer or diastereomer thereof or a mixture thereof, or a pharmaceutically acceptable salt thereof, wherein R³ are identical or different and are each independently selected from the group consisting of hydrogen, halogen, C₁₋₆ alkyl, C₁₋₆haloalkyl, hydroxy, cyano and amino, wherein the C₁₋₆alkyl and C₁₋₆haloalkyl are optionally substituted with one or more substituents selected from the group consisting of halogen, hydroxy and C₁₋₆alkoxy.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein n is 1, 2 or 3; preferably, n is 2.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R⁷ is selected from the group consisting of hydrogen, alkyl and haloalkyl; preferably, R⁷ is selected from the group consisting of C₁₋₆ alkyl and C₁₋₆ haloalkyl.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R⁸ are identical or different and are each independently selected from C₁₋₆ alkyl; wherein the C₁₋₆ alkyl is optionally substituted with one or more substituents selected from the group consisting of halogen and cyano.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein R⁹ and R¹⁰ are identical or different and are each independently selected from the group consisting of hydrogen, C₁₋₆ alkyl and C₁₋₆ haloalkyl.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein p is 0.

In some preferred embodiments of the present disclosure, provided is a compound of general formula (I) or general formula (II) or a tautomer, mesomer, racemate, enantiomer or diastereomer thereof or a mixture thereof, or a pharmaceutically acceptable salt thereof, wherein q is 1.

**Table A. Typical compounds of general formula (I) the present disclosure include, but are not limited to:**

| Example No. | Structure and name of compound |
|---|---|
| 1 | 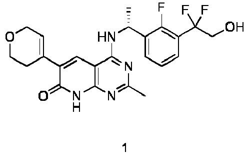 |
| | (*R*)-4-((1-(3-(1,1-difluoro-2-hydroxyethyl)-2-fluorophenyl)ethyl)amino) -6-(3,6-dihydro-2*H*-pyran-4-yl)-2-methylpyrido[2,3-*d*]pyrimidin-7(8*H*)-one 1 |
| 2 | 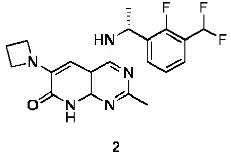 |
| | (*R*)-6-(azetidin-1-yl)-4-((1-(3-(difluoromethyl)-2-fluorophenyl)ethyl)am ino)-2-methylpyrido[2,3-*d*]pyrimidin-7(8*H*)-one **2** |
| 3 | 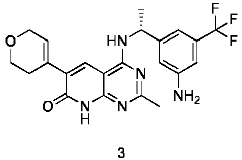 |
| | (*R*)-4-((1-(3-amino-5-(trifluoromethyl)phenyl)ethyl)amino)-6-(3,6-dihy dro-2*H*-pyran-4-yl)-2-methylpyrido[2,3-*d*] pyrimidin-7(8*H*)-one **3** |
| 4 | 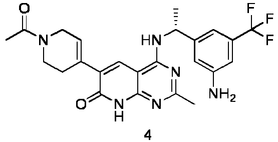 |
| | (*R*)-6-(1-acetyl-1,2,3,6-tetrahydropyridin-4-yl)-4-((1-(3-amino-5-(trifluo romethyl)phenyl)ethyl)amino)-2-methylpyrido[2,3-*d*]pyrimidin-7(8*H*)-one **4** |
| 5 | 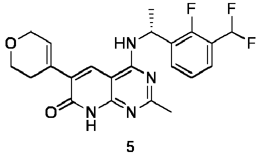 |
| | (*R*)-4-((1-(3-(difluoromethyl)-2-fluorophenyl)ethyl)amino)-6-(3,6-dihyd ro-2*H*-pyran-4-yl)-2-methylpyrido[2,3-*d*]pyrimidin-7(8*H*)-one **5** |
| 6 | 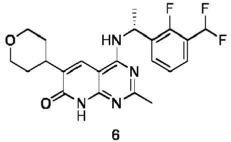 |
| | (*R*)-4-((1-(3-(difluoromethyl)-2-fluorophenyl)ethyl)amino)-2-methyl-6-( tetrahydro-2*H*-pyran-4-yl)pyrido[2,3-*d*]pyrimidin-7(8*H*)-one **6** |
| 7 | 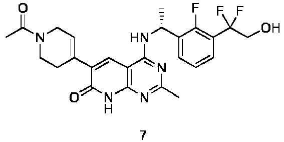 |
| | (*R*)-6-(1-acetyl-1,2,3,6-tetrahydropyridin-4-yl)-4-((1-(3-(1,1-difluoro-2-hydroxyethyl)-2-fluorophenyl)ethyl)amino)-2-methylpyrido[2,3-*d*]pyri midin-7(8*H*)-one **7** |
| 8 | 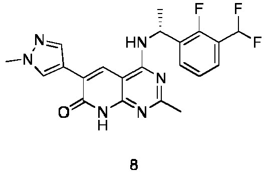 |
| | (*R*)-4-((1-(3-(difluoromethyl)-2-fluorophenyl)ethyl)amino)-2-methyl-6-( 1-methyl-1*H*-pyrazol-4-yl)pyrido[2,3-*d*]pyrimidin-7(8*H*)-one **8** |
| 9 | 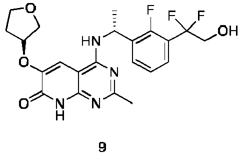 |
| | 4-(((*R*)-1-(3-(1,1-difluoro-2-hydroxyethyl)-2-fluorophenyl)ethyl)amino) -2-methyl-6-(((*R*)-tetrahydrofuran-3-yl)oxy)pyrido[2,3-*d*]pyrimidin-7(8 *H*)-one **9** |
| 10 | 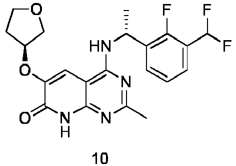 |
| | 4-(((*R*)-1-(3-(difluoromethyl)-2-fluorophenyl)ethyl)amino)-2-methyl-6-( ((*S*)-tetrahydrofuran-3-yl)oxy)pyrido[2,3-*d*]pyrimidin-7(8*H*)-one **10** |
| 11a | 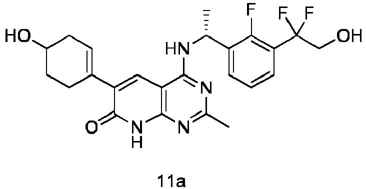 |
| | 4-(((*R*)-1-(3-(1,1-difluoro-2-hydroxyethyl)-2-fluorophenyl)ethyl)amino) -6-(4-hydroxycyclohex-1-en-1-yl)-2-methylpyrido[2,3-*d*]pyrimidin-7(8 *H*)-one **11a** |
| 11-p1 | 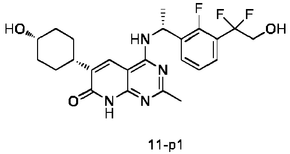 |
| | 4-(((*R*)-1-(3-(1,1-difluoro-2-hydroxyethyl)-2-fluorophenyl)ethyl)amino) -6-((1*s*,4*S*)-4-hydroxycyclohexyl)-2-methylpyrido[2,3-*d*]pyrimidin-7(8 *H*)-one **11-p1** |
| 11-p2 | 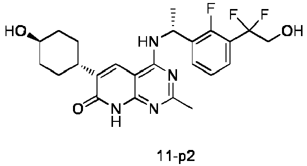 |
| | 4-(((*R*)-1-(3-(1,1-difluoro-2-hydroxyethyl)-2-fluorophenyl)ethyl)amino) -6-((1*r*,4*R*)-4-hydroxycyclohexyl)-2-methylpyrido[2,3-*d*]pyrimidin-7(8 *H*)-one **11-p2** |
| 12a | 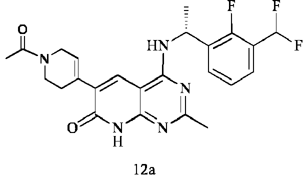 |
| | (*R*)-6-(1-acetyl-1,2,3,6-tetrahydropyridin-4-yl)-4-((1-(3-(difluoromethyl )-2-fluorophenyl)ethyl)amino)-2-methylpyrido[2,3-*d*]pyrimidin-7(8*H*)-o ne **12a** |
| 12 | 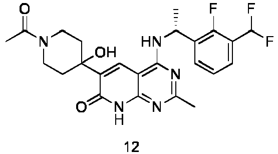 |
| | (*R*)-6-(1-acetyl-4-hydroxypiperidin-4-yl)-4-((1-(3-(difluoromethyl)-2-fl uorophenyl)ethyl)amino)-2-methylpyrido[2,3-*d*]pyrimidin-7(8*H*)-one **12** |
| 13 | 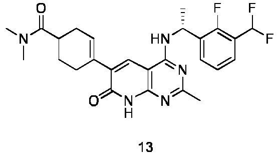 |
| | 4-(4-(((*R*)-1-(3-(difluoromethyl)-2-fluorophenyl)ethyl)amino)-2-methyl-7-oxo-7,8-dihydropyrido[2,3-*d*]pyrimidin-6-yl)-*N*,*N*-dimethylcyclohex-3-ene-1-carboxamide **13** |
| 14-p1 | 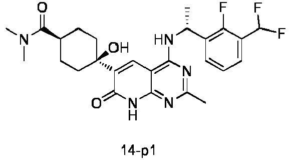 |
| | (1*S*,4*s*)-4-(4-(((*R*)-1-(3-(difluoromethyl)-2-fluorophenyl)ethyl)amino)-2-methyl-7-oxo-7,8-dihydropyrido[2,3-*d*]pyrimidin-6-yl)-4-hydroxy-*N*,*N-*dimethylcyclohexane carboxamide **14-p1** |
| 14-p2 | 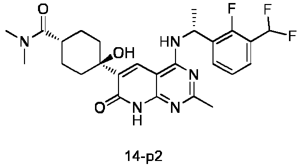 |
| | (1*R*,4*r*)-4-(4-(((*R*)-1-(3-(difluoromethyl)-2-fluorophenyl)ethyl)amino)-2 -methyl-7-oxo-7,8-dihydropyrido[2,3-*d*]pyrimidin-6-yl)-4-hydroxy-*N*,*N* -dimethylcyclohexane carboxamide **14-p2** |
| 15 | 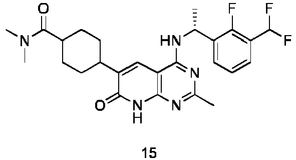 |
| | (*R*)-4-(4-((1-(3-(difluoromethyl)-2-fluorophenyl)ethyl)amino)-2-methyl-7-oxo-7,8-dihydropyrido[2,3-*d*]pyrimidin-6-yl)-*N*,*N*-dimethylcyclohexa ne carboxamide **15** |
| 16 | 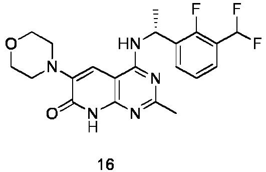 |
| | (*R*)-4-((1-(3-(difluoromethyl)-2-fluorophenyl)ethyl)amino)-2-methyl-6-morpholinopyrido[2,3-*d*]pyrimidin-7(8*H*)-one **16** |
| 17d | 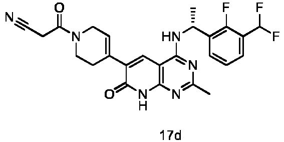 |
| | (*R*)-3-(4-(4-((1-(3-(difluoromethyl)-2-fluorophenyl)ethyl)amino)-2-met hyl-7-oxo-7,8-dihydropyrido[2,3-*d*]pyrimidin-6-yl)-3,6-dihydropyridin-1(2*H*)-yl)-3-oxopropanenitrile **17d** |
| 17 | 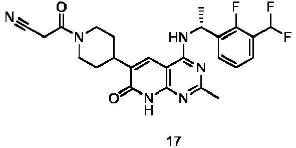 |
| | (*R*)-3-(4-(4-((1-(3-(difluoromethyl)-2-fluorophenyl)ethyl)amino)-2-met hyl-7-oxo-7,8-dihydropyrido[2,3-*d*]pyrimidin-6-yl)piperidin-1-yl)-3-ox opropanenitrile **17** |
| 18d | 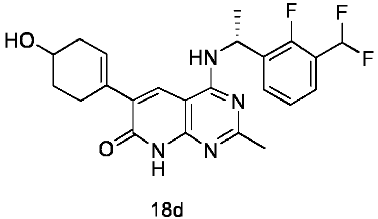 |
| | 4-(((*R*)-1-(3-(difluoromethyl)-2-fluorophenyl)ethyl)amino)-6-(4-hydrox ycyclohex-1-en-1-yl)-2-methylpyrido[2,3-*d*]pyrimidin-7(8*H*)-one **18d** |
| 18-p1 | 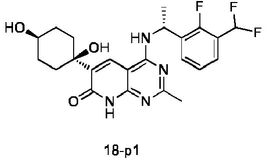 |
| | 4-(((*R*)-1-(3-(difluoromethyl)-2-fluorophenyl)ethyl)amino)-6-((1*s*,4*S*)-1, 4-dihydroxycyclohexyl)-2-methylpyrido[2,3-*d*]pyrimidin-7(8*H*)-one **18-p1** |
| 18-p2 | 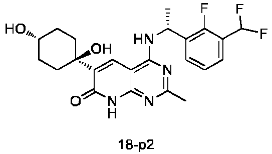 |
| | 4-(((*R*)-1-(3-(difluoromethyl)-2-fluorophenyl)ethyl)amino)-6-((1*r*,4*R*)-1 ,4-dihydroxycyclohexyl)-2-methylpyrido[2,3-*d*]pyrimidin-7(8*H*)-one **18-p2** |
| 19 | 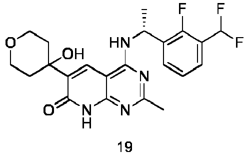 |
| | (*R*)-4-((1-(3-(difluoromethyl)-2-fluorophenyl)ethyl)amino)-6-(4-hydrox ytetrahydro-2*H*-pyran-4-yl)-2-methylpyrido[2,3-*d*]pyrimidin-7(8*H*)-one **19** |
| 20a | 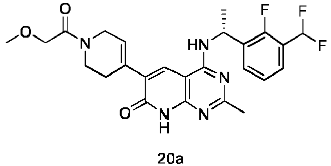 |
| | (*R*)-4-((1-(3-(difluoromethyl)-2-fluorophenyl)ethyl)amino)-6-(1-(2-met hoxyacetyl)-1,2,3,6-tetrahydropyridin-4-yl)-2-methylpyrido[2,3-*d*]pyri midin-7(8*H*)-one **20a** |
| 20 | 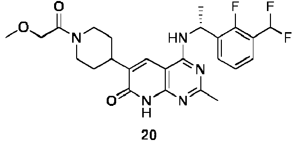 |
| | (*R*)-4-((1-(3-(difluoromethyl)-2-fluorophenyl)ethyl)amino)-6-(1-(2-met hoxyacetyl)piperidin-4-yl)-2-methylpyrido[2,3-*d*]pyrimidin-7(8*H*)-one **20** |
| 21 | 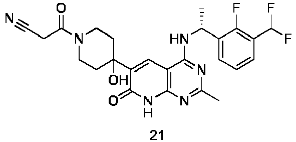 |
| | (*R*)-3-(4-(4-((1-(3-(difluoromethyl)-2-fluorophenyl)ethyl)amino)-2-met hyl-7-oxo-7,8-dihydropyrido[2,3-*d*]pyrimidin-6-yl)-4-hydroxypiperidin-1-yl)-3-oxopropanenitrile **21** |
| 22a | 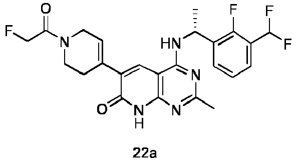 |
| | (*R*)-4-((1-(3-(difluoromethyl)-2-fluorophenyl)ethyl)amino)-6-(1-(2-fluor oacetyl)-1,2,3,6-tetrahydropyridin-4-yl)-2-methylpyrido[2,3-*d*]pyrimidi n-7(8*H*)-one **22a** |
| 22 | 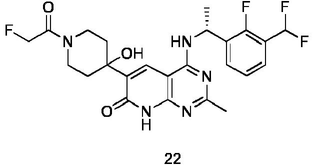 |
| | *rac*-(*R*)-4-((1-(3-(difluoromethyl)-2-fluorophenyl)ethyl)amino)-6-(1-(2-fluoroacetyl)-4-hydroxypiperidin-4-yl)-2-methylpyrido[2,3-*d*]pyrimidin -7(8*H*)-one **22** |
| 23 | 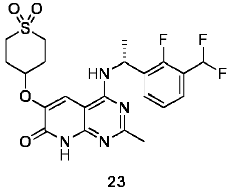 |
| | (*R*)-4-((1-(3-(difluoromethyl)-2-fluorophenyl)ethyl)amino)-6-((1,1-dioxi dotetrahydro-2*H*-thiopyran-4-yl)oxy)-2-methylpyrido[2,3-*d*]pyrimidin-7 (8*H*)-one **23** |
| 24 | 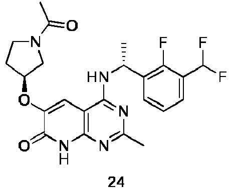 |
| | 6-(((*S*)-1-acetylpyrrolidin-3-yl)oxy)-4-(((*R*)-1-(3-(difluoromethyl)-2-flu orophenyl)ethyl)amino)-2-methylpyrido[2,3-*d*]pyrimidin-7(8*H*)-one **24** |
| 25-p1 | 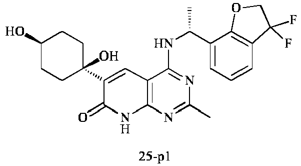 |
| | *rac*-4-(((*R*)-1-(3,3-difluoro-2,3-dihydrobenzofuran-7-yl)ethyl)amino)-6-((1*s*,4*S*)-1,4-dihydroxycyclohexyl)-2-methylpyrido[2,3-*d*]pyrimidin-7(8 *H*)-one **25-p1** |
| 25-p2 | 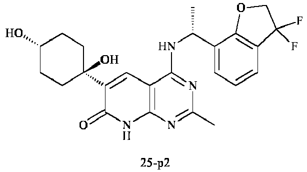 |
| | *rac*-4-(((*R*)-1-(3,3-difluoro-2,3-dihydrobenzofuran-7-yl)ethyl)amino)-6-((1*r*,4*R*)-1,4-dihydroxycyclohexyl)-2-methylpyrido[2,3-*d*]pyrimidin-7(8 *H*)-one **25-p2** |
| 26c | 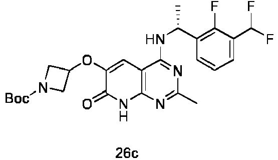 |
| | *Tert*-butyl (*R*)-3-((4-((1-(3-(difluoromethyl)-2-fluorophenyl)ethyl)amino)-2-methyl -7-oxo-7,8-dihydropyrido[2,3-*d*]pyrimidin-6-yl)oxy)azetidine-1-carbox ylate **26c** |
| 26d | 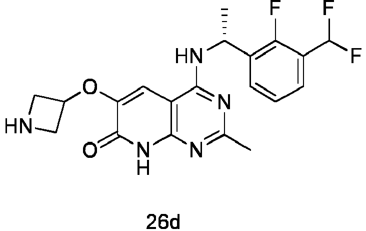 |
| | (*R*)-6-(azetidin-3-yloxy)-4-((1-(3-(difluoromethyl)-2-fluorophenyl)ethyl )amino)-2-methylpyrido[2,3-*d*]pyrimidin-7(8*H*)-one **26d** |
| 26 | 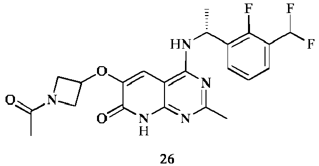 |
| | *rac*-(*R*)-6-((1-acetylazetidin-3-yl)oxy)-4-((1-(3-(difluoromethyl)-2-fluor ophenyl)ethyl)amino)-2-methylpyrido[2,3-*d*]pyrimidin-7(8*H*)-one **26** |
| 27 | 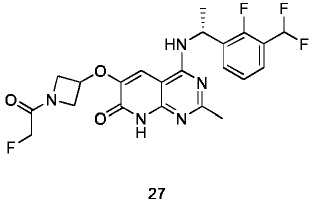 |
| | (*R*)-4-((1-(3-(difluoromethyl)-2-fluorophenyl)ethyl)amino)-6-((1-(2-fluo roacetyl)azetidin-3-yl)oxy)-2-methylpyrido[2,3-*d*]pyrimidin-7(8*H*)-one **27** |
| 28-p 1 | 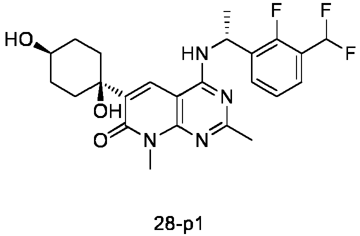 |
| | 4-(((*R*)-1-(3-(difluoromethyl)-2-fluorophenyl)ethyl)amino)-6-((1*s*,4*S*)-1, 4-dihydroxycyclohexyl)-2,8-dimethylpyrido[2,3-*d*]pyrimidin-7(8*H*)-one **28-p1** |
| 28-p2 | 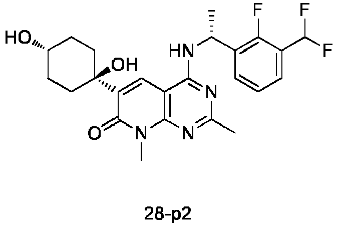 |
| | 4-(((*R*)-1-(3-(difluoromethyl)-2-fluorophenyl)ethyl)amino)-6-((1*r,*4*R*)-1 ,4-dihydroxycyclohexyl)-2,8-dimethylpyrido[2,3-*d*]pyrimidin-7(8*H*)-on e **28-p2** |
| 29a | 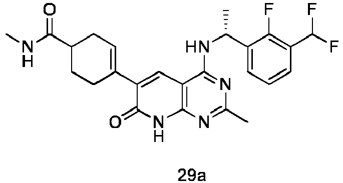 |
| | 4-(4-(((*R*)-1-(3-(difluoromethyl)-2-fluorophenyl)ethyl)amino)-2-methyl-7-oxo-7,8-dihydropyrido[2,3-*d*]pyrimidin-6-yl)-*N*-methylcyclohex-3-en e-1-carboxamide **29a** |
| 29 | 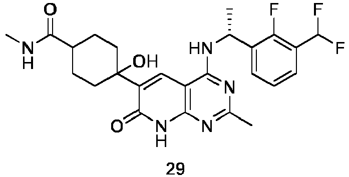 |
| | (*R*)-4-(4-((1-(3-(difluoromethyl)-2-fluorophenyl)ethyl)amino)-2-methyl-7-oxo-7,8-dihydropyrido[2,3-*d*]pyrimidin-6-yl)-4-hydroxy-*N*-methylcyc lohexane-1-carboxamide **29** |

Another aspect of the present disclosure relates to a compound of general formula (IA) or a tautomer, mesomer, racemate, enantiomer or diastereomer thereof or a mixture thereof, or a pharmaceutically acceptable salt thereof: 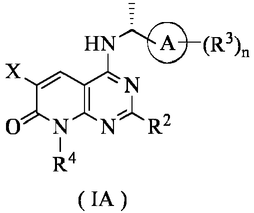 wherein:
X is halogen, preferably Br;
ring A, R² to R⁴ and n are as defined in the compound of general formula (I).

Another aspect of the present disclosure relates to a compound of general formula (IIA) or a tautomer, mesomer, racemate, enantiomer or diastereomer thereof or a mixture thereof, or a pharmaceutically acceptable salt thereof: 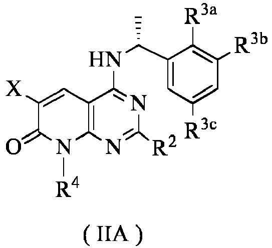 wherein:
X is halogen, preferably Br;
R², R^{3a}, R^{3b}, R^{3c} and R⁴ are as defined in the compound of general formula (II).

Typical compounds of general formula (IA) or general formula (IIA) of the present disclosure include, but are not limited to:

| Example No. | Structure and name of compound |
|---|---|
| 1g | 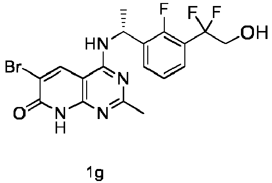 |
| | (*R*)-6-bromo-4-((1-(3-(1,1-difluoro-2-hydroxyethyl)-2-fluorophenyl)eth yl)amino)-2-methylpyrido[2,3-*d*]pyrimidin-7(8*H*)-one **1g** |
| 2c | 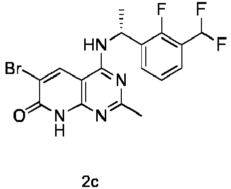 |
| | (*R*)-6-bromo-4-((1-(3-(difluoromethyl)-2-fluorophenyl)ethyl)amino)-2-methylpyrido[2,3-*d*]pyrimidin-7(8*H*)-one **2c** |
| | 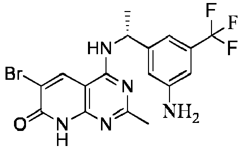 |
| | (*R*)-6-bromo-4-((1-(3-amino-5-(trifluoroethyl)phenyl)ethyl)amino)-2-m ethylpyrido[2,3-*d*]pyrimidin-7(8*H*)-one |
| 28a | 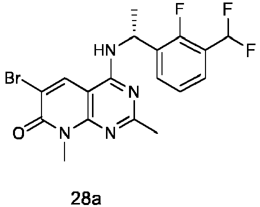 |
| | (*R*)-6-bromo-4-((1-(3-(difluoromethyl)-2-fluorophenyl)ethyl)amino)-2,8 -dimethylpyrido[2,3-*d*]pyrimidin-7(8*H*)-one **28a** |

Another aspect of the present disclosure relates to a compound of general formula (IB) or a tautomer, mesomer, racemate, enantiomer or diastereomer thereof or a mixture thereof, or a pharmaceutically acceptable salt thereof: 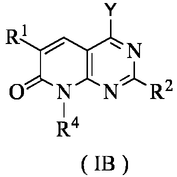 wherein: y is halogen, preferably Cl;
R² is selected from the group consisting of halogen, alkyl, haloalkyl, alkoxy, haloalkoxy, hydroxyalkyl, cyano and cycloalkyl;
R¹ and R⁴ are as defined in the compound of general formula (I).

Typical compounds of general formula (IB) of the present disclosure include, but are not limited to:

| Example No. | Structure and name of compound |
|---|---|
| 9c | 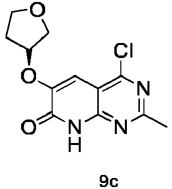 |
| | (*R*)-4-chloro-2-methyl-6-((tetrahydrofuran-3-yl)oxy)pyrido[2,3-*d*]pyrim idin-7(8*H*)-one |

Another aspect of the present disclosure relates to a method for preparing a compound of general formula (I) or a tautomer, mesomer, racemate, enantiomer or diastereomer thereof or a mixture thereof, or a pharmaceutically acceptable salt thereof, which comprises the following step: 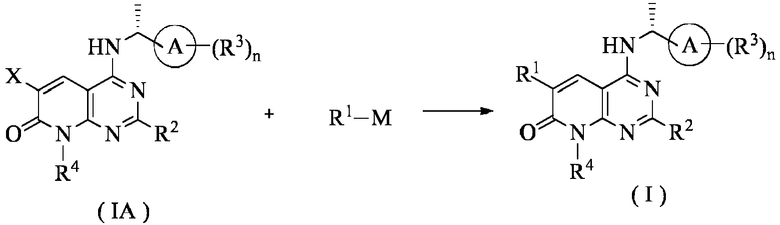 subjecting a compound of general formula (IA) or a tautomer, mesomer, racemate, enantiomer or diastereomer thereof or a mixture thereof, or a pharmaceutically acceptable salt thereof to a reaction with R¹-M to give a compound of general formula (I) or a tautomer, mesomer, racemate, enantiomer or diastereomer thereof or a mixture thereof, or a pharmaceutically acceptable salt thereof;
wherein:
X is halogen, preferably Br;
M is selected from the group consisting of 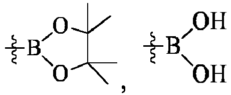 and hydrogen;
ring A, R¹ to R⁴ and n are as defined in the compound of general formula (I).

Another aspect of the present disclosure relates to a method for preparing a compound of general formula (IAA) or a tautomer, mesomer, racemate, enantiomer or diastereomer thereof or a mixture thereof, or a pharmaceutically acceptable salt thereof, which comprises the following step: 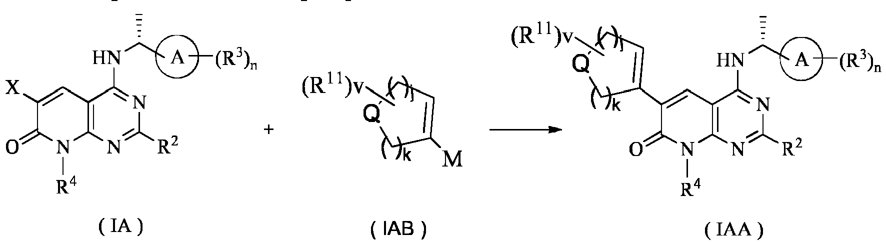 subjecting a compound of general formula (IA) or a tautomer, mesomer, racemate, enantiomer or diastereomer thereof or a mixture thereof, or a pharmaceutically acceptable salt thereof to a reaction with a compound of general formula (IAB) to give a compound of general formula (IAA) or a tautomer, mesomer, racemate, enantiomer or diastereomer thereof or a mixture thereof, or a pharmaceutically acceptable salt thereof; wherein:
X is halogen, preferably Br;
M is selected from the group consisting of 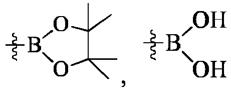 and hydrogen;
Q is selected from the group consisting of oxygen, sulfur, 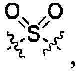 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
v is 0, 1, 2 or 3;
ring A, R² to R⁴, R⁷ to R¹⁰, p, q and n are as defined in the compound of general formula (I).

Another aspect of the present disclosure relates to a method for preparing a compound of general formula (I) or a tautomer, mesomer, racemate, enantiomer or diastereomer thereof or a mixture thereof, or a pharmaceutically acceptable salt thereof, which comprises the following step: 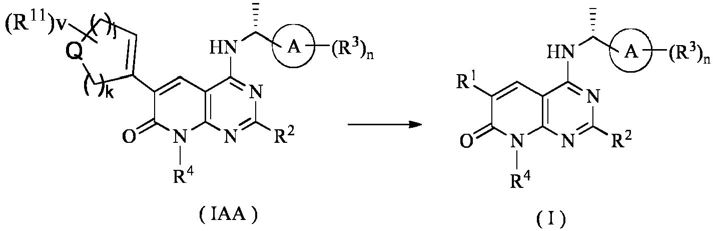 subjecting a compound of general formula (IAA) or a tautomer, mesomer, racemate, enantiomer or diastereomer thereof or a mixture thereof, or a pharmaceutically acceptable salt thereof to an oxidation reaction to give a compound of general formula (I) or a tautomer, mesomer, racemate, enantiomer or diastereomer thereof or a mixture thereof, or a pharmaceutically acceptable salt thereof;
wherein:
R¹ is 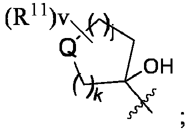
Q is selected from the group consisting of oxygen, sulfur, 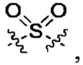 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
v is 0, 1, 2 or 3;
ring A, R² to R⁴, R⁷ to R¹⁰, p, q and n are as defined in the compound of general formula (I).

Another aspect of the present disclosure relates to a method for preparing a compound of general formula (II) or a tautomer, mesomer, racemate, enantiomer or diastereomer thereof or a mixture thereof, or a pharmaceutically acceptable salt thereof, which comprises the following step: 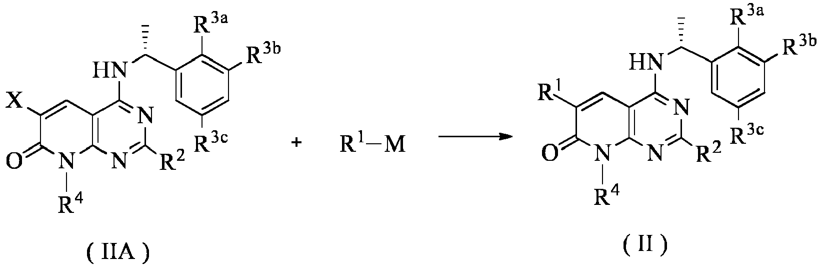 subjecting a compound of general formula (IIA) or a tautomer, mesomer, racemate, enantiomer or diastereomer thereof or a mixture thereof, or a pharmaceutically acceptable salt thereof to a reaction with R¹-M to give a compound of general formula (II) or a tautomer, mesomer, racemate, enantiomer or diastereomer thereof or a mixture thereof, or a pharmaceutically acceptable salt thereof;
wherein:
X is halogen, preferably Br;
M is selected from the group consisting of 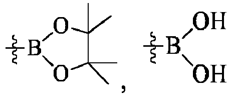 and hydrogen;
R¹, R², R^{3a}, R^{3b}, R^{3c} and R⁴ are as defined in the compound of general formula (II). Another aspect of the present disclosure relates to a method for preparing a compound of general formula (IIAA) or a tautomer, mesomer, racemate, enantiomer or diastereomer thereof or a mixture thereof, or a pharmaceutically acceptable salt thereof, which comprises the following step: 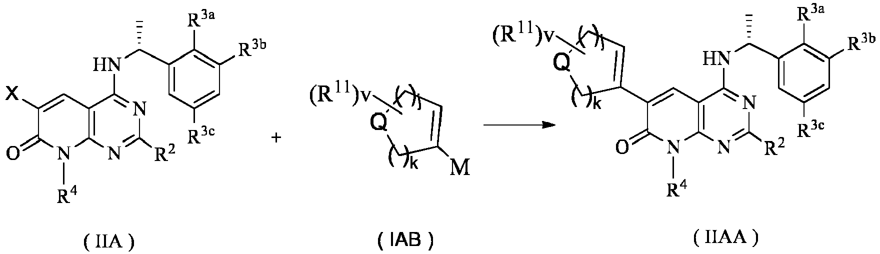 subjecting a compound of general formula (IIA) or a tautomer, mesomer, racemate, enantiomer or diastereomer thereof or a mixture thereof, or a pharmaceutically acceptable salt thereof to a reaction with a compound of general formula (IAB) to give a compound of general formula (IIAA) or a tautomer, mesomer, racemate, enantiomer or diastereomer thereof or a mixture thereof, or a pharmaceutically acceptable salt thereof;
   wherein:
   X is halogen, preferably Br;
   M is selected from the group consisting of 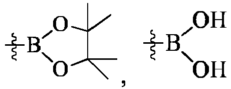 and hydrogen;
   Q is selected from the group consisting of oxygen, sulfur 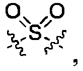 NR^{11a} and CR^{11b}R^{11c};
   R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
   j is 0, 1 or 2;
   k is 1 or 2;
   v is 0, 1, 2 or 3;
   R², R^{3a}, R^{3b}, R^{3c}, R⁴, R⁷ to R¹⁰, p and q are as defined in the compound of general formula (II).

Another aspect of the present disclosure relates to a method for preparing a compound of general formula (II) or a tautomer, mesomer, racemate, enantiomer or diastereomer thereof or a mixture thereof, or a pharmaceutically acceptable salt thereof, which comprises the following step: 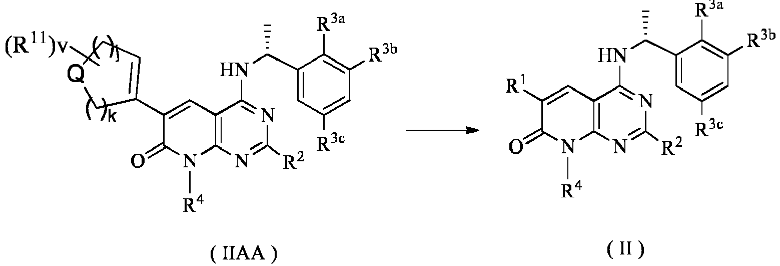 subjecting a compound of general formula (IIAA) or a tautomer, mesomer, racemate, enantiomer or diastereomer thereof or a mixture thereof, or a pharmaceutically acceptable salt thereof to an oxidation reaction to give a compound of general formula (I) or a tautomer, mesomer, racemate, enantiomer or diastereomer thereof or a mixture thereof, or a pharmaceutically acceptable salt thereof; wherein:
R¹ is 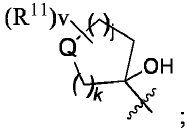
Q is selected from the group consisting of oxygen, sulfur, 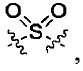 NR^{11a}and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
v is 0, 1, 2 or 3;
R², R^{3a}, R^{3b}, R^{3c}, R⁴, R⁷ to R¹⁰, p and q are as defined in the compound of general formula (II).

Another aspect of the present disclosure relates to a method for preparing a compound of general formula (I) or a tautomer, mesomer, racemate, enantiomer or diastereomer thereof or a mixture thereof, or a pharmaceutically acceptable salt thereof, which comprises the following step: 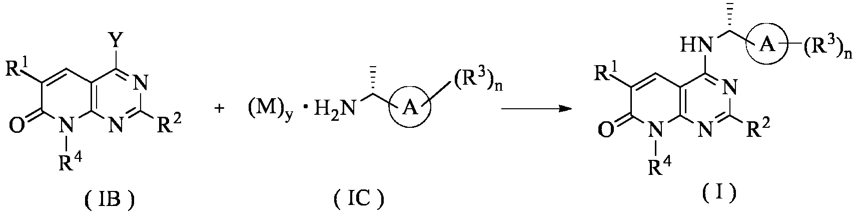 subjecting a compound of general formula (IB) or a tautomer, mesomer, racemate, enantiomer or diastereomer thereof or a mixture thereof, or a pharmaceutically acceptable salt thereof to a reaction with a compound of general formula (IC) to give a compound of general formula (I) or a tautomer, mesomer, racemate, enantiomer or diastereomer thereof or a mixture thereof, or a pharmaceutically acceptable salt thereof; wherein:
M is HCl;
y is 0 or 1;
Y is halogen, preferably Cl;
ring A, R¹ to R⁴ and n are as defined in the compound of general formula (I).

Another aspect of the present disclosure relates to a method for preparing a compound of general formula (II) or a tautomer, mesomer, racemate, enantiomer or diastereomer thereof or a mixture thereof, or a pharmaceutically acceptable salt thereof, which comprises the following step: 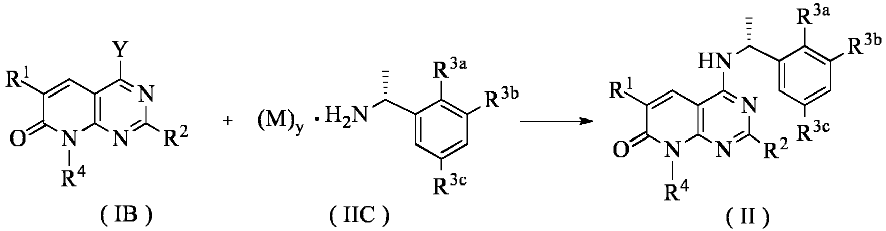 subjecting a compound of general formula (IB) or a tautomer, mesomer, racemate, enantiomer or diastereomer thereof or a mixture thereof, or a pharmaceutically acceptable salt thereof to a reaction with a compound of general formula (IIC) to give a compound of general formula (II) or a tautomer, mesomer, racemate, enantiomer or diastereomer thereof or a mixture thereof, or a pharmaceutically acceptable salt thereof; wherein:
M is HCl;
y is 0 or 1;
Y is halogen, preferably Cl;
R¹, R², R^{3a}, R^{3b}, R^{3c} and R⁴ are as defined in the compound of general formula (II). Another aspect of the present disclosure relates to a pharmaceutical composition comprising a therapeutically effective amount of the compound of general formula (I) or general formula (II) and compounds shown in Table A or the tautomer, mesomer, racemate, enantiomer or diastereomer thereof or the mixture thereof, or the pharmaceutically acceptable salt thereof, and one or more pharmaceutically acceptable carrier, diluent or excipient.

The present disclosure further relates to use of the compound of general formula (I) or general formula (II) and compounds shown in Table A or the atropisomer, tautomer, mesomer, racemate, enantiomer or diastereomer thereof or the mixture thereof, or the pharmaceutically acceptable salt thereof, or the pharmaceutical composition comprising the same in preparing a medicament for the inhibition of SOS 1.

The present disclosure further relates to use of the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same in preparing a medicament for the treatment and/or prevention of a cancer, an inflammation, an RAS disease, Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome, hereditary gingival fibromatosis, or other proliferative diseases, preferably in preparing a medicament for the treatment and/or prevention of a cancer, wherein particularly, the cancer is selected from the group consisting of melanoma, skin cancer, liver cancer, kidney cancer, lung cancer, nasopharyngeal cancer, stomach cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, brain tumor, myeloma, astrocytoma, glioblastoma and glioma; the RAS disease is preferably neurofibromatosis type 1 (NF1); the lung cancer is preferably non-small cell lung cancer, further preferably metastatic non-small cell lung cancer; the leukemia is preferably chronic lymphocytic leukemia or acute myelogenous leukemia; the lymphoma is preferably diffuse large B cell lymphoma; the myeloma is preferably multiple myeloma; the osteoma is preferably osteochondroma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

The present disclosure further relates to a method for inhibiting SOS1, which comprises administering to a patient in need a therapeutically effective amount of the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same.

The present disclosure further relates to a method for treating and/or preventing an SOS1-mediated disease, which comprises administering to a patient in need a therapeutically effective amount of the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same.

The present disclosure further relates to a method for treating and/or preventing a cancer, an inflammation, an RAS disease, Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome, hereditary gingival fibromatosis, or other proliferative diseases, preferably a method for treating and/or preventing cancer, which comprises administering to a patient in need a therapeutically effective amount of the compound of general formula (I) or general formula (II) and compounds shown in Table A or the tautomer, mesomer, racemate or enantiomer, diastereomer thereof or the mixture thereof, or the pharmaceutically acceptable salt thereof, or the pharmaceutical composition comprising the same; wherein the cancer is preferably selected from the group consisting of melanoma, skin cancer, liver cancer, kidney cancer, lung cancer, nasopharyngeal cancer, stomach cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, brain tumor, myeloma, astrocytoma, glioblastoma and glioma; the RAS disease is preferably neurofibromatosis type 1 (NF1); the lung cancer is preferably non-small cell lung cancer, further preferably metastatic non-small cell lung cancer; the leukemia is preferably chronic lymphocytic leukemia or acute myelogenous leukemia; the lymphoma is preferably diffuse large B cell lymphoma; the myeloma is preferably multiple myeloma; the osteoma is preferably osteochondroma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

The present disclosure further relates to the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same, for use as a medicament.

The present disclosure further relates to the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same, for use as an SOS1 inhibitor.

The present disclosure further relates to the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same, for use in treating and/or preventing an SOS1-mediated disease.

The present disclosure further relates to the compound of general formula (I) or general formula (II) and compounds shown in Table A or the tautomer, mesomer, racemate, enantiomer or diastereomer thereof or the mixture thereof, or the pharmaceutically acceptable salt thereof, or the pharmaceutical composition comprising the same, for use in treating and/or preventing cancer, inflammation, an RAS disease, Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome, hereditary gingival fibromatosis, or other proliferative diseases, preferably in treating and/or preventing cancer; wherein the cancer is preferably selected from the group consisting of melanoma, skin cancer, liver cancer, kidney cancer, lung cancer, nasopharyngeal cancer, stomach cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, brain tumor, myeloma, astrocytoma, glioblastoma and glioma; the RAS disease is preferably neurofibromatosis type 1 (NF1); the lung cancer is preferably non-small cell lung cancer, further preferably metastatic non-small cell lung cancer; the leukemia is preferably chronic lymphocytic leukemia or acute myelogenous leukemia; the lymphoma is preferably diffuse large B cell lymphoma; the myeloma is preferably multiple myeloma; the osteoma is preferably osteochondroma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular or subcutaneous), or administration by inhalation or insufflation. The compound of the present disclosure may also be formulated into a sustained-release dosage form, such as tablets, hard or soft capsules, aqueous or oily suspensions, emulsions, injections, dispersible powders or granules, suppositories, lozenges or syrups.

As a general guide, the active compound is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder and a lubricant. Such a tablet may be uncoated or may be coated by known techniques for masking the taste of the drug or delaying the disintegration and absorption of the drug in the gastrointestinal tract and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant or a wetting agent. The aqueous suspension may also comprise one or more preservatives, one or more colorants, one or more corrigents and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative and an antioxidant. Such a formulation may also comprise a palliative, a preservative, a colorant and an antioxidant.

The pharmaceutical composition of the present disclosure may be in the form of a sterile injectable aqueous solution. Acceptable vehicles or solvents that can be used include water, Ringer's solution and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of administration, the daily dose of the compound or the type of pharmaceutically acceptable salts, can be verified according to conventional treatment regimens.

### Definition of terms

Unless otherwise stated, the terms used in the specification and claims have the following meanings.

The term "alkyl" refers to a saturated aliphatic hydrocarbon group which is a linear or branched group containing 1 to 20 carbon atoms, preferably alkyl containing 1 to 12 (e.g., 1, 2, 3, 4, 5, 6, 7, 8, 9, 10, 11 and 12) carbon atoms, and more preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, it may be substituted at any accessible connection site, wherein the substituent is preferably one or more substituents independently and optionally selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group, which is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11 and 12) carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. The alkylene may be substituted or unsubstituted. When substituted, it may be substituted at any accessible connection site, wherein the substituent is preferably one or more substituents independently and optionally selected from the group consisting of alkenyl, alkynyl, alkoxy, haloalkoxy, cycloalkyloxy, heterocyclyloxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to an alkyl compound containing at least one carbon-carbon double bond in the molecule, wherein the alkyl is as defined above. The alkenyl is preferably one containing 2 to 12 (e.g., 2, 3, 4, 5, 6, 7, 8, 9, 10, 11 and 12) carbon atoms and more preferably one containing 2 to 6 carbon atoms. The alkenyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing at least one carbon-carbon triple bond in the molecule, wherein the alkyl is as defined above. The alkynyl is preferably one containing 2 to 12 (e.g., 2, 3, 4, 5, 6, 7, 8, 9, 10, 11 and 12) carbon atoms and more preferably one containing 2 to 6 carbon atoms. The alkynyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, preferably 3 to 10 carbon atoms (e.g., 3, 4, 5, 6, 7, 8, 9 or 10), and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one carbon atom (referred to as the spiro atom), wherein the spiro cycloalkyl may contain one or more double bonds. The spiro cycloalkyl is preferably 6- to 14-membered, and more preferably 7- to 10-membered (e.g., 7-membered, 8-membered, 9-membered or 10-membered). According to the number of the spiro atoms shared among the rings, the spiro cycloalkyl may be monospiro cycloalkyl, bispiro cycloalkyl or polyspiro cycloalkyl, preferably monospiro cycloalkyl and bispiro cycloalkyl, and more preferably 3-membered/5-membered, 3-membered/6-membered, 4-membered/4-membered, 4-membered/5-membered, 4-membered/6-membered, 5-membered/5-membered or 5-membered/6-membered monospiro cycloalkyl. Non-limiting examples of spiro cycloalkyl include:

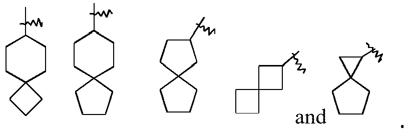

The term "fused cycloalkyl" refers to a 5- to 20-membered carbon polycyclic group in which each ring shares a pair of adjacent carbon atoms with the other rings in the system, wherein one or more of the rings may contain one or more double bonds. The fused cycloalkyl is preferably 6- to 14-membered, and more preferably 7- to 10-membered (e.g., 7-membered, 8-membered, 9-membered or 10-membered). According to the number of the formed rings, the fused cycloalkyl may be bicyclic, tricyclic, tetracyclic or polycyclic fused cycloalkyl, preferably bicyclic or tricyclic fused cycloalkyl, and more preferably 5-membered/5-membered or 5-membered/6-membered bicyclic fused heterocyclyl. Non-limiting examples of fused cycloalkyl include:

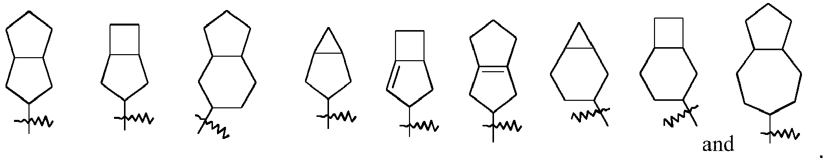

The term "bridged cycloalkyl" refers to a 5- to 20-membered carbon polycyclic group in which any two rings share two carbon atoms that are not directly connected to each other, wherein the bridged cycloalkyl may contain one or more double bonds. The bridged cycloalkyl is preferably 6- to 14-membered, and more preferably 7- to 10-membered (e.g., 7-membered, 8-membered, 9-membered or 10-membered). According to the number of the formed rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

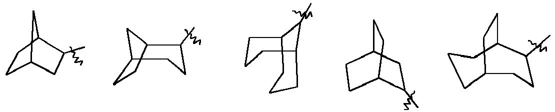

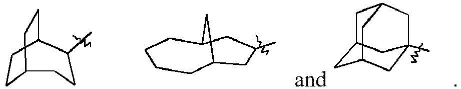

The cycloalkyl ring includes those in which the cycloalkyl described above (including monocyclic, spiro, fused and bridged rings) is fused to an aryl, heteroaryl or heterocycloalkyl ring, wherein the ring connected to the parent structure is cycloalkyl.

Non-limiting examples include 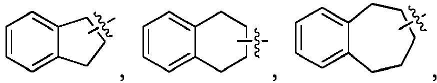 and the like, and preferably

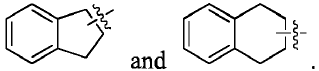

The cycloalkyl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include methoxy, ethoxy, propoxy and butoxy. The alkoxy may be optionally substituted or unsubstituted. When substituted, the substituent is preferably one or more groups independently selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 ring atoms, wherein one or more of the ring atoms is a heteroatom selected from the group consisting of nitrogen, oxygen and sulfur, the sulfur optionally being oxo (i.e., form sulfoxide or sulfone), but excluding a cyclic portion of -O-O-, -O-S- or -S-S-; and the remaining ring atoms are carbon. The heterocyclyl preferably contains 3 to 12 ring atoms, of which 1 to 4 (e.g., 1, 2, 3 and 4) are heteroatoms; more preferably 3 to 10 (e.g., 3, 4, 5, 6, 7, 8, 9 and 10) ring atoms, of which 1 to 3 (e.g., 1, 2 and 3) are heteroatoms; more preferably 3 to 6 ring atoms, of which 1 to 3 are heteroatoms; and most preferably 5 or 6 ring atoms, of which 1 to 3 are heteroatoms. Non-limiting examples of monocyclic heterocyclyl include azetidinyl, furyl, pyrrolidinyl, tetrahydropyranyl, 3,6-dihydropyranyl, 1,2,3,6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5-20 membered polycyclic heterocyclyl group in which monocyclic rings share one atom (referred to as the spiro atom), wherein one or more of the ring atoms is a heteroatom selected from the group consisting of nitrogen, oxygen and sulfur, the sulfur optionally being oxo (i.e., form sulfoxide or sulfone); and the remaining ring atoms are carbon. The spiro heterocyclyl may contain one or more double bonds. The spiro heterocyclyl is preferably 6- to 14-membered, and more preferably 7- to 10-membered (e.g., 7-membered, 8-membered, 9-membered or 10-membered). According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

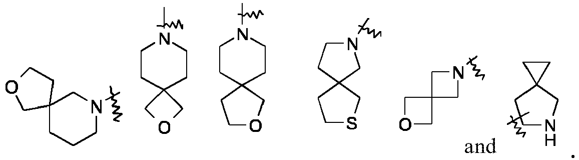

The term "fused heterocyclyl" refers to a 5- to 20-membered polycyclic heterocyclyl group in which each ring shares a pair of adjacent atoms with the other rings in the system, wherein one or more of the rings may contain one or more double bonds, wherein one or more of the ring atoms is a heteroatom selected from the group consisting of nitrogen, oxygen and sulfur, the sulfur optionally being oxo (i.e., form sulfoxide or sulfone); and the remaining ring atoms are carbon. The fused heterocyclyl is preferably 6- to 14-membered, and more preferably 7- to 10-membered (e.g., 7-membered, 8-membered, 9-membered or 10-membered). According to the number of the formed rings, the fused heterocyclyl may be bicyclic, tricyclic, tetracyclic or polycyclic fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered and 6-membered/6-membered bicyclic fused heterocyclyl. Non-limiting examples of fused heterocyclyl include:

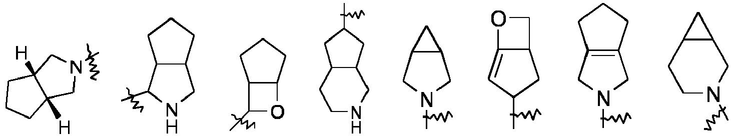

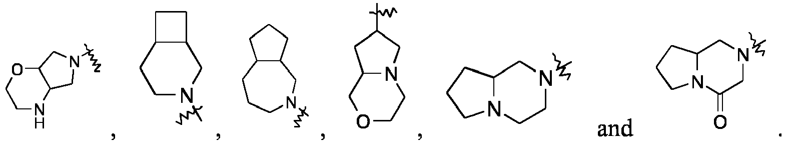

The term "bridged heterocyclyl" refers to a 5- to 14-membered polycyclic heterocyclyl group in which any two rings share two atoms which are not directly connected, wherein the bridged heterocyclyl may contain one or more double bonds, wherein one or more of the ring atoms is a heteroatom selected from the group consisting of nitrogen, oxygen and sulfur, the sulfur optionally being oxo (i.e., form sulfoxide or sulfone); and the remaining ring atoms are carbon. The bridged heterocyclyl is preferably 6- to 14-membered, and more preferably 7- to 10-membered (e.g., 7-membered, 8-membered, 9-membered or 10-membered). According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

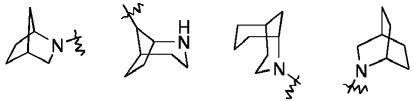

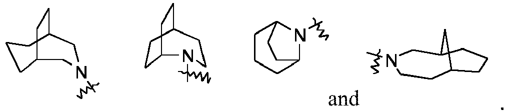

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl. Non-limiting examples include: 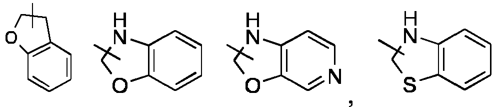 , etc.

The heterocyclyl may be substituted or unsubstituted. When substituted, it may be substituted at any accessible connection site, wherein the substituent is preferably one or more substituents independently and optionally selected from the group consisting of hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered, preferably 6- to 10-membered carbon monocyclic or fused polycyclic (in which the rings share a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is an aryl ring. Non-limiting examples include:

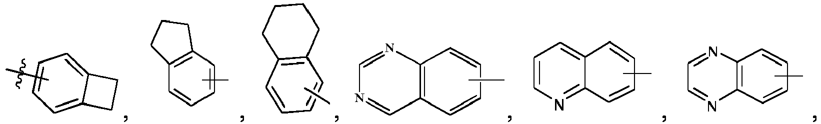

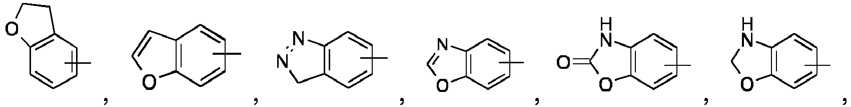

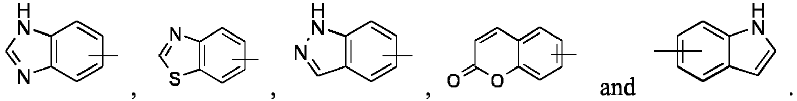

The aryl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3 and 4) heteroatoms and 5 to 14 ring atoms, wherein the heteroatoms are selected from the group consisting of oxygen, sulfur and nitrogen. The heteroaryl is preferably 5- to 10-membered (e.g., 5-membered, 6-membered, 7-membered, 8-membered, 9-membered or 10-membered) and more preferably 5-membered or 6-membered, e.g., furyl, thienyl, pyridinyl, pyrrolyl, *N*-alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring connected to the parent structure is a heteroaryl ring. Non-limiting examples include:

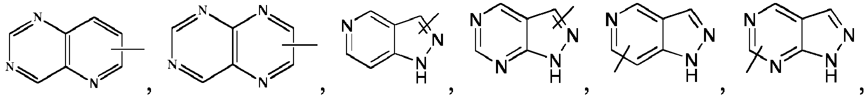

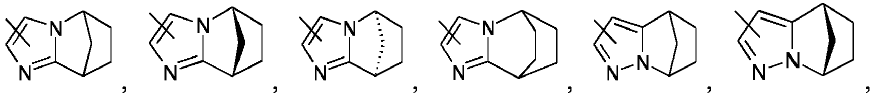

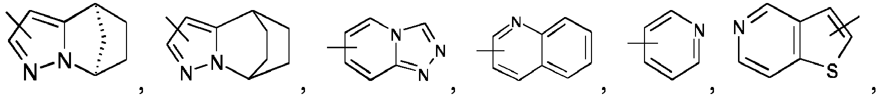

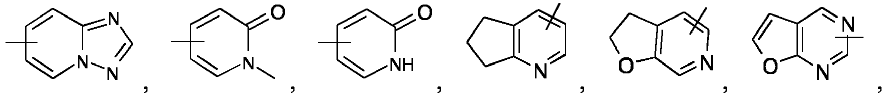

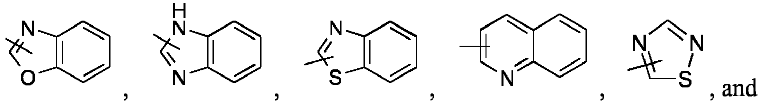

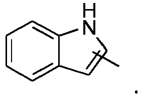

The heteroaryl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom (i.e., a monovalent group), or residues derived from the parent ring by removal of two hydrogen atoms from the same ring atom or two different ring atoms (i.e., divalent groups), i.e., "divalent cycloalkyl", "divalent heterocyclyl", "arylene" and "heteroarylene".

The term "amino protecting group" refers to a group that can be easily removed and is intended to protect an amino group from being changed when a reaction is conducted elsewhere in the molecule. Non-limiting examples include (trimethylsilyl)ethoxymethyl, tetrahydropyranyl, tert-butoxycarbonyl, acetyl, benzyl, allyl, p-methoxybenzyl, and the like. Those groups may be optionally substituted with 1 to 3 substituents selected from the group consisting of halogen, alkoxy and nitro.

The term "hydroxy protecting group" is a suitable group known in the art for protecting hydroxy. See the hydroxy protecting groups in the literature *("*Protective Groups in Organic Synthesis", 5th Ed. T.W.Greene & P.G.M.Wuts). As an example, preferably, the hydroxy protecting group may be (C₁₋₁₀ alkyl or aryl)₃silyl, e.g., triethylsilyl, triisopropylsilyl, *tert*-butyldimethylsilyl, tert-butyldiphenylsilyl or the like; C₁₋₁₀ alkyl or substituted alkyl, preferably alkoxy or aryl-substituted alkyl, more preferably C₁₋₆ alkoxy-substituted C₁₋₆ alkyl or phenyl-substituted C₁₋₆ alkyl, and most preferably C₁₋₄ alkoxy-substituted C₁₋₄ alkyl, e.g., methyl, *tert*-butyl, allyl, benzyl, methoxymethyl (MOM), ethoxyethyl, 2-tetrahydropyranyl (THP) or the like; (C₁₋₁₀ alkyl or aryl)acyl, e.g., formyl, acetyl, benzoyl, p-nitrobenzoyl or the like; (C₁₋₆ alkyl or C₆₋₁₀ aryl)sulfonyl; or (C₁₋₆ alkoxy or C₆₋₁₀ aryloxy)carbonyl.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein aryl is as defined above.

The term "heteroaryloxy" refers to heteroaryl-O-, wherein heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine or iodine.

The term "hydroxy" refers to -OH.

The term "mercapto" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

The present disclosure further comprises various deuterated compounds. Each available hydrogen atom connected to a carbon atom may be independently replaced with a deuterium atom. Those skilled in the art are able to synthesize the deuterated compounds with reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages.

The term "optional" or "optionally" means that the event or circumstance subsequently described may, but not necessarily, occur, and that the description includes instances where the event or circumstance occurs or does not occur. For example, the expression "a heterocyclyl group optionally substituted with alkyl" means that the alkyl may be, but not necessarily, present, and includes instances where the heterocyclyl group is or not substituted with the alkyl.

The term "substituted" means that one or more, preferably 1 to 5, and more preferably 1 to 3 hydrogen atoms in the group are independently substituted with a corresponding number of substituents. Those skilled in the art are able to determine (experimentally or theoretically) possible or impossible substitution without undue efforts. For example, it may be unstable when an amino or hydroxy group having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition is intended to promote the administration to an organism, so as to facilitate the absorption of the active ingredient, thereby exerting biological activities.

The term "pharmaceutically acceptable salt" refers to the salts of the compound of the present disclosure, which are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs and pharmacological active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "solvate" used herein refers to a substance formed by the physical binding of the compound of the present disclosure to one or more, preferably 1 to 3, solvent molecules, whether organic or inorganic. The physical bonding includes hydrogen bonding. In certain cases, e.g., when one or more, preferably 1 to 3, solvent molecules are incorporated in the crystal lattice of the crystalline solid, the solvate will be isolated. Exemplary solvates include, but are not limited to, hydrates, ethanolates, methanolates, and isopropanolates. Solvation methods are well known in the art.

The term "prodrug" refers to a substance that can be converted *in vivo* under physiological conditions, e.g., by hydrolysis in blood, to generate the active prodrug compound.

The term "pharmaceutically acceptable" used herein means that those compounds, materials, compositions and/or dosage forms which are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

The compound of the present disclosure may also include an isotopic derivative thereof. The term "isotopic derivative" refers to compounds that differ in structure only by having one or more enriched isotopic atoms. For example, compounds with the structure of the present disclosure having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic or receptor study.

### Synthesis Method of Compounds of the Present Disclosure

In order to achieve the purpose of the present disclosure, the following technical schemes are adopted in the present disclosure:

### Scheme 1

Provided is a method for preparing the compound of general formula (I) or the salt thereof of the present disclosure, or a method for preparing the tautomer, mesomer, racemate, enantiomer or diastereomer thereof or the mixture thereof, or the pharmaceutically acceptable salt thereof, which comprises the following step: 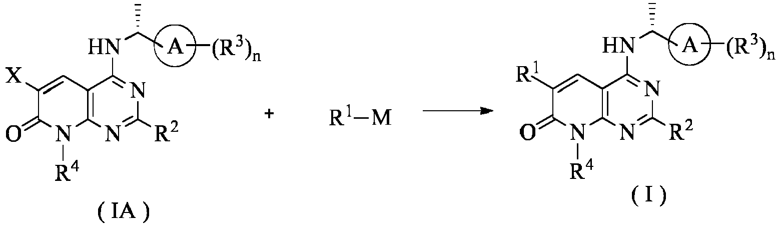 subjecting a compound of general formula (IA) or a tautomer, mesomer, racemate, enantiomer or diastereomer thereof or a mixture thereof, or a pharmaceutically acceptable salt thereof to a reaction with R¹-M under alkaline conditions and in the presence of a catalyst, to give a compound of general formula (I) or a tautomer, mesomer, racemate, enantiomer or diastereomer thereof or a mixture thereof, or a pharmaceutically acceptable salt thereof; wherein:
X is halogen, preferably Br;
M is selected from the group consisting of 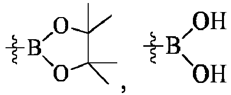 and hydrogen;
ring A, R¹ to R⁴ and n are as defined in general formula (I).

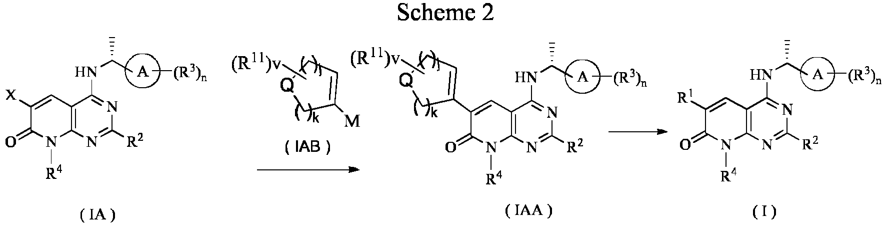

Subjecting a compound of general formula (IA) or a tautomer, mesomer, racemate, enantiomer or diastereomer thereof or a mixture thereof, or a pharmaceutically acceptable salt thereof to a reaction with a compound of general formula (IAB) under alkaline conditions and in the presence of a catalyst, to give a compound of general formula (IAA) or a tautomer, mesomer, racemate, enantiomer or diastereomer thereof or a mixture thereof, or a pharmaceutically acceptable salt thereof;
subjecting a compound of general formula (IAA) or a tautomer, mesomer, racemate, enantiomer or diastereomer thereof or a mixture thereof, or a pharmaceutically acceptable salt thereof to an oxidation reaction in the presence of a catalyst and an oxidant, to give a compound of general formula (I) or a tautomer, mesomer, racemate, enantiomer or diastereomer thereof or a mixture thereof, or a pharmaceutically acceptable salt thereof;
wherein:
X is halogen, preferably Br;
M is selected from the group consisting of 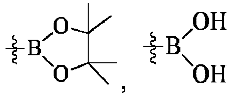 and hydrogen;
Q is selected from the group consisting of oxygen, sulfur, 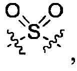 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
R¹ is 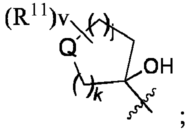
j is 0, 1 or 2;
k is 1 or 2;
v is 0, 1, 2 or 3;
ring A, R² to R⁴, R⁷ to R¹⁰, p, q and n are as defined in the compound of general formula (I).

### Scheme 3

Provided is a method for preparing the compound of general formula (II) or the salt thereof of the present disclosure, or a method for preparing the tautomer, mesomer, racemate, enantiomer or diastereomer thereof or the mixture thereof, or the pharmaceutically acceptable salt thereof, which comprises the following step: 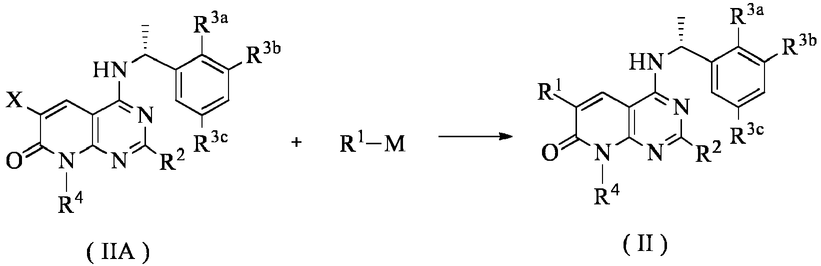 subjecting a compound of general formula (IIA) or a tautomer, mesomer, racemate, enantiomer or diastereomer thereof or a mixture thereof, or a pharmaceutically acceptable salt thereof to a reaction with R¹-M under alkaline conditions and in the presence of a catalyst, to give a compound of general formula (II) or a tautomer, mesomer, racemate, enantiomer or diastereomer thereof or a mixture thereof, or a pharmaceutically acceptable salt thereof; wherein:
X is halogen, preferably Br;
M is selected from the group consisting of 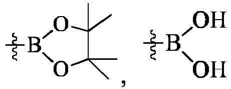 and hydrogen;
R¹, R², R^{3a}, R^{3b}, R^{3c} and R⁴ are as defined in the compound of general formula (II).

### Scheme 4

Another aspect of the present disclosure relates to a method for preparing a compound of general formula (IIAA) or a tautomer, mesomer, racemate, enantiomer or diastereomer thereof or a mixture thereof, or a pharmaceutically acceptable salt thereof, which comprises the following step:

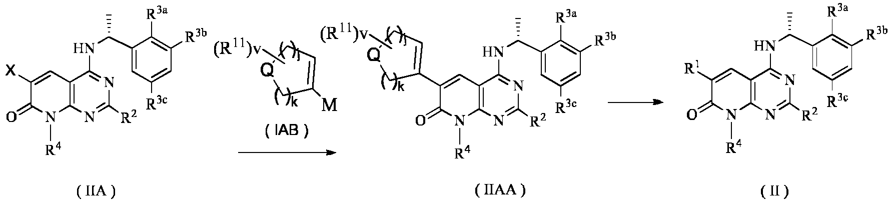

Subjecting a compound of general formula (IIA) or a tautomer, mesomer, racemate, enantiomer or diastereomer thereof or a mixture thereof, or a pharmaceutically acceptable salt thereof to a reaction with a compound of general formula (IAB) under alkaline conditions and in the presence of a catalyst, to give a compound of general formula (IIAA) or a tautomer, mesomer, racemate, enantiomer or diastereomer thereof or a mixture thereof, or a pharmaceutically acceptable salt thereof;
subjecting a compound of general formula (IIAA) or a tautomer, mesomer, racemate, enantiomer or diastereomer thereof or a mixture thereof, or a pharmaceutically acceptable salt thereof to an oxidation reaction in the presence of a catalyst and an oxidant, to give a compound of general formula (I) or a tautomer, mesomer, racemate, enantiomer or diastereomer thereof or a mixture thereof, or a pharmaceutically acceptable salt thereof;
wherein:
X is halogen, preferably Br;
M is selected from the group consisting of 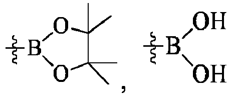 and hydrogen;
Q is selected from the group consisting of oxygen, sulfur, 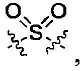 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
R¹ is 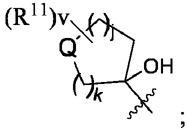
j is 0, 1 or 2;
k is 1 or 2;
v is 0, 1, 2 or 3;
R², R^{3a}, R^{3b}, R^{3c}, R⁴, R⁷ to R¹⁰, p and q are as defined in the compound of general formula (II).

The reagents that provide alkaline conditions in Schemes 1 to 4 include organic and inorganic bases, wherein the organic bases include, but are not limited to, triethylamine, *N*,*N*-diisopropylethylamine, *n*-butyllithium, lithium diisopropylamide, potassium acetate, sodium *tert*-butoxide, potassium *tert*-butoxide and 1,8-diazabicycloundec-7-ene; and the inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide, preferably sodium carbonate. The catalysts used in Schemes 1 to 4 include, but are not limited to, tetrakis(triphenylphosphine)palladium(0), palladium dichloride, palladium acetate, [1,1 '-bis(diphenylphosphino)ferrocene]palladium(II) dichloride dichloromethane complex, [1,1'-bis(dibenzylphosphino)ferrocene]palladium(II) dichloride, tris(dibenzylideneacetone)dipalladium(0), cuprous iodide/*L*-proline, and the like. When M is 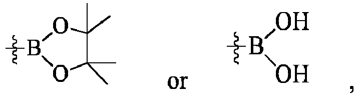 the preferred catalyst is [1,1 '-bis(diphenylphosphino)ferrocene]palladium(II) dichloride dichloromethane complex; and when M is a hydrogen atom, the preferred catalyst is cuprous iodide/*L*-proline.

The catalyst systems used in the oxidation reactions of Schemes 2 and 4 include, but are not limited to, PhSiH/Mn(dpm)₂ (or Mn(dpm)₃ or Mn(acac)₂ or Mn(TMHD)₃ or Co(sdmg)₃), tetraphenylporphyrin manganese(III) complex/NaBH₄ (or Pt-H₂), tetraphenylporphyrin cobalt(II) complex/NaBH₄ (or EtNBH₄), (bis(salicyl-γ-iminopropyl)methylamine)cobalt(II)/primary alcohol, Co(acac)₂, Co(salen), Co(acacen), and BH₃; and the oxidants used include, but are not limited to, oxygen, air, hydrogen peroxide, and the like;
wherein Mn(dpm)₂ is manganese bis(2,2,6,6-tetramethyl-3,5-heptanedionate), Mn(dpm)₃ is manganese tris(2,2,6,6-tetramethyl-3,5-heptanedionate) (CAS registry number: 14324-99-3, also known as tris(2,2,6,6-tetramethyl-3,5-heptanedionato)manganese), Mn(acac)₂ is manganese(II) bis(acetylacetonate) (CAS registry number: 14024-58-9, also known as manganese acetylacetonate), Mn(TMHD)₃ is tris(dipivaloylmethanato)manganese (CAS 14324-99-3), Co(acac)₂ is cobalt(II) bis(acetylacetonate) (CAS registry number: 193620-63-2), Co(salen) is *N*,*N'*-bis(salicylidene)ethylenediamine cobalt(II) (CAS registry number: 14167-18-1), Co(acacen) is cobalt (II) *N*,*N'*-bis(acetylacetonato)ethylenediamine, Co(sdmg)₃ is sodium bis(*N*-salicylidene-2-aminoisobutyrone)cobaltate (CAS registry number: 704900-51-6). The above reactions are preferably performed in solvents including, but not limited to acetic acid, methanol, ethanol, acetonitrile, *n*-butanol, toluene, tetrahydrofuran, dichloromethane, petroleum ether, ethyl acetate, n-hexane, dimethyl sulfoxide, 1,4-dioxane, ethylene glycol dimethyl ether, water, *N*,*N*-dimethylacetamide, *N*,*N*-dimethylformamide and mixtures thereof.

### Scheme 5

Provided is a method for preparing the compound of general formula (I) or the salt thereof of the present disclosure, or a method for preparing the tautomer, mesomer, racemate, enantiomer or diastereomer thereof or the mixture thereof, or the pharmaceutically acceptable salt thereof, which comprises the following step: 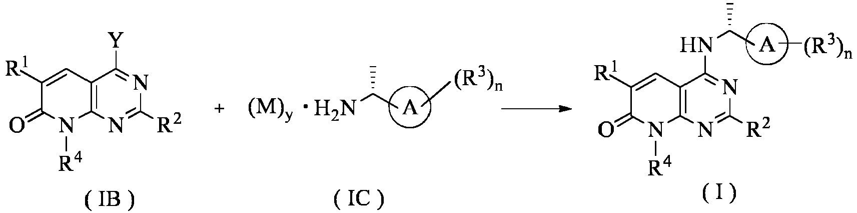 subjecting a compound of general formula (IB) or a tautomer, mesomer, racemate, enantiomer or diastereomer thereof or a mixture thereof, or a pharmaceutically acceptable salt thereof to a reaction with a compound of general formula (IC) under alkaline conditions to give a compound of general formula (I) or a tautomer, mesomer, racemate, enantiomer or diastereomer thereof or a mixture thereof, or a pharmaceutically acceptable salt thereof;
wherein:
M is HCl;
y is 0 or 1;
Y is halogen, preferably Cl;
ring A, R¹ to R⁴ and n are as defined in general formula (I).

### Scheme 6

Provided is a method for preparing the compound of general formula (II) or the salt thereof of the present disclosure, or a method for preparing the tautomer, mesomer, racemate, enantiomer or diastereomer thereof or the mixture thereof, or the pharmaceutically acceptable salt thereof, which comprises the following step: 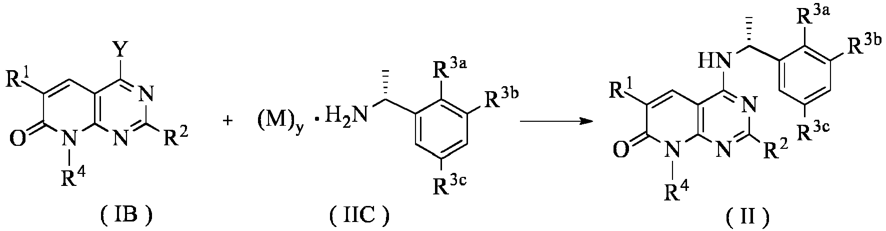 subjecting a compound of general formula (IB) or a tautomer, mesomer, racemate, enantiomer or diastereomer thereof or a mixture thereof, or a pharmaceutically acceptable salt thereof to a reaction with a compound of general formula (IIC) under alkaline and microwave conditions to give a compound of general formula (II) or a tautomer, mesomer, racemate, enantiomer or diastereomer thereof or a mixture thereof, or a pharmaceutically acceptable salt thereof;
wherein:
M is HCl;
y is 0 or 1;
Y is halogen, preferably Cl;
R¹, R², R^{3a}, R^{3b}, R^{3c} and R⁴ are as defined in the compound of general formula (II).

The reagents that provide alkaline conditions in Schemes 5 and 6 include organic and inorganic bases, wherein the organic bases include, but are not limited to, triethylamine, N,N-diisopropylethylamine, n-butyllithium, lithium diisopropylamide, potassium acetate, sodium *tert*-butoxide, potassium *tert*-butoxide and 1,8-diazabicycloundec-7-ene; and the inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide, preferably *N*,*N*-diisopropylethylamine.

The above reactions are preferably performed in solvents including, but not limited to acetic acid, methanol, ethanol, acetonitrile, *n*-butanol, toluene, tetrahydrofuran, dichloromethane, petroleum ether, ethyl acetate, n-hexane, dimethyl sulfoxide, 1,4-dioxane, ethylene glycol dimethyl ether, water, *N*,*N*-dimethylacetamide, *N*,*N*-dimethylformamide and mixtures thereof.

### DETAILED DESCRIPTION

The present disclosure is further described below with reference to examples below, which are not intended to limit the scope of the present disclosure.

### Examples

The structure of the compound is determined by nuclear magnetic resonance (NMR) spectroscopy and/or mass spectrometry (MS). NMR shift (δ) is given in a unit of 10⁻⁶ (ppm). NMR spectra are determined using a Bruker AVANCE-400 nuclear magnetic resonance instrument or Bruker AVANCE NEO 500M, with deuterated dimethyl sulfoxide (DMSO-*d₆*), deuterated chloroform (CDCl₃) and deuterated methanol (CD₃OD) as determination solvents and tetramethylsilane (TMS) as an internal standard.

MS data are determined using Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) or THERMO Ultimate 3000-Q Exactive (manufacturer: THERMO, MS model: THERMO Q Exactive).

High performance liquid chromatography (HPLC) analysis is performed using Agilent HPLC 1200DAD, Agilent HPLC 1200VWD and Waters HPLC e2695-2489 high performance liquid chromatographs.

Chiral HPLC analysis is performed using an Agilent 1260 DAD high performance liquid chromatograph.

High performance liquid preparative chromatography is performed using Waters 2545-2767, Waters 2767-SQ Detecor2, Shimadzu LC-20AP and Gilson GX-281 preparative chromatographs.

Chiral preparative HPLC is performed using a Shimadzu LC-20AP preparative chromatograph.

CombiFlash rapid preparation instrument used is Combiflash Rf200 (TELEDYNE ISCO).

Huanghai HSGF254 or Qingdao GF254 silica gel plates of specifications 0.15 mm to 0.2 mm are adopted for thin layer chromatography (TLC) analysis and 0.4 mm to 0.5 mm for TLC separation and purification.

Yantai Huanghai silica gel of 200-300 mesh is generally used as a carrier in column chromatography.

The mean inhibition of kinase and the IC₅₀ value are determined using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor is used in the microwave reactions.

In the examples, a solution refers to an aqueous solution unless otherwise specified.

In the examples, the reaction temperature is room temperature, i.e., 20 °C to 30 °C, unless otherwise specified.

The monitoring of the reaction progress in the examples is conducted by thin layer chromatography (TLC). The developing solvent for reactions, the eluent system for column chromatography purification and the developing solvent system for thin layer chromatography include: A: dichloromethane/methanol system, B: *n*-hexane/ethyl acetate system, and C: petroleum ether/ethyl acetate system. The volume ratio of the solvents is adjusted according to the polarity of the compound, or by adding a small amount of basic or acidic reagents such as triethylamine and acetic acid.

### Example 1

### (R)-4-((1-(3-(1,1-difluoro-2-hydroxyethyl)-2-fluorophenyl)ethyl)amino)-6-(3,6-dihydro-2H-pyran-4-yl)-2-methylpyrido[2,3-d]pyrimidin-7(8H)-one 1

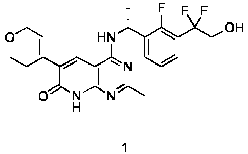

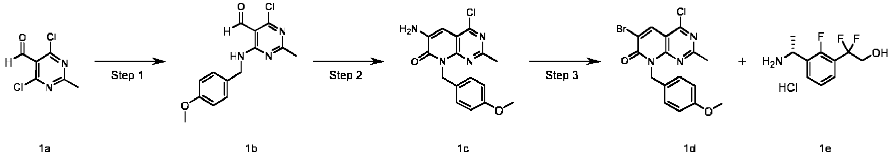

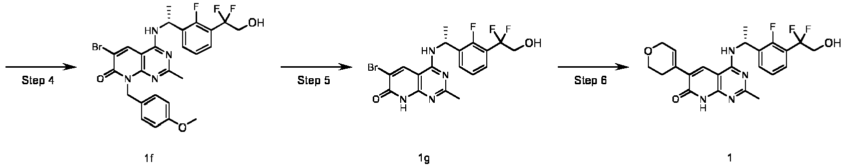

### Step 1

4-chloro-6-((4-methoxybenzyl)amino)-2-methylpyrimidine-5-carbaldehyde **1b** The compound 4,6-dichloropyrimidine-2-methyl-5-carbaldehyde **1a** (2 g, 10.47 mmol), 4-methoxybenzylamine (1.5 g, 10.95 mmol) and triethylamine (2.11 g, 20.85 mmol) were dissolved in 40 mL of dichloromethane, and the solution was stirred for 2 h under an ice bath. The reaction solution was concentrated under reduced pressure to give a crude product **1b** (3 g, yield: 98.2%), which was directly used in the next step without purification.

MS m/z (ESI): 292.0 [M+1].

### Step 2

6-amino-4-chloro-8-(4-methoxybenzyl)-2-methylpyrido[2,3-*d*]pyrimidin-7(8*H*)-one **1c** 4-methoxybenzaldehyde (700 mg, 5.14 mmol), glycine methyl ester hydrochloride (645 mg, 5.14 mmol) and triethylamine (1.2 g, 11.86 mmol) were dissolved in 6 mL of methanol, and the solution was stirred for 8 h, followed by the addition of compound **1b** (1.5 g, 5.14 mmol). After the mixture was stirred for 14 h, 6 mL of 70% acetic acid was added, and the resulting mixture was reacted at 45 °C for 14 h. The reaction solution was cooled to room temperature, neutralized to pH 8 with a saturated sodium bicarbonate solution, extracted with ethyl acetate (30 mL × 2), dried over anhydrous sodium sulfate, filtered and concentrated under reduced pressure, and the residue was purified by column chromatography with an eluent system C to give the title compound **1c** (260 mg, yield: 15.3%).

MS m/z (ESI): 331.1 [M+1].

### Step 3

6-bromo-4-chloro-8-(4-methoxybenzyl)-2-methylpyrido[2,3-*d*]pyrimidin-7(8*H*)-one **1d** Cuprous bromide (234.2 mg, 1.63 mmol) and isoamyl nitrite (191.2 mg, 1.63 mmol) were dissolved in 5 mL of *N*,*N*-dimethylformamide, and the solution was stirred for 0.5 h, followed by the addition of compound **1c** (180 mg, 0.54 mmol). The mixture was stirred for 14 h. The reaction was quenched with water, and the reaction solution was extracted with ethyl acetate (10 mL × 3), dried over anhydrous sodium sulfate, filtered and concentrated, and purified by column chromatography with an eluent system C to give the title compound **1d** (140 mg, yield: 65.1%).

MS m/z (ESI): 394.0[M+1].

### Step 4

### (R)-6-bromo-4-((1-(3-(1,1-difluoro-2-hydroxyethyl)-2-fluorophenyl)ethyl)amino)-8-(4-methoxybenzyl)-2-methylpyrido[2,3-d]pyrimidin-7(8H)-one 1f

Compound **1d** (60 mg, 0.15 mmol), compound (*R*)-2-(3-(1-aminoethyl)-2-fluorophenyl)-2,2-difluoroethanol hydrochloride 1e (58 mg, 0.23 mmol, prepared by the method disclosed in Example B-5 on page 105 of the specification in the patent application "US20190194192A1"), and N,N-diisopropylethylamine (370 mg, 2.86 mmol) were dissolved in 2 mL of *N*,*N*-dimethylacetamide, and the solution was reacted at 120 °C for 2 h under a microwave condition. The reaction solution was cooled and concentrated under reduced pressure, and the residue was purified by thin layer chromatography with developing solvent system C to give the title compound **1f** (60 mg, yield: 68.3%).

MS m/z (ESI): 577.1 [M+1].

### Step 5

### (R)-6-bromo-4-((1-(3-(1,1-difluoro-2-hydroxyethyl)-2-fluorophenyl)ethyl)amino)-2-met hylpyrido[2,3-d]pyrimidin-7(8H)-one 1g

Compound **1f** (60 mg, 103.9 µmol) was dissolved in 2 mL of trifluoroacetic acid, and the solution was reacted at 72 °C for 0.5 h under a microwave condition. The reaction solution was concentrated to give the title compound **1g** (47 mg, yield: 98.9%).

MS m/z (ESI): 456.1 [M+1].

### Step 6

### (R)-4-((1-(3-(1,1-difluoro-2-hydroxyethyl)-2-fluorophenyl)ethyl)amino)-6-(3,6-dihydro-2H-pyran-4-yl)-2-methylpyrido[2,3-d]pyrimidin-7(8H)-one 1

Compound **1g** (47 mg, 102.7 µmol), compound 2-(3,6-dihydro-2*H-*pyran-4-yl)-4,4,5,5-tetramethyl-1,3,2-dioxaborolan (34 mg, 161.8 µmol, Accela ChemBio Co., Ltd.), [1,1'-bis(diphenylphosphino)ferrocene]palladium(II) dichloride dichloromethane complex (13mg, 15.9 µmol) and anhydrous sodium carbonate (46 mg, 434 µmol) were dissolved in 2 mL of dioxane and 0.4 mL of water, and the mixture was purged with argon 3 times, and reacted at 80 °C for 1 h under a microwave condition. The reaction solution was cooled to room temperature and filtered through celite, and the filtrate was concentrated under reduced pressure. The residue was purified by high performance liquid preparative chromatography to give the title compound 1 (15 mg, yield: 29.7%).

MS m/z (ESI): 461.1[M+1].

¹H NMR (500 MHz, CD₃OD) δ 8.21 (s, 1H), 7.56 (td, 1H), 7.45 (td, 1H), 7.21 (t, 1H), 6.64 (tt, 1H), 5.80 (q, 1H), 4.32 (q, 2H), 4.03 (td, 2H), 3.93 (t, 2H), 2.57 (dtd, 2H), 2.35 (s, 3H), 1.64 (d, 3H).

### Example 2

### (R)-6-(azetidin-1-yl)-4-((1-(3-(difluoromethyl)-2-fluorophenyl)ethyl)amino)-2-methylp yrido[2,3-d]pyrimidin-7(8H)-one 2

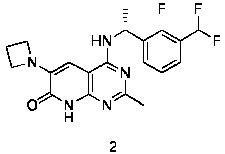

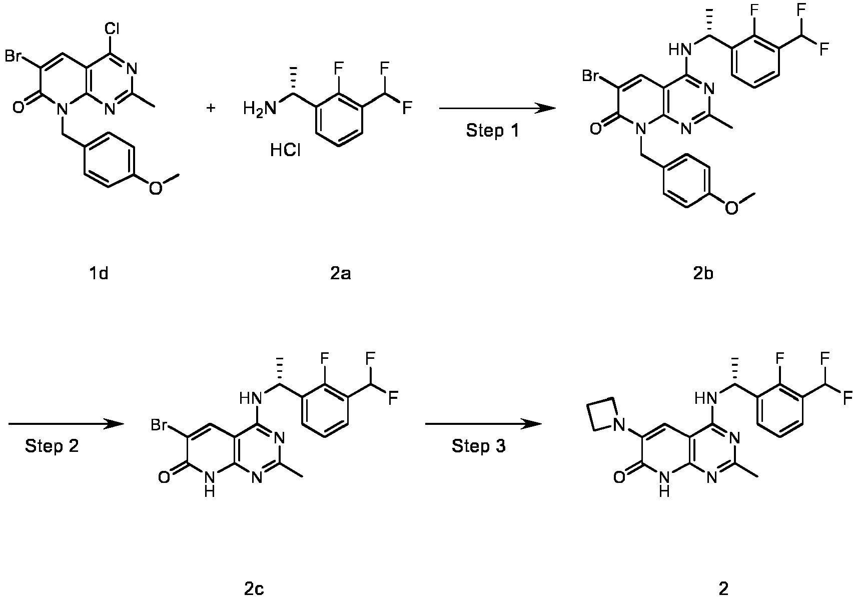

### Step 1

### (R)-6-bromo-4-((1 -(3 -(difluoromethyl)-2-fluorophenyl)ethyl)amino)-8-(4-methoxybenz yl)-2-methylpyrido[2,3-d]pyrimidin-7(8H)-one 2b

Compound **1d** (130 mg, 0.33 mmol), compound (*R*)-1-(3-(difluoromethyl)-2-fluorophenyl)ethylamine hydrochloride **2a** (111.5 mg, 0.49 mmol, prepared by the method disclosed in Example B-5 on page 141 of the specification in the patent application "WO2019122129A1"), and *N*,*N*-diisopropylethylamine (638.6 mg, 4.94 mmol) were dissolved in 2 mL of *N*,*N*-dimethylacetamide, and the solution was reacted at 120 °C for 2 h under a microwave condition. The reaction solution was cooled and concentrated under reduced pressure, and the residue was purified by thin layer chromatography with developing solvent system C to give the title compound **2b** (105 mg, yield: 58.2%). MS m/z (ESI): 547.2 [M+1].

### Step 2

### (R)-6-bromo-4-((1-(3-(difluoromethyl)-2-fluorophenyl)ethyl)amino)-2-methylpyrido[2, 3-d]pyrimidin-7(8H)-one 2c

Compound **2b** (40 mg, 73.1 µmol) was dissolved in 2 mL of trifluoroacetic acid, and the solution was reacted at 72 °C for 0.5 h under microwave irradiation. The reaction solution was concentrated to give the title compound **2c** (30 mg, yield: 96.2%).

MS m/z (ESI): 427.1 [M+1].

### Step 3

### (R)-6-(azetidin-1-yl)-4-((1-(3-(difluoromethyl)-2-fluorophenyl)ethyl)amino)-2-methylp yrido[2,3-d]pyrimidin-7(8H)-one 2

Compound **2c** (30 mg, 70.2 µmol) and compound azetidine (12 mg, 210.7 µmol) were dissolved in 2 mL of dimethyl sulfoxide, and cuprous iodide (13.4 mg, 70.2 µmol), *L*-proline (8.1 mg, 70.2 µmol), and anhydrous potassium phosphate (44.7 mg, 210.6 µmol) were added sequentially, purged with nitrogen three times, heated to 110 °C and reacted for 14 h. The reaction solution was cooled and filtered, and the filtrate was concentrated under reduced pressure. The residue was purified by high performance liquid preparative chromatography to give the title compound **2** (5 mg, yield: 17.6%).

MS m/z (ESI): 404.2 [M+1].

¹H NMR (500 MHz, CD₃OD) δ 7.55 (t, 1H), 7.45 (t, 1H), 7.21 (t, 1H), 7.00 (t, 1H), 5.77 (d, 1H), 4.06 (t, 3H), 2.34 (t, 2H), 2.30 (t, 3H), 2.18-2.15 (m, 1H), 2.04-2.02(m, 1H), 1.62 (d, 3H).

### Example 3

### (R)-4-((1-(3-amino-5-(trifluoromethyl)phenyl)ethyl)amino)-6-(3,6-dihydro-2H-pyran-4-yl)-2-methylpyrido[2,3-d]pyrimidin-7(8H)-one 3

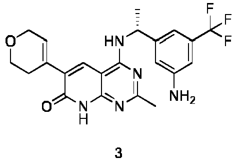

Compound **3** (2 mg, yield: 4.2%) was obtained by following the synthetic route described in Example **1** with the starting compound **1e** in the step 3 replaced by the compound (*R*)-1-(3-amino-5-(trifluoromethyl)aniline hydrochloride (prepared by the method disclosed in Example B-6n on page 106 of the specification in the patent application "WO2018115380A1").

MS m/z (ESI): 446.1 [M+1].

¹H NMR (500 MHz, CD₃OD): δ 8.19 (s, 1H), 6.98-6.90(m, 2H), 6.82(s, 1H), 6.64(s, 1H), 5.58-5.55(m, 1H), 4.34-4.32(m, 2H), 3.95-3.92(m, 2H), 2.59-2.57(m, 2H), 2.41(s, 3H), 1.62(d, 3H).

### Example 4

### (R)-6-(1-acetyl-1,2,3,6-tetrahydropyridin-4-yl)-4-((1-(3-amino-5-(trifluoromethyl)pheny l)ethyl)amino)-2-methylpyrido[2,3-d]pyrimidin-7(8H)-one 4

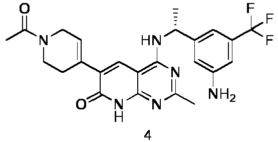

Compound **4** (5 mg, yield: 10.6%) was obtained by following the synthetic route described in Example **1** with the starting compound **1e** in the step 3 replaced by the compound (*R*)-1-(3-amino-5-(trifluoromethyl)aniline hydrochloride (prepared by the method disclosed in Example B-6n on page 106 of the specification in patent application "WO2018115380A1"), and with the compound 2-(3,6-dihydro-2*H-*pyran-4-yl)-4,4,5,5-tetramethyl-1,3,2-dioxaborolan in the step 6 replaced by the compound 1-(4-(4,4,5,5-tetramethyl-1,3,2-dioxaborolan-2-yl)-5,6-dihydropyridin-1(2*H*)-yl)ethano ne.

MS m/z (ESI): 487.1 [M+1].

¹H NMR (500 MHz, CD₃OD): δ 8.20 (s, 1H), 6.98-6.95(m, 2H), 6.82(s, 1H), 6.38(s, 1H), 5.58-5.55(m, 1H), 4.25-4.22(m, 2H), 3.81-3.73(m, 2H), 2.68-2.66(m, 2H), 2.41(s, 3H), 2.19(d, 3H), 1.62(d, 3H).

### Example 5

### (R)-4-((1-(3-(difluoromethyl)-2-fluorophenyl)ethyl)amino)-6-(3,6-dihydro-2H pyran-4-yl)-2-methylpyrido[2,3-d]pyrimidin-7(8H)-one 5

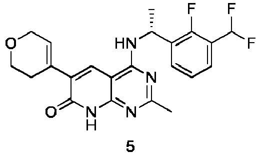

Compound **5** (5 mg, yield: 17.7%) was obtained by following the synthetic route in Example **1** with the starting compound **1e** in the step 3 replaced by compound **2a.**

MS m/z (ESI): 431.1 [M+1].

¹H NMR (500 MHz, CD₃OD): δ 8.22 (s, 1H), 7.61-7.59(m, 1H),7.59-7.58(m, 1H), 7.27-7.25(m, 1H), 7.02(s, 1H), 6.75-6.72(m, 1H), 5.79-5.78(m, 1H), 4.35-4.34(m, 2H), 3.95-3.94(m, 2H), 2.59-2.56(m, 2H), 2.35(s, 3H), 1.66(d, 3H).

### Example 6

### (R)-4-((1-(3-(difluoromethyl)-2-fluorophenyl)ethyl)amino)-2-methyl-6-(tetrahydro-2H-pyran-4-yl)pyrido[2,3-d]pyrimidin-7(8H)-onc 6

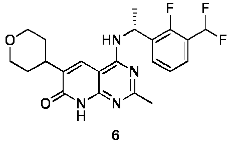

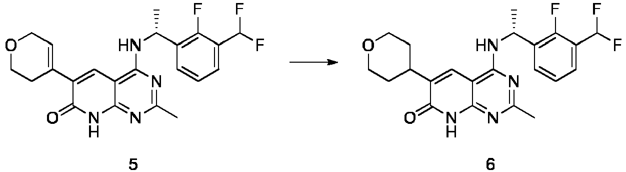

Compound 5 (50 mg, 116 µmol) was dissolved in 10 mL of methanol, and 10% palladium on carbon catalyst (50 mg) was added. The mixture purged with hydrogen 3 times and stirred for 16 h. The reaction solution was filtered through celite, and the filtrate was dried by rotary evaporation. The residue was purified by high performance liquid preparative chromatography to give the title compound 6 (10 mg, yield: 19.9%).

MS m/z (ESI): 433.1 [M+1].

¹H NMR (500 MHz, CD₃OD): δ 8.16(s, 1H), 7.61-7.58(m, 1H), 7.50-7.47(m, 1H), 7.27-7.25(m, 1H), 7.02(s, 1H), 5.82-5.78(m, 1H), 4.10-4.07(m, 2H), 3.64-3.59(m, 2H), 3.13-3.11(m, 1H), 2.35(s, 3H), 1.88-1.84(m, 2H), 1.79-1.75(m, 2H), 1.67(d, 3H).

### Example 7

### (R)-6-(1-acetyl-1,2,3,6-tetrahydropyridin-4-yl)-4-((1-(3-(1,1-difluoro-2-hydroxyethyl)-2 -fluorophenyl)ethyl)amino)-2-methylpyrido[2,3-d]pyrimidin-7(8H)-one 7

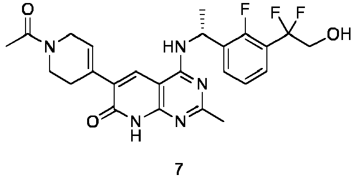

Compound 7 (15 mg, yield: 17.3%) was obtained by following the synthetic route described in Example **1** with the compound 2-(3,6-dihydro-2H pyran-4-yl)-4,4,5,5-tetramethyl-1,3,2-dioxaborolan in the step 6 replaced by the compound 1-(4-(4,4,5,5-tetramethyl-1,3,2-dioxaborolan-2-yl)-5,6-dihydropyridin-1(2*H*)-yl)ethano ne.

MS m/z (ESI): 502.1 [M+1].

¹H NMR (500 MHz, CD₃OD) δ 8.23 (d, 1H), 7.57 (t, 1H), 7.50-7.43 (m, 1H), 7.22 (t, 1H), 6.58-6.35 (m, 1H), 5.81 (q, 1H), 4.24 (dq, 2H), 4.04 (td, 2H), 3.81 (t, 5.8 1H), 3.75 (t, 1H), 2.68 (s, 1H), 2.60 (s, 1H), 2.37 (s, 3H), 2.18 (d, 3H), 1.65 (dd, 3H).

### Example 8

### (R)-4-((1-(3-(difluoromethyl)-2-fluorophenyl)ethyl)amino)-2-methyl-6-(1-methyl-1H-p yrazol-4-yl)pyrido[2,3-d]pyrimidin-7(8H)-one 8

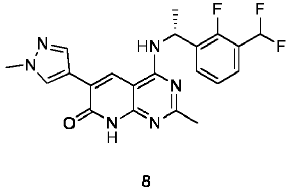

Compound **8** (10 mg, yield: 10%) was obtained by following the synthetic route in Example **1** with the starting compound **1e** in the step 3 replaced by compound **2a,** and with the compound 2-(3,6-dihydro-2*H*-pyran-4-yl)-4,4,5,5-tetramethyl-1,3,2-dioxaborolan in the step 6 replaced by the compound 1-methyl-1*H-*pyrazole-4-boronic acid.

MS m/z (ESI): 429.1 [M+1].

¹H NMR (500 MHz, CD₃OD) δ 8.64 (s, 1H), 8.34 (s, 1H), 8.12 (s, 1H), 7.62 (t, 1H), 7.49 (t, 1H), 7.26 (t, 1H), 7.14-6.92 (t, 1H), 5.81 (q, 1H), 3.96 (s, 3H), 2.36 (s, 3H), 1.69 (d, 3H).

### Example 9

### 4-(((R)-1-(3-(1,1-difluoro-2-hydroxyethyl)-2-fluorophenyl)ethyl)amino)-2-methyl-6-((( R)-tetrahydrofuran-3-yl)oxy)pyrido[2,3-d]pyrimidin-7(8H)-one 9

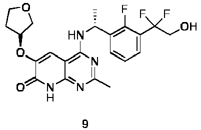

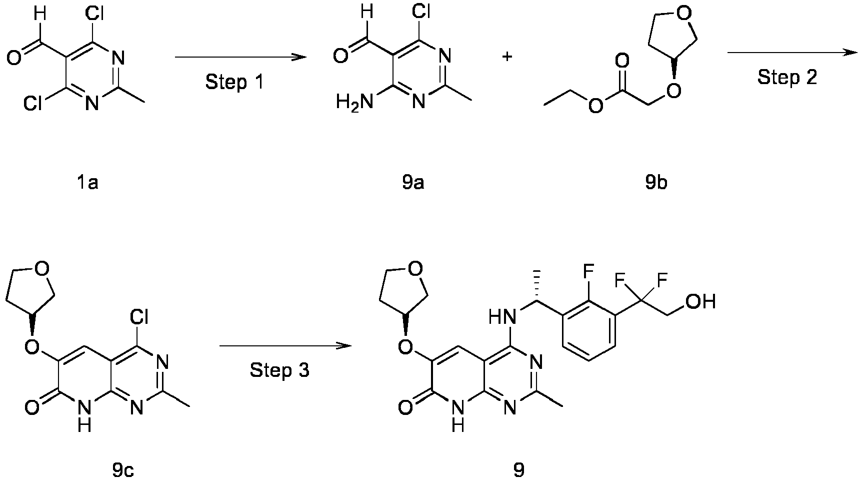

### Step 1

### 4-amino-6-chloro-2-methylpyrimidine-5-carbaldehyde 9a

Compound **1a** (700 mg, 3.66 mmol) was dissolved in 30 mL of ammonia in 1,4-dioxane, and the solution was stirred for 14 h. The reaction solution was filtered and concentrated to give a crude product **9a** (620 mg, yield: 98.6%) which was directly used in the next step without purification.

MS m/z (ESI): 172.1 [M+1].

### Step 2

### (R)-4-chloro-2-methyl-6-((tetrahydrofuran-3-yl)oxy)pyrido[2,3-d]pyrimidin-7(8H)-one 9c

Compound **9a** (380 mg, 2.2 mmol) and compound (*R*)-ethyl 2-((tetrahydrofuran-3-yl)oxy)acetate **9b** (482.2 mg, 2.77 mmol, prepared by the well-known method *"*ACS Medicinal Chemistry Letters, 2019, 10 (6), 996-1001") were dissolved in 5 mL of tetrahydrofuran, and potassium tert-butoxide (372.8 mg, 2.77 mmol) was added. The mixture was reacted at 70 °C for 14 h. The reaction solution was cooled to room temperature and filtered through celite, and the filtrate was concentrated under reduced pressure. The residue was purified by column chromatography with an eluent system A to give the title compound **9c** (110 mg, yield: 17.6%).

MS m/z (ESI): 282.1 [M+1].

### Step 3

### 4-(((R)-1-(3-(1,1-difluoro-2-hydroxyethyl)-2-fluorophenyl)ethyl)amino)-2-methyl-6-((( R)-tetrahydrofuran-3-yl)oxy)pyrido[2,3-d]pyrimidin-7(8H)-one 9

Compound **9c** (50 mg, 177.5 µmol), compound **1e** (50 mg, 195.2 mmol), and *N,N*-diisopropylethylamine (68.8 mg, 532.5 mmol) were dissolved in 2 mL of *N,N-*dimethylacetamide, and the solution was reacted at 120 °C for 2 h under microwave irradiation. The reaction solution was cooled and concentrated under reduced pressure, and the residue was purified by high performance liquid preparative chromatography to give the title compound **9** (13 mg, yield: 15.7%).

MS m/z (ESI): 465.1 [M+1].

¹H NMR (500 MHz, CD₃OD) δ 7.61 (s, 1H), 7.60-7.54 (m, 1H), 7.49-7.42 (m, 1H), 7.21 (t, 1H), 5.80 (q, 1H), 5.13 (ddt, 1H), 4.13-3.96 (m, 5H), 3.92 (td, 4.2 Hz, 1H), 2.36 (s, 3H), 2.30 (td, 1H), 2.27-2.21 (m, 1H), 1.66 (d, 3H).

### Example 10

### 4-(((R)-1-(3-(difluoromethyl)-2-fluorophenyl)ethyl)amino)-2-methyl-6-(((S)-tetrahydrof uran-3-yl)oxy)pyrido[2,3-d]pyrimidin-7(8H)-one 10

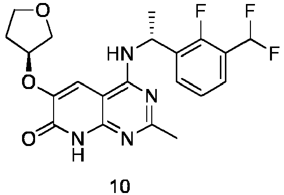

Compound **10** (31 mg, yield: 42%) was obtained by following the synthetic route in Example **9** with the starting compound **1e** in the step 3 replaced by compound **2a.**

MS m/z (ESI): 435.1 [M+1].

1H NMR (500 MHz, CD₃OD ): δ 7.61-7.59 (m, 2H), 7.50-7.47 (m, 1H), 7.26-7.23 (m, 1H), 7.13-6.91(t, 1H), 5.79-5.78 (m, 1H), 5.14(m, 1H), 4.06-3.92 (m, 4H), 2.34 (s, 3H), 2.32-2.21 (m, 2H), 1.67-1.66 (d, 3H).

### Example 11

### 4-(((R)-1-(3-(1,1-difluoro-2-hydroxyethyl)-2-fluorophenyl)ethyl)amino)-6-((1s,4S)-4-hy droxycyclohexyl)-2-methylpyrido[2,3-d]pyrimidin-7(8H)-one 11-p1

### 4-(((R)-1-(3-(1,1-difluoro-2-hydroxyethyl)-2-fluorophenyl)ethyl)amino)-6-((1r,4R)-4-h ydroxycyclohexyl)-2-methylpyrido[2,3-d]pyrimidin-7(8H)-one 11-p2

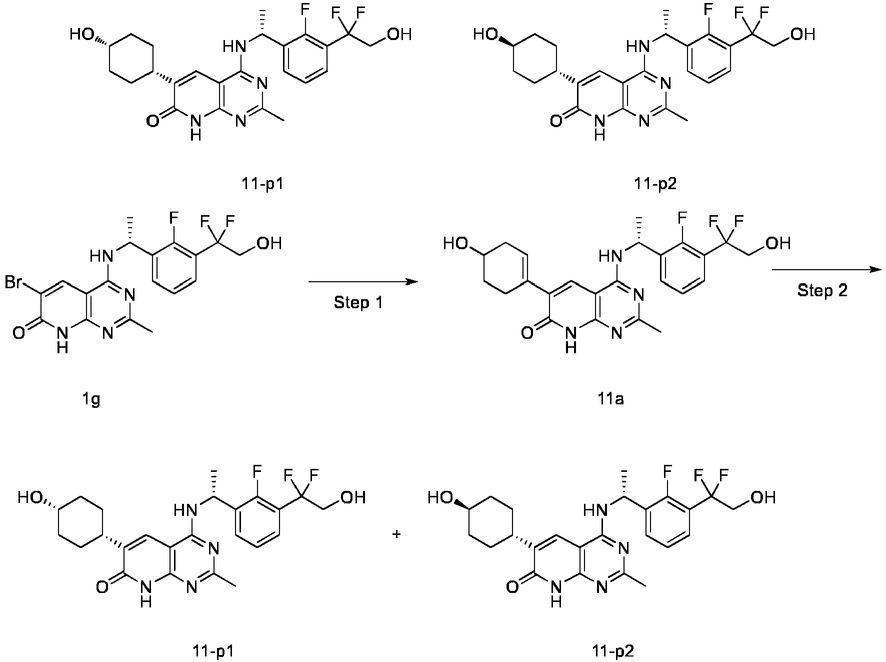

### Step 1

### 4-(((R)-1-(3-(1,1-difluoro-2-hydroxyethyl)-2-fluorophenyl)ethyl)amino)-6-(4-hydroxyc yclohex-1-en-1-yl)-2-methylpyrido[2,3-d]pyrimidin-7(8H)-onc 11a

Compound **1g** (100 mg, 284.3 µmol), 3,6-dihydro-4-(4,4,5,5-tetramethyl-1,3,2-dioxaborolan-2-yl)-2*H*-pyran (76.5 mg, 341.2 µmol, Accela ChemBio), [1,1'-bis(diphenylphosphino)ferrocene]palladium(II) dichloride dichloromethane complex (23.1 mg, 28.4 µmol) and anhydrous sodium carbonate (55 mg, 518.3 µmol) were dissolved in 5 mL of dioxane and 1 mL of water, and the solution was purged with argon three times and reacted at 80 °C for 1 h under microwave irradiation. The reaction solution was cooled to room temperature and filtered through celite, and the filtrate was concentrated under reduced pressure. The residue was purified by column chromatography with an eluent system A to give the title compound **11a** (90 mg, yield: 66.7%).

MS m/z (ESI): 475.1[M+1].

### Step 2

### 4-(((R)-1-(3-(1,1-difluoro-2-hydroxyethyl)-2-fluorophenyl)ethyl)amino)-6-((1s,4S)-4-hy droxycyclohexyl)-2-methylpyrido[2,3-d]pyrimidin-7(8H)-one 11-p1

### 4-(((R)-1-(3-(1,1-difluoro-2-hydroxyethyl)-2-fluorophenyl)ethyl)amino)-6-((1r,4R)-4-h ydroxycyclohexyl)-2-methylpyrido[2,3-d]pyrimidin-7(8H)-one 11-p2

Compound **11a** (40 mg, 84.3 mmol) was dissolved in methanol (10 mL), and 10% palladium on carbon catalyst (30 mg) was added. The mixture was purged with hydrogen three times and stirred for 16 h. The reaction solution was filtered through celite, and the filtrate was concentrated under reduced pressure. The residue was purified by high performance liquid preparative chromatography to give the title compounds (5 mg and 8 mg, yield: 12.4% and 19.9%).

### Single-configuration compound (shorter retention time) (5 mg, 12.4%)

MS m/z (ESI): 477.2 [M+1].

HPLC analysis: retention time: 10.2 min, purity: 98.5% (chromatographic column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30% -95%).

¹H NMR (400 MHz, CD₃OD ): 8.15 (s, 1H),7.58-7.56 (m, 1H), 7.48-7.46 (m, 1H), 7.24-7.21 (m, 1H), 5.80 (q, 1H), 4.06-4.01 (m, 2H), 3.65-3.62 (m, 1H), 2.66-2.61 (m, 1H), 2.36(s, 3H), 2.11-2.09 (m, 2H), 2.00-1.98 (m, 2H), 1.65-1.45(m, 7H).

### Single-configuration compound (longer retention time) (8 mg, 19.9%):

MS m/z (ESI): 477.2 [M+1].

HPLC analysis: retention time: 10.89 min, purity: 97.2% (column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30%-95%).

¹H NMR (500 MHz, CD₃OD ): 8.15 (s, 1H),7.60-7.58 (m, 1H), 7.47-7.45 (m, 1H), 7.24-7.21 (m, 1H), 5.82(q, 1H), 4.10-4.04 (m, 3H), 2.91-2.87 (m, 1H), 2.36(s, 3H), 1.96-1.60(m, 11H).

### Example 12

### (R)-6-(1-acetyl-4-hydroxypiperidin-4-yl)-4-((1-(3-(difluoromethyl)-2-fluorophenyl)ethy 1)amino)-2-methylpyrido[2,3-d]pyrimidin-7(8H)-one 12

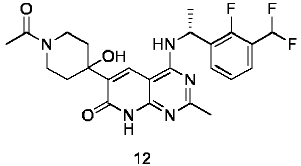

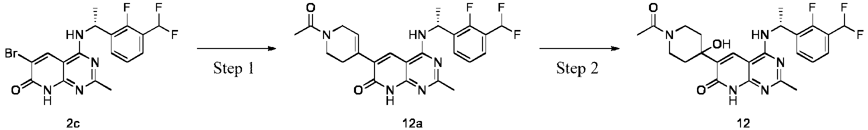

### Step 1

### (R)-6-(1-acetyl-1,2,3,6-tetrahydropyridin-4-yl)-4-((1-(3-(difluoromethyl)-2-fluoropheny l)ethyl)amino)-2-methylpyrido[2,3-d]pyrimidin-7(8H)-one 12a

Compound 2c (100 mg, 234.1 µmol), compound 1-(4-(4,4,5,5-tetramethyl-1,3,2-dioxaborolan-2-yl)-5,6-dihydropyridin-1(2*H*)-yl)ethano ne (71 mg, 282.7 µmol, Accela ChemBio), [1,1 '-bis(diphenylphosphino)ferrocene]palladium(II) dichloride dichloromethane complex (26 mg, 31.8 µmol) and anhydrous sodium carbonate (50 mg, 468 µmol) were dissolved in 5 mL of dioxane and 1 mL of water, and the solution was purged with argon 3 times and reacted at 80 °C for 1 h under microwave irradiation. The reaction solution was cooled to room temperature and filtered through celite, and the filtrate was concentrated under reduced pressure. The residue was purified by column chromatography with an eluent system A to give the title compound **12a** (100 mg, yield: 90.6%).

MS m/z (ESI): 472.1[M+1].

### Step 2

### (R)-6-(1-acetyl-4-hydroxypiperidin-4-yl)-4-((1-(3-(difluoromethyl)-2-fluorophenyl)ethy l)amino1)-2-methylpyrido[2,3-d]pyrimidin-7(8H)-one 12

Compound **12a** (100 mg, 212.1 µmol) was dissolved in dichloromethane (0.5 mL) and isopropanol (5 mL), and manganese acetylacetonate (27 mg, 106.7 µmol) and phenylsilane (31 mg, 286 µmol) were added. The mixture was purged with oxygen three times and stirred for 16 h. The reaction solution was concentrated, and the residue was purified by high performance liquid preparative chromatography to give the title compound **12** (30 mg, yield: 28.9%).

MS m/z (ESI): 490.1 [M+1].

1H NMR (500 MHz, CD₃OD ): δ 8.36 (s, 1H), 7.62-7.59 (m, 1H), 7.50-7.48 (m, 1H), 7.27-7.24 (m, 1H), 7.13-6.91(t, 1H), 5.83-5.78 (m, 1H), 4.52-4.49 (m, 1H), 3.87-3.84 (m, 1H), 3.68-3.63 (m, 1H), 3.18-3.12 (m, 1H), 2.36 (s, 3H), 2.34-2.19 (m, 2H), 2.17 (s, 3H), 1.96-1.82 (m, 2H), 1.67-1.65 (d, 3H).

### Example 13

### 4-(4-(((R)-1-(3-(difluoromethyl)-2-fluorophenyl)ethyl)amino)-2-methyl-7-oxo-7,8-dihy dropyrido[2,3-d]pyrimidin-6-yl)-N,N-dimethylcyclohex-3-ene-1-carboxamide 13

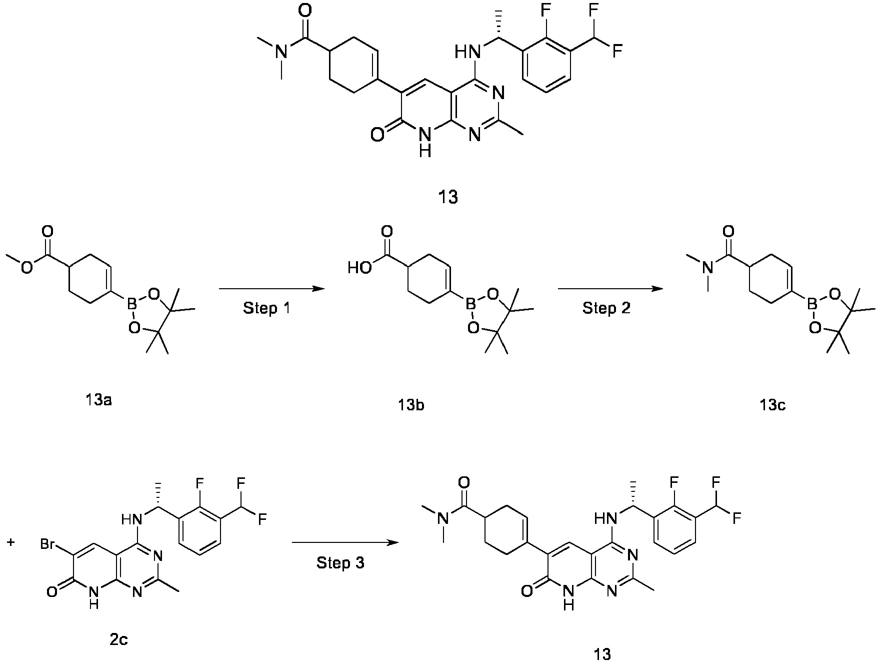

### Step 1

4-(4,4,5,5-tetramethyl-1,3,2-dioxaborolan-2-yl)cyclohex-3-eneacetic acid **13b** The compound methyl 4-(4,4,5,5-tetramethyl-1,3,2-dioxaborolan-2-yl)cyclohex-3-enecarboxylate **13a** (1 g, 3.76 mmol, Shanghai Bidepharm) and lithium hydroxide monohydrate (631 mg, 15 mmol) were dissolved in a mixed solvent of 10 mL of tetrahydrofuran, 2 mL of water and 5 mL of methanol, and the solution was stirred for 16 h. 2N hydrochloric acid was added dropwise to adjust pH to 5-6, and the reaction solution was concentrated to dryness under reduced pressure to give the title compound **13b** (1.8 g), which was directly used in the next step without purification.

MS m/z (ESI): 251.1 [M-1].

### Step 2

### N,N-dimethyl-4-(4,4,5,5-tetramethyl-1,3,2-dioxaborolan-2-yl)cyclohex-3-eneacetamide 13c

Compound **13b** (900 mg, 3.57 mmol) and dimethylamine hydrochloride (349 mg, 4.28 mmol) were dissolved in 25 mL of *N*,*N*-dimethylformamide, and 2-(7-azobenzotriazol)-*N*,*N*,*N*,*N*-tetramethyluronium hexafluorophosphate (1.76 g, 4.64 mmol) and *N*,*N*-diisopropylethylamine (1.38 g, 10.71 mmol) were added. The mixture was stirred for 16 h and concentrated to dryness under reduced pressure, and the residue was purified by column chromatography with an eluent system C to give the title compound **13c** (800 mg, yield: 80%).

MS m/z (ESI): 280.1 [M+1].

### Step 3

### 4-(4-(((R)-1-(3-(difluoromethyl)-2-fluorophenyl)ethyl)amino)-2-methyl-7-oxo-7,8-dihy

dropyrido[2,3-*d*]pyrimidin-6-yl)-*N*,*N*-dimethylcyclohex-3-ene-1-carboxamide **13** Compound **2c** (300 mg, 702.2 µmol), compound **13c** (392 mg, 1.4 mmol), [1,1 '-bis(diphenylphosphino)ferrocene]palladium(II) dichloride dichloromethane complex (59 mg, 70 µmol) and anhydrous sodium carbonate (112 mg, 1.06 mmol) were dissolved in 30 mL of dioxane and 3 mL of water, and the solution was purged with argon three times and reacted at 100 °C for 3 h. The reaction solution was cooled to room temperature and filtered through celite, and the filtrate was concentrated under reduced pressure. The residue was purified by column chromatography with an eluent system A to give the title compound **13** (200 mg, yield: 57%).

MS m/z (ESI): 500.1[M+1].

¹H NMR (500 MHz, CD₃OD ): δ 8.19(s, 1H), 7.60(t, 1H), 7.50(t, 1H), 7.26(t, 1H), 7.02(t, 1H), 6.33-6.30(m, 1H), 5.81(q, 1H), 3.18(s, 3H), 3.05-3.02(m, 1H), 2.99(s, 3H), 2.66-2.61(m, 1H), 2.55-2.46(m, 3H), 2.40(s, 3H), 2.00-1.96(m, 1H), 1.83-1.78(m, 1H), 1.65(d, 3H).

### Example 14

### (1S,4s)-4-(4-(((R)-1-(3-(difluoromethyl)-2-fluorophenyl)ethyl)amino)-2-methyl-7-oxo-7 ,8-dihydropyrido[2,3-d]pyrimidin-6-yl)-4-hydroxy-N,N-dimethylcyclohexane carboxamide 14-p1

### (1R,4r)-4-(4-(((R)-1-(3-(difluoromethyl)-2-fluorophenyl)ethyl)amino)-2-methyl-7-oxo-7,8-dihydropyrido[2,3-d]pyrimidin-6-yl)-4-hydroxy-N,N-dimethylcyclohexane carboxamide 14-p2

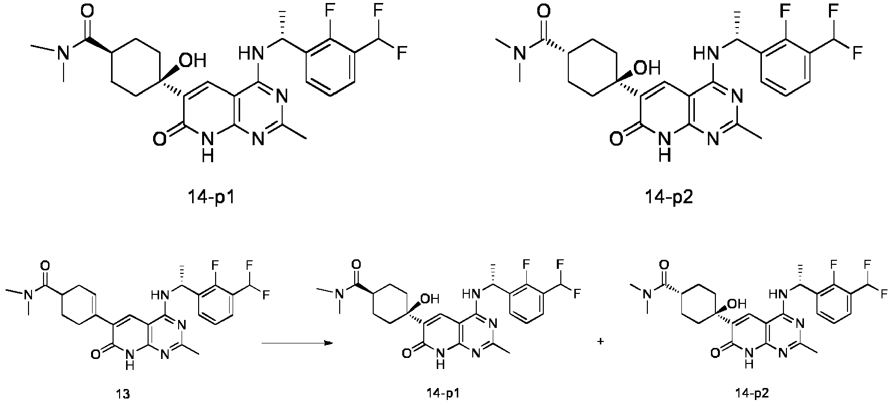

Compound **13** (100 mg, 200 µmol) was dissolved in dichloromethane (0.5 mL) and isopropanol (5 mL), and tris(dipivaloylmethanato)manganese (37 mg, 146 µmol) and phenylsilane (31 mg, 286. µmol) were added. The mixture was purged with oxygen three times and stirred for 16 h. The reaction solution was concentrated under reduced pressure, and the residue was purified by high performance liquid preparative chromatography to give the title compounds (10 mg and 15 mg, yield: 9.6% and 14.5%).

### Single-configuration compound (shorter retention time) (10 mg, 9.6%):

MS m/z (ESI): 518.1 [M+1].

HPLC analysis: retention time: 10.6 min, purity: 98.5% (chromatographic column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30% -95%).

¹H NMR (500 MHz, CD₃OD ) δ 8.38(s, 1H), 7.63(t, 1H), 7.51(t, 1H), 7.27(t, 1H), 7.02(t, 1H), 5.83(q, 1H), 3.17(s, 3H), 2.97(s, 3H), 2.83-2.78(m, 1H), 2.36(s, 3H), 2.33-2.26(m, 2H), 2.13-2.08(m, 2H), 1.90-1.87(m, 2H), 1.69-1.67(m, 2H), 1.66(d, 3H).

### Single-configuration compound (longer retention time) (15 mg, 14.5%):

MS m/z (ESI): 518.1 [M+1].

HPLC analysis: retention time: 11.89 min, purity: 97.2% (column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30%-95%).

¹H NMR (500 MHz, CD₃OD ) δ 8.30(s, 1H), 7.63(t, 1H), 7.50(t, 1H), 7.27(t, 1H), 7.03(t, 1H), 5.83(q, 1H), 3.17(s, 3H), 2.98-2.97(m, 1H), 2.95(s, 3H), 2.60-2.53(m, 2H), 2.30(s, 3H), 2.05-2.00(m, 2H), 1.91-1.88(m, 2H), 1.73-1.67(m, 2H), 1.66(d, 3H).

### Example 15

### (R)-4-(4-((1-(3-(difluoromethyl)-2-fluorophenyl)ethyl)amino)-2-methyl-7-oxo-7,8-dihy dropyrido[2,3-d]pyrimidin-6-yl)-N,N-dimethylcyclohexane carboxamide 15

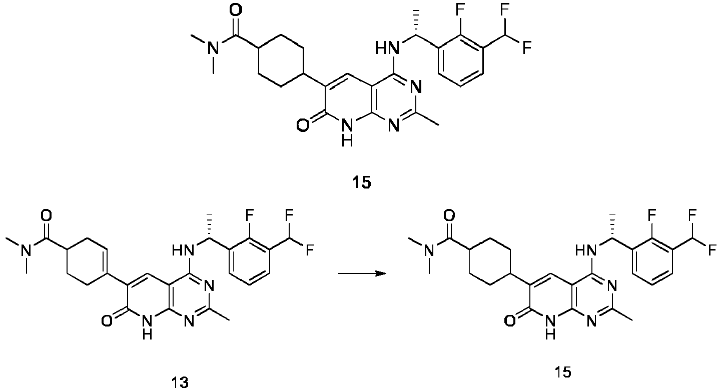

Compound 13 (50 mg, 100 µmol) was dissolved in methanol (5 mL), and 10% palladium on carbon catalyst (20 mg) was added. The mixture was purged with hydrogen three times and stirred for 16 h. The reaction solution was filtered through celite, and the filtrate was concentrated under reduced pressure. The residue was purified by high performance liquid preparative chromatography to give the title compound **15** (5 mg, yield: 10%).

MS m/z (ESI): 502.0 [M+1].

1H NMR (500 MHz, CD₃OD ) δ 8.13(s, 1H), 7.62(t, 1H), 7.50(t, 1H), 7.27(t, 1H), 7.03(t, 1H), 5.81(q, 1H), 3.14(s, 3H), 3.08-3.06(m, 1H), 2.98(s, 3H), 2.95-2.91(m, 1H), 2.35(s, 3H), 2.05-1.98(m, 4H), 1.82-1.79(m, 4H), 1.67(d, 3H).

### Example 16

### (R)-4-((1-(3-(difluoromethyl)-2-fluorophenyl)ethyl)amino)-2-methyl-6-morpholinopyri do[2,3-d]pyrimidin-7(8H)-one 16

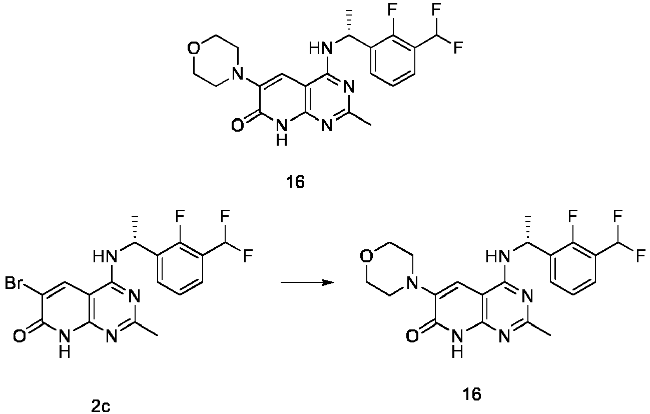

Compound **2c** (50 mg, 117 µmol) and morpholine (30 mg, 351 µmol) were dissolved in 5 mL of dimethylsulfoxide, and cuprous iodide (22 mg, 117 µmol), *L*-proline (13 mg, 117 µmol), and potassium phosphate (74 mg, 351 µmol) were added. The system was purged with nitrogen and stirred at 110 °C for 16 h. The reaction solution was filtered, and the filtrate was concentrated under reduced pressure. The residue was purified by high performance liquid preparative chromatography to give the title compound **16** (20 mg, yield: 39%).

MS m/z (ESI): 434.0 [M+1].

¹H NMR (500 MHz, CD₃OD ): δ 7.60-7.56 (m, 2H), 7.49-7.47 (m, 1H), 7.26-7.23 (m, 1H), 7.02 (t, 1H), 5.81 (q, 1H), 3.90-3.88 (m, 4H), 3.23-3.19 (m, 4H), 2.34 (s, 3H), 1.67 (d, 3H).

### Example 17

### (R)-3-(4-(4-((1-(3-(difluoromethyl)-2-fluorophenyl)ethyl)amino)-2-methyl-7-oxo-7,8-di hydropyrido[2,3-d]pyrimidin-6-yl)piperidin-1-yl)-3-oxopropanenitrile 17

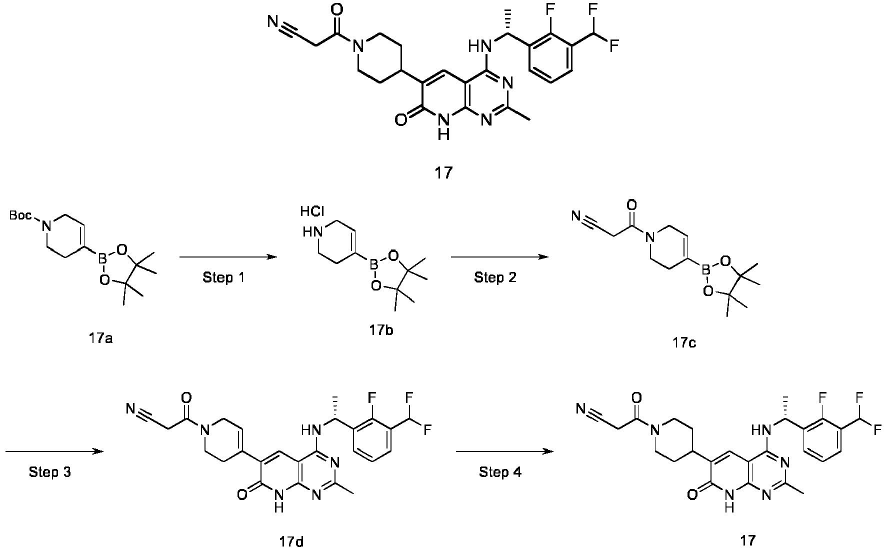

### Step 1

### 4-(4,4,5,5-tetramethyl-1,3,2-dioxaborolan-2-yl)-1,2,3,6-tetrahydropyridine hydrochloride 17b

The compound *tert*-butyl 4-(4,4,5,5-tetramethyl-1,3,2-dioxaborolan-2-yl)-5,6-dihydropyridine-1(2*H*)-carboxylate **17a** (1 g, 3.23 mmol, Shanghai Bidepharm) was dissolved in 4N 1,4-dioxane hydrochloride solution, and the solution was stirred for 3 h. The reaction solution was concentrated under reduced pressure to give a crude product **17b** (790 mg, yield: 99.4%), which was directly used in the next step without purification.

MS m/z (ESI): 210.1 [M+1].

### Step 2

### 3-oxo-3-(4-(4,4,5,5-tetramethyl-1,3,2-dioxaborolan-2-yl)-5,6-dihydropyridin-1(2H)-yl) propionitrile 17c

Compound **17b** (789 mg, 3.23 mmol) and cyanoacetic acid (302 mg, 3.55 mmol, J&K Chemical) were dissolved in *N*,*N*-dimethylformamide (20 mL), and 2-(7-azobenzotriazol)-*N*,*N*,*N*,*N-*tetramethyluronium hexafluorophosphate (1.47 g, 3.86 mmol) and *N*,*N*-diisopropylethylamine (1.25 g, 9.67 mmol) were added sequentially. The mixture was stirred for 16 h. The reaction solution was concentrated under reduced pressure, and the residue was purified by silica gel column chromatography with an eluent system A to give the title compound **17c** (800 mg, yield: 89%).

MS m/z (ESI): 277.2 [M+1].

### Step 3

### (R)-3-(4-(4-((1-(3-(difluoromethyl)-2-fluorophenyl)ethyl)amino)-2-methyl-7-oxo-7,8-di hydropyrido[2,3-d]pyrimidin-6-yl)-3,6-dihydropyridin-1(2H)-yl)-3-oxopropanenitrile 17d

Compound **2c** (70 mg, 163.8 µmol), compound **17c** (90 mg, 325.9 µmol), [1,1'-bis(diphenylphosphino)ferrocene]palladium(II) dichloride dichloromethane complex (13 mg, 15.9 µmol) and anhydrous sodium carbonate (34 mg, 320.8 µmol) were dissolved in 5 mL of dioxane and 1 mL of water, and the solution was purged with nitrogen 3 times and reacted at 80 °C for 1 h under microwave irradiation. The reaction solution was cooled to room temperature and filtered through celite, and the filtrate was concentrated under reduced pressure. The residue was purified by column chromatography with an eluent system A to give the title compound **17d** (80 mg, yield: 98.3%).

MS m/z (ESI): 497.1[M+1].

### Step 4

### (R)-3-(4-(4-((1-(3-(difluoromethyl)-2-fluorophenyl)ethyl)amino)-2-methyl-7-oxo-7,8-di hydropyrido[2,3-d]pyrimidin-6-yl)piperidin-1-yl)-3-oxopropanenitrile 17

Compound **17d** (100 mg, 201.4 µmol) was dissolved in methanol (10 mL), and 10% palladium on carbon catalyst (20 mg) was added. The mixture was purged with hydrogen three times and stirred for 16 h. The reaction solution was filtered through celite, and the filtrate was concentrated under reduced pressure. The residue was purified by high performance liquid preparative chromatography to give the title compound **17** (10 mg, yield: 10%).

MS m/z (ESI): 499.1 [M+1].

¹H NMR (500 MHz, CD₃OD ): δ 8.15 (s, 1H), 7.57 (t, 1H), 7.47 (t, 1H), 7.23 (t, 1H), 7.00 (t, 1H), 5.76 (qd, 1H), 4.71- 4.66 (m, 2H), 3.90 (ddd, 1H), 3.37 - 3.32 (m, 1H), 3.28 (d, 1H), 3.12 (tt, 1H), 2.83 (td, 1H), 2.33 (d, 3H), 2.10 - 2.00 (m, 1H), 1.98 - 1.91 (m, 1H), 1.73-1.61 (m, 5H).

### Example 18

### 4-(((R)-1-(3-(difluoromethyl)-2-fluorophenyl)ethyl)amino)-6-((1s,4S)-1,4-dihydroxycyc lohexyl)-2-methylpyrido[2,3-d]pyrimidin-7(8H)-one 18-p1

### 4-(((R)-1-(3 -(difluoromethyl)-2-fluorophenyl)ethyl)amino)-6-((1r,4R)-1,4-dihydroxycyc lohexyl)-2-methylpyrido[2,3-d]pyrimidin-7(8H)-one 18-p2

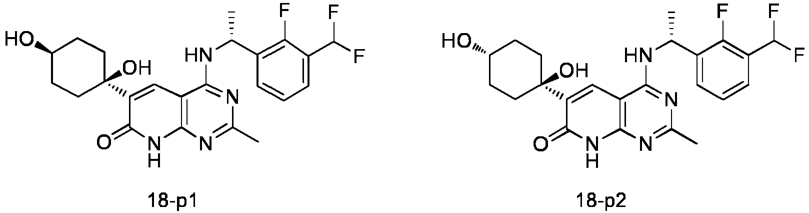

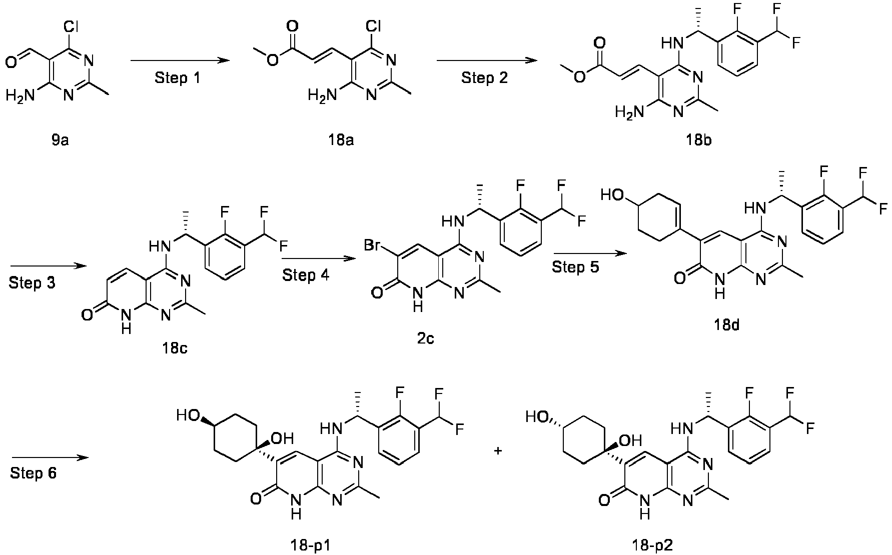

### Step 1

### Methyl (E)-3-(4-amino-6-chloro-2-methylpyrimidin-5-yl)acrylate 18a

Compound **9a** (5.8 g, 33.8 mmol) and methyl (triphenylphosphoranylidene)acetate (11.3 g, 33.8 mmol, Shanghai Bidepharm) were dissolved in tetrahydrofuran (100 mL), and the solution was stirred at 70 °C for 2 h. The reaction solution was concentrated under reduced pressure, and the residue was purified by silica gel column chromatography with an eluent system A to give the title compound **18a** (6 g, yield: 78%).

MS m/z (ESI): 228.2 [M+1].

### Step 2

### Methyl

### (R,E)-3-(4-amino-6-((1-(3-(difluoromethyl)-2-fluorophenyl)ethyl)amino)-2-methylpyri midin-5-yl)acrylate 18b

Compound **18a** (1 g, 4.39 mmol), compound **2a** (1.09 g, 4.83 mmol), and *N,N-*diisopropylethylamine (1.7 g, 13.1 mmol) were dissolved in 2 mL of dimethyl sulfoxide, and the solution was reacted at 130 °C for 5 h under microwave irradiation. The reaction solution was cooled, and water was added. The resulting mixture was extracted with ethyl acetate (20 mL × 3). The organic phases were combined and concentrated under reduced pressure to give the title compound **18b** (crude product, 1.5 g, yield: 89.8%), which was directly used in the next step without purification.

MS m/z (ESI): 381.1 [M+1].

### Step 3

### (R)-4-((1-(3-(difluoromethyl)-2-fluorophenyl)ethyl)amino)-2-methylpyrido[2,3-d]pyrim idin-7(8H)-one 18c

Compound **18b** (1.5 g, 3.94 mmol) was dissolved in methanol (30 mL), and sodium methoxide (425 mg, 7.88 mmol) was added. The mixture was refluxed for 3 h. The reaction solution was concentrated under reduced pressure, and the residue was purified by column chromatography with an eluent system C to give the title compound **18c** (0.9 g, yield: 65.5%).

MS m/z (ESI): 349.2 [M+1].

### Step 4

### (R)-6-bromo-4-((1-(3-(difluoromethyl)-2-fluorophenyl)ethyl)amino)-2-methylpyrido[2, 3-d]pyrimidin-7(8H)-one 2c

Compound **18c** (1.8 g, 5.16 µmol) was dissolved in tetrahydrofuran (50 mL), and N-bromosuccinimide (1.38 g, 7.75 mmol) was added. The mixture was reacted at 40 °C for 2 h. The reaction solution was cooled to room temperature, and the reaction was quenched with saturated sodium thiosulfate. The mixture was extracted with ethyl acetate (20 mL × 3). The organic phases were combined and concentrated under reduced pressure, and the residue was purified by column chromatography with an eluent system A to give the title compound **2c** (1.1 g, yield: 49.8%).

MS m/z (ESI): 428.2 [M+1].

### Step 5

### 4-(((R)-1-(3-(difluoromethyl)-2-fluorophenyl)ethyl)amino)-6-(4-hydroxycyclohex-1-en-1-yl)-2-methylpyrido[2,3-d]pyrimidin-7(8H)-one 18d

Compound **2c** (850 mg, 1.98 mmol), compound 4-(4,4,5,5-tetramethyl-1,3,2-dioxaborolan-2-yl)cyclohex-3-enol (891.7 mg, 3.98 mmol, Accela ChemBio), [1,1'-bis(diphenylphosphino)ferrocene]palladium(II) dichloride dichloromethane complex (162.3 mg, 198.9 µmol) and anhydrous sodium carbonate (421.7 mg, 3.98 mmol) were dissolved in 5 mL of dioxane and 1 mL of water, and the solution was purged with nitrogen 3 times and reacted at 80 °C for 14 h. The reaction solution was cooled to room temperature and filtered through celite, and the filtrate was concentrated under reduced pressure. The residue was purified by column chromatography with an eluent system A to give the title compound **18d** (550 mg, yield: 62.1%).

MS m/z (ESI): 445.2[M+1].

### Step 6

### 4-(((R)-1-(3-(difluoromethyl)-2-fluorophenyl)ethyl)amino)-6-((1s,4S)-1,4-dihydroxycyc lohexyl)-2-methylpyrido[2,3-d]pyrimidin-7(8H)-one 18-p1

### 4-(((R)-1 -(3 -(difluoromethyl)-2-fluorophenyl)ethyl)amino)-6-((1r,4R)-1 ,4-dihydroxycyc lohexyl)-2-methylpyrido[2,3-d]pyrimidin-7(8H)-one 18-p2

Compound **18d** (100 mg, 224.9 µmol) was dissolved in dichloromethane (0.5 mL) and isopropanol (5 mL), and tris(dipivaloylmethanato)manganese (40.8 mg, 67.4 µmol) and phenylsilane (73 mg, 674.µmol) were added. The mixture was purged with oxygen three times and stirred for 16 h. The reaction solution was concentrated under reduced pressure, and the residue was purified by high performance liquid preparative chromatography to give the title compounds **18-p1** (16 mg, yield: 15.3%) and **18-p2** (20 mg, yield: 19.2%).

### Single configuration compound 18-p1 (shorter retention time) (16 mg, 15.3%)

MS m/z (ESI): 463.1 [M+1].

HPLC analysis: retention time: 11.2 min, purity: 98.5% (chromatographic column: SharpSil-T, Prep 30×150 mm; 5 µm; mobile phase: water (10 mmol/L ammonium bicarbonate)/acetonitrile; gradient ratio: water (10 mM ammonium bicarbonate) 30%-50%).

¹H NMR (500 MHz, CD₃OD ): δ 8.35 (s, 1H), 7.60 (t, 1H), 7.50 (d, 1H), 7.25 (t, 1H), 7.14-6.92 (t, 1H), 5.81 (q, 1H), 3.67 (tt, 1H), 2.35 (s, 3H), 2.19 (td, 2H), 1.95-1.81 (m, 6H), 1.66 (d, 3H).

### Single configuration compound 18-p2 (longer retention time) (20 mg, 19.2%)

MS m/z (ESI): 463.1 [M+1].

HPLC analysis: retention time: 12.1 min, purity: 99.2% (column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30%-50%).

¹H NMR (500 MHz, CD₃OD ): δ 8.30 (s, 1H), 7.60 (t, 1H), 7.48 (t, 1H), 7.25 (t, 1H), 7.14-6.92 (t, 1H), 5.81 (q, 1H), 4.06 (s, 1H), 2.34 (d, 5H), 2.09 (t, 2H), 1.83 (s, 2H), 1.68 (dd, 5H).

### Example 19

### (R)-4-((1-(3-(difluoromethyl)-2-fluorophenyl)ethyl)amino)-6-(4-hydroxytetrahydro-2H-pyran-4-yl)-2-methylpyrido[2,3-d]pyrimidin-7(8H)-one 19

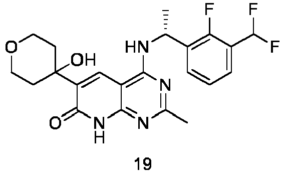

The title compound **19** (38 mg, yield: 77.6%) was obtained by following the synthetic route in Example 12 with the starting compound 1-(4-(4,4,5,5-tetramethyl-1,3,2-dioxaborolan-2-yl)-5,6-dihydropyridin-1(2*H*)-yl)ethano ne in the step 1 replaced by the compound 2-(3,6-dihydro-2*H-*pyran-4-yl)-4,4,5,5-tetramethyl-1,3,2-dioxaborolan (Shanghai Bidepharm).

MS m/z (ESI): 449.1 [M+1].

¹H NMR (500 MHz, CD₃OD ): δ 8.34(s, 1H), 7.62-7.59 (m, 1H), 7.50-7.48 (m, 1H), 7.27-7.24 (m, 1H), 7.13-6.92 (t, 1H), 5.83-5.79 (m, 1H), 4.02-3.97 (m, 2H), 3.87-3.84 (m, 2H), 2.42-2.37 (m, 2H), 2.36 (s, 3H), 1.82-1.80 (m, 2H),1.67-1.66 (d, 3H).

### Example 20

### (R)-4-((1-(3-(difluoromethyl)-2-fluorophenyl)ethyl)amino)-6-(1-(2-methoxyacetyl)piper idin-4-yl)-2-methylpyrido[2,3-d]pyrimidin-7(8H)-one 20

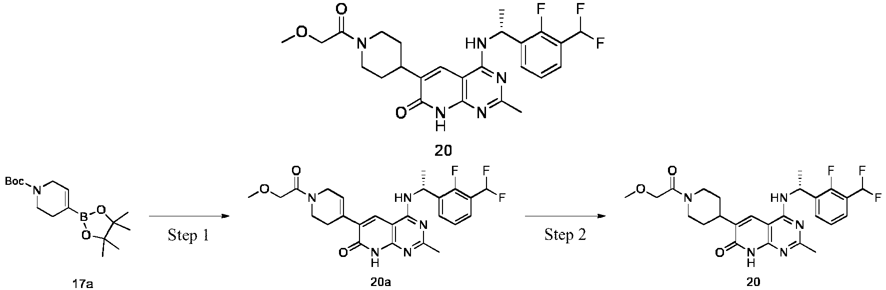

### Step 1

### (R)-4-((1-(3-(difluoromethyl)-2-fluorophenyl)ethyl)amino)-6-(1-(2-methoxyacetyl)-1,2, 3,6-tetrahydropyridin-4-yl)-2-methylpyrido[2,3-d]pyrimidin-7(8H)-one 20a

The title compound **20a** (90 mg, yield: 87.5%) was obtained by following the synthetic route in Example 17 with the starting compound cyanoacetic acid in the step 2 replaced by the compound methoxyacetic acid.

MS m/z (ESI): 502.1 [M+1].

### Step 2

### (R)-4-((1-(3-(difluoromethyl)-2-fluorophenyl)ethyl)amino)-6-(1-(2-methoxyacetyl)piper idin-4-yl)-2-methylpyrido[2,3-d]pyrimidin-7(8H)-one 20

Compound **20a** (80 mg, 179.4 µmol) was dissolved in methanol (10 mL), and 10% palladium on carbon catalyst (20 mg) was added. The mixture was purged with hydrogen three times and stirred for 16 h. The reaction solution was filtered through celite, and the filtrate was concentrated under reduced pressure. The residue was purified by high performance liquid preparative chromatography to give the title compound **20** (15 mg, yield: 16.5%).

MS m/z (ESI): 504.1 [M+1].

¹H NMR (400 MHz, CD₃OD ): 8.15 (s, 1H), 7.60-7.59 (m, 1H), 7.50-7.49 (m, 1H), 7.26-7.23 (m, 1H), 7.13-7.03 (m, 1H), 5.80 (q, 1H), 4.72-4.70 (m, 1H), 4.60 (s, 2H), 4.26-4.20 (m, 2H), 4.04-4.01 (m, 1H), 3.37 (s, 3H), 3.17-3.12 (m, 2H), 2.83-2.78 (m, 1H), 2.35 (s, 3H), 2.04-1.96 (m, 2H), 1.66 (d, 3H).

### Example 21

### (R)-3-(4-(4-(( 1-(3-(difluoromethyl)-2-fluorophenyl)ethyl)amino)-2-methyl-7-oxo-7, 8-di hydropyrido[2,3-d]pyrimidin-6-yl)-4-hydroxypiperidin-1-yl)-3-oxopropanenitrile 21

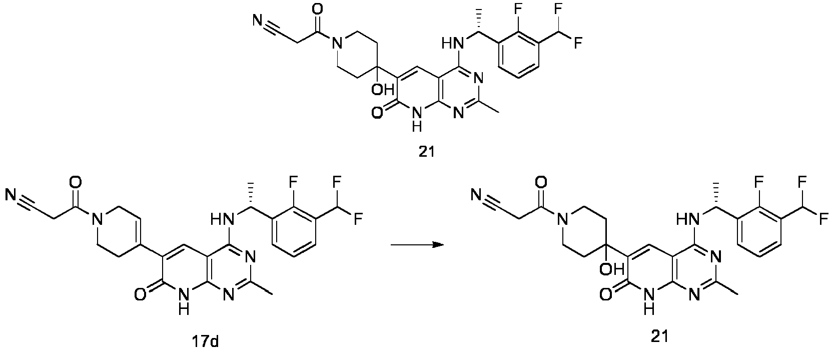

Compound **17d** (130 mg, 261.8 µmol) was dissolved in dichloromethane (0.5 mL) and isopropanol (5 mL), and manganese acetylacetonate (31.6 mg, 52.3 µmol) and phenylsilane (56.6 mg, 523.6 µmol) were added. The mixture was purged with oxygen three times and stirred for 16 h. The reaction solution was concentrated, and the residue was purified by high performance liquid preparative chromatography to give the title compound **21** (20 mg, yield: 14.8%).

MS m/z (ESI): 515.2 [M+1].

¹H NMR (500 MHz, CD₃OD ): δ 8.37 (s, 1H), 7.60 (t, 1H), 7.48 (t, 1H), 7.25 (t, 1H), 7.01 (t, 1H), 5.80 (q, 1H), 4.61 (s, 2H), 4.52-4.43 (m, 1H), 3.72-3.64 (m, 2H), 3.22 (td, 1H), 2.38 (dd, 1H), 2.36 (s, 3H), 2.32 (dt, 1H), 1.93 (dt, 1H), 1.84 (ddt, 1H), 1.66 (d, 3H).

### Example 22

### rac-(R)-4-((1-(3-(difluoromethyl)-2-fluorophenyl)ethyl)amino)-6-(1-(2-fluoroacetyl)-4-hydroxypiperidin-4-yl)-2-methylpyrido[2,3-d]pyrimidin-7(8H)-one 22

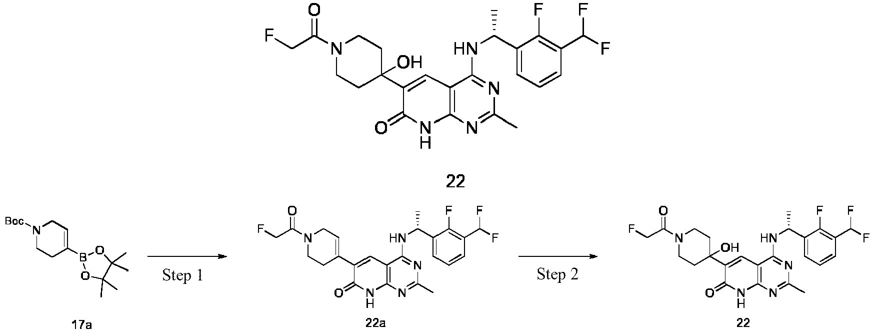

### Step 1

### (R)-4-((1-(3-(difluoromethyl)-2-fluorophenyl)ethyl)amino)-6-(1-(2-fluoroacetyl)-1,2,3,6 -tetrahydropyridin-4-yl)-2-methylpyrido[2,3-d]pyrimidin-7(8H)-one 22a

The title compound **22a** (120 mg, yield: 87.5%) was obtained by following the synthetic route in Example 17 with the starting compound cyanoacetic acid in the step 2 replaced by the compound fluoroacetic acid (European Journal of Organic Chemistry, 2014, 2014, 12, 2451-2459).

MS m/z (ESI): 490.1 [M+1].

### Step 2

### rac-(R)-4-((1-(3-(difluoromethyl)-2-fluorophenyl)ethyl)amino)-6-(1-(2-fluoroacetyl)-4-hydroxypiperidin-4-yl)-2-methylpyrido[2,3-d]pyrimidin-7(8H)-one 22

Compound **22a** (120 mg, 245.2 µmol) was dissolved in dichloromethane (0.5 mL) and isopropanol (5 mL), and manganese acetylacetonate (74.1 mg, 122.5 µmol) and phenylsilane (53 mg, 490 µmol) were added. The mixture was purged with oxygen three times and stirred for 16 h. The reaction solution was concentrated, and the residue was purified by high performance liquid preparative chromatography to give the title compound **22** (20 mg, yield: 16%).

MS m/z (ESI): 508.2 [M+1].

¹H NMR (500 MHz, DMSO-*d₆*): δ 8.44 (d, 1H), 7.68 (t, 1H), 7.50 (d, 1H), 7.36-7.28 (m, 1H), 7.18 (d, 1H), 5.76 (t, 1H), 5.56 (s, 1H), 5.36-5.01 (m, 2H), 4.30 (d, 1H), 3.42 (s, 2H), 3.01 (s, 1H), 2.27 (s, 3H), 2.00 (d, 1H), 1.57 (d, 3H), 1.27 (d, 2H).

### Example 23

### (R)-4-((1-(3-(difluoromethyl)-2-fluorophenyl)ethyl)amino)-6-((1,1-dioxidotetrahydro-2 H-thiopyran-4-yl)oxy)-2-methylpyrido[2,3-d]pyrimidin-7(8H)-one 23

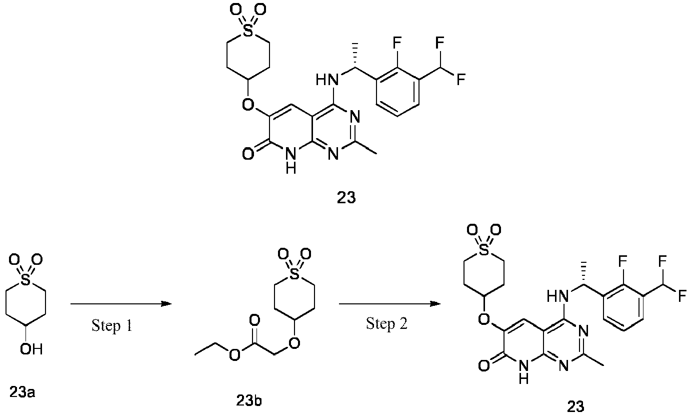

### Step 1

### Ethyl 2-((1,1-dioxotetrahydro-2H thiopyran-4-yl)oxy)acetate 23b

The compound 4-hydroxytetrahydro-2*H*-thiopyran 1,1-dioxide **23a** (1 g, 6.65 mmol, Shanghai Bidepharm) was dissolved in tetrahydrofuran (20 mL), and sodium hydride (399.4 mg, 9.98 mmol) was added under an ice bath. The mixture was stirred for 0.5 h with the temperature maintained, followed by the addition of ethyl bromoacetate (1.1 g, 6.65 mmol). The resulting mixture was naturally warmed to room temperature and reacted for 14 h. The reaction was quenched with water, and the reaction solution was extracted with dichloromethane (20 mL × 3). The organic phases were combined and concentrated under reduced pressure, and the residue was purified by silica gel column chromatography with an eluent system C to give the title compound **23b** (200 mg, yield: 12.7%).

MS m/z (ESI): 237.2 [M+1].

### Step 2

### (R)-4-((1-(3-(difluoromethyl)-2-fluorophenyl)ethyl)amino)-6-((1,1-dioxidotetrahydro-2 H-thiopyran-4-yl)oxy)-2-methylpyrido[2,3-d]pyrimidin-7(8H)-one 23

The title compound **23** (5 mg, yield: 11.5%) was obtained by following the synthetic route in Example 9 with the starting compound **9b** in the step 2 replaced by compound **23b,** and with the starting compound **1e** in the step 3 replaced by compound **2a.**

MS m/z (ESI): 497.2 [M+1].

¹H NMR (500 MHz, CDCl₃) δ 7.59 (s, 1H), 7.51 (t, 1H), 7.28 (s, 1H), 7.23 (t, 1H), 6.93 (t, 1H), 5.79 (s, 1H), 5.00 (s, 1H), 3.48 (td, 2H), 3.00 (d, 2H), 2.53 (s, 3H), 2.48 (s, 2H), 2.39 (t, 2H), 1.73 (d, 3H).

### Example 24

### 6-(((S)-1-acetylpyrrolidin-3-yl)oxy)-4-(((R)-1-(3 -(difluoromethyl)-2-fluorophenyl)ethyl) amino)-2-methylpyrido[2,3-d]pyrimidin-7(8H)-one 24

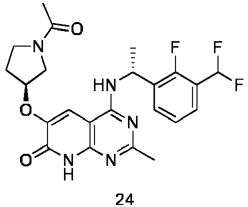

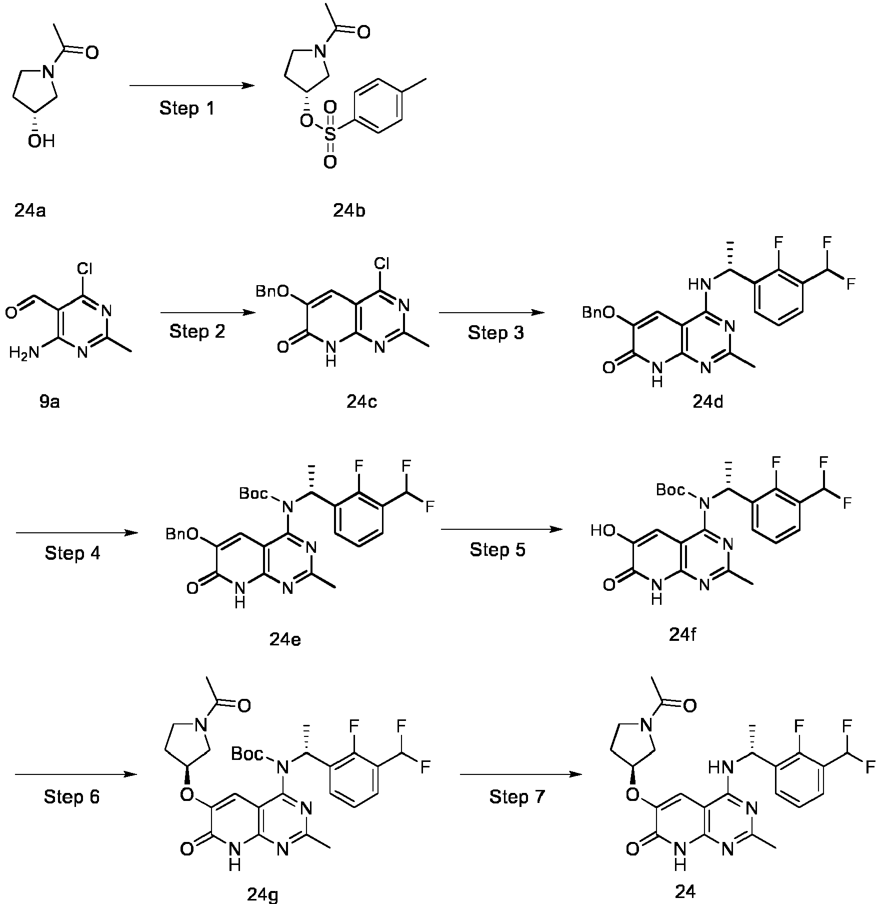

### Step 1

### (R)-1-acetylpyrrolidin-3-yl 4-methylbenzenesulfonate 24b

(*R*)-1-(3-hydroxypyrrolidin-1-yl)ethanone **24a** (1 g, 7.74 mmol), 4-dimethylaminopyridine (95 mg, 0.77 mmol), and triethylamine (1.5 g, 14.8 mmol) were dissolved in 20 mL of dichloromethane, and 4-toluenesulfonyl chloride (1.7 g, 8.9 mmol) was added. The mixture was stirred for 3 h. The reaction solution was concentrated under reduced pressure, and the residue was purified by column chromatography with an eluent system C to give the title compound **24b** (800 mg, yield: 36.4%).

MS m/z (ESI): 284.0 [M+1].

### Step 2

### 6-(benzyloxy)-4-chloro-2-methylpyrido[2,3-d]pyrimidin-7(8H)-one 24c

Compound **9a** (300 mg, 1.74 mmol), compound ethyl 2-benzyloxyacetate (425 mg, 2.18 mmol, Accela ChemBio) was dissolved in 5 mL of tetrahydrofuran, and potassium tert-butoxide (246 mg, 2.19 mmol) was added. The mixture was refluxed for 3 h. The reaction solution was cooled to room temperature, and water was added. The resulting mixture was extracted with ethyl acetate (10 mL × 3). The organic phases were combined, dried over anhydrous sodium sulfate, filtered and concentrated under reduced pressure, and the residue was purified by column chromatography with an eluent system C to give the title compound **24c** (200 mg, yield: 37.9%).

MS m/z (ESI): 302.1 [M+1].

### Step 3

### (R)-6-(benzyloxy)-4-((1-(3-(difluoromethyl)-2-fluorophenyl)ethyl)amino)-2-methylpyri do[2,3-d]pyrimidin-7(8H)-one 24d

Compound **24c** (200 mg, 662.8 µmol), compound **2a** (130 mg, 687.2 mmol), and *N,N-*diisopropylethylamine (172 mg, 1.33 mmol) were dissolved in 2 mL of dimethyl sulfoxide, and the solution was reacted at 120 °C for 2 h under microwave irradiation. The reaction solution was cooled, and water was added. The resulting mixture was extracted with ethyl acetate (10 mL × 3). The organic phases were combined, dried over anhydrous sodium sulfate, filtered and concentrated under reduced pressure, and the residue was purified by column chromatography with an eluent system C to give the title compound **24d** (210 mg, yield: 69.7%).

MS m/z (ESI): 455.1 [M+1].

### Step 4

### Tert-butyl (R)-(6-(benzyloxy)-2-methyl-7-oxo-7,8-dihydropyrido[2,3-d]pyrimidin-4-yl)(1-(3-(diflu oromethyl)-2-fluorophenyl)ethyl)carbamate 24e

Compound **24d** (180 mg, 396 mmol), 4-dimethylaminopyridine (50 mg, 0.40 mmol) were dissolved in 10 mL of dichloromethane, and di-*tert*-butyl dicarbonate (100 mg, 0.45 mmol) was added. The mixture was stirred for 14 h. Water was added to the reaction solution, and the resulting mixture was extracted with ethyl acetate (10 mL × 3). The organic phases were combined, dried over anhydrous sodium sulfate and filtered, and the filtrate was concentrated under reduced pressure. The residue was purified by column chromatography with an eluent system C to obtain the title compound **24e** (100 mg, yield: 45.5%).

MS m/z (ESI): 555.2 [M+1].

### Step 5

### Tert-butyl (R)-(1-(3-(difluoromethyl)-2-fluorophenyl)ethyl)(6-hydroxy-2-methyl-7-oxo-7,8-dihydr opyrido[2,3-d]pyrimidin-4-yl)carbamate 24f

Compound **24e** (100 mg, 180.3 µmol) was dissolved in methanol (10 mL), and 10% palladium on carbon catalyst (20 mg) was added. The mixture was purged with hydrogen three times and stirred for 16 h. The reaction solution was filtered through celite, and the filtrate was dried by rotary evaporation to give the title compound **24f** (80 mg, yield: 95.5%).

MS m/z (ESI): 465.1 [M+1].

### Step 6

### Tert-butyl

### (6-(((S)-1-acetylpyrrolidin-3-yl)oxy)-2-methyl-7-oxo-7,8-dihydropyrido[2,3-d]pyrimidi n-4-yl)((R)-1-(3-(difluoromethyl)-2-fluorophenyl)ethyl)carbamate 24g

Compound **24f** (80 mg, 172.2 µmol) was dissolved in *N,N*-dimethylformamide (5 mL), and potassium carbonate (24 mg, 173.6 mmol) and **24b** (45 mg, 158.8 µmol) were added. The mixture was heated to 60 °C and reacted for 1 h. The reaction solution was cooled and filtered, and the filtrate was concentrated under reduced pressure. The residue was purified by column chromatography with an eluent system A to give the title compound **24g** (50 mg, yield: 50.4%).

MS m/z (ESI): 576.0 [M+1].

### Step 7

### 6-(((S)-1-acetylpyrrolidin-3-yl)oxy)-4-(((R)-1-(3-(difluoromethyl)-2-fluorophenyl)ethyl) amino)-2-methylpyrido[2,3-d]pyrimidin-7(8H)-one 24

Compound **24g** (50 mg, 86.8 µmol) was dissolved in 2 mL of dichloromethane solvent, and 1 mL of trifluoroacetic acid was added. The mixture was stirred for 2 h. The reaction solution was concentrated under reduced pressure, the residue was dissolved in 5 mL of dichloromethane and 1 mL of methanol, followed by the addition of solid sodium bicarbonate. The mixture was stirred for 10 min, and the pH was adjusted to alkalinity. The reaction solution was filtered and concentrated, and the residue was purified by high performance liquid preparative chromatography to give the title compound **24** (3.2 mg, yield: 7.7%).

MS m/z (ESI): 476.2 [M+1].

¹H NMR (500 MHz, CD₃OD ): δ 7.90-7.88 (m, 1H), 7.67-7.64 (m, 1H), 7.54-7.51 (m, 1H), 7.30-7.27 (m, 1H), 7.13-6.91 (t, 1H), 5.87-5.86 (m, 1H), 5.23-5.16 (m, 1H), 3.95-3.57 (m, 4H), 2.47 (s, 3H), 2.46-2.14 (m, 2H), 2.13-2.11 (d, 3H), 1.73-1.71 (d, 3H).

### Example 25

### rac-4-(((R)-1-(3,3-difluoro-2,3-dihydrobenzofuran-7-yl)ethyl)amino)-6-((1s,4S)-1,4-dih ydroxycyclohexyl)-2-methylpyrido[2,3-d]pyrimidin-7(8H)-one 25-p1

### rac-4-(((R)-1-(3,3-difluoro-2,3-dihydrobenzofuran-7-yl)ethyl)amino)-6-((1r,4R)-1,4-dih ydroxycyclohexyl)-2-methylpyrido[2,3-d]pyrimidin-7(8H)-one 25-p2

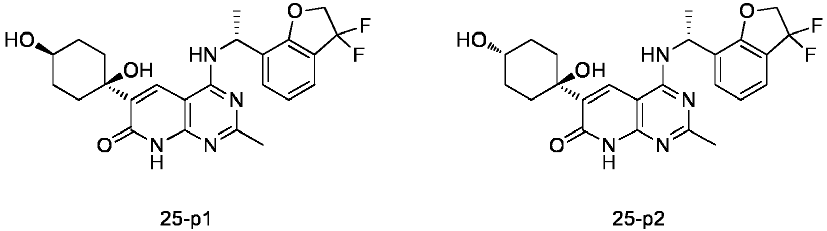

The title compounds **25-p1** (8 mg, yield: 3.8%) and **25-p2** (15 mg, yield: 7.2%) were prepared by following the synthetic route in Example 18 with the starting compound **2a** in the step 2 replaced by the compound rac-(*R*)-1-(3,3-difluoro-2,3-dihydrobenzofuran-7-yl)ethylamine hydrochloride (prepared by the method disclosed in Example B-5 on page 105 of the specification in patent application "US20190194192A1").

### Single configuration compound 25-p1 (shorter retention time) (8 mg, 3.8%)

MS m/z (ESI): 473.1 [M+1].

HPLC analysis: retention time: 10.5 min, purity: 98.5% (chromatographic column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30%-50%).

H NMR (400 MHz, CD₃OD): 8.31 (s, 1H),7.52-7.42 (m, 2H), 7.08-7.05 (m, 1H), 5.73 (q, 1H), 4.74 (t, 2H), 3.68-3.63 (m, 1H), 2.37 (s, 3H), 2.23-2.20(m, 2H), 1.91-1.84 (m, 6H), 1.64(d, 3H).

### Single-configuration compound 25-p2 (longer retention time) (15 mg, 7.2%):

MS m/z (ESI): 473.1 [M+1].

HPLC analysis: retention time: 11.3 min, purity: 99.2% (column: SharpSil-T, Prep 30×150 mm; 5 µm; mobile phase: water (10 mmol/L ammonium bicarbonate)/acetonitrile; gradient ratio: water (10 mmol/L ammonium bicarbonate) 30%-50%).

¹H NMR (400 MHz, CD₃OD): 8.27 (s, 1H),7.52-7.42 (m, 2H), 7.08-7.05 (m, 1H), 5.74 (q, 1H), 4.74 (t, 2H), 4.06-4.05 (m, 1H), 2.37-2.30 (m, 5H), 2.12-2.06 (m, 2H), 1.82-1.64(m, 7H).

### Example 26

### rac-(R)-6-((1-acetylazetidin-3-yl)oxy)-4-((1-(3-(difluoromethyl)-2-fluorophenyl)ethyl)a mino)-2-methylpyrido[2,3-d]pyrimidin-7(8H)-one 26

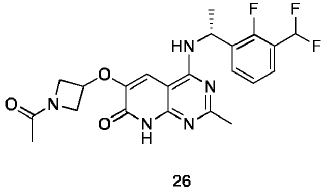

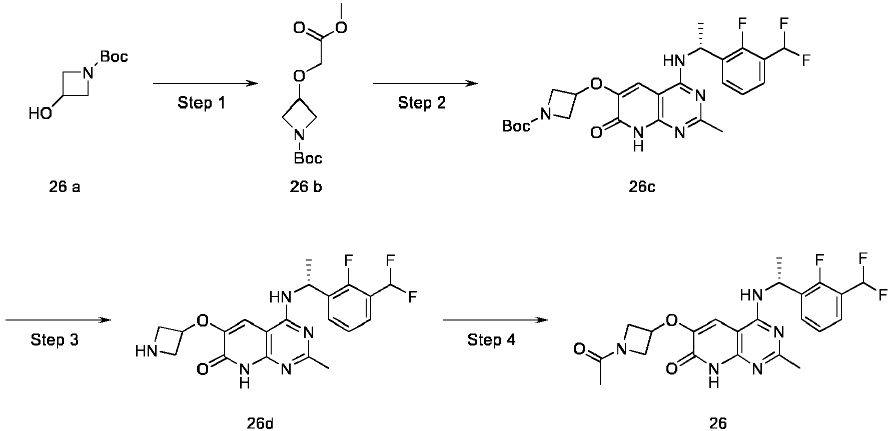

### Step 1

*Tert*-butyl 3-(2-methoxy-2-oxoethoxy)azetidine-1-carboxylate **26b** *Tert*-butyl 3-hydroxyazetidine-1-carboxylate **26a** (1 g, 5.77 mmol, Shanghai Bidepharm) was dissolved in tetrahydrofuran (20 mL), and sodium hydride (132.7 mg, 5.77 mmol) was added under an ice bath. The mixture was stirred for 0.5 h with the temperature maintained, followed by the addition of ethyl bromoacetate (883.2 g, 5.77 mmol). The resulting mixture was naturally warmed to room temperature and reacted for 14 h. Water was added to the reaction solution, and the mixture was extracted with dichloromethane (20 mL × 3). The organic phases were combined and concentrated under pressure, and the residue was purified by silica gel column chromatography with an eluent system C to give the title compound **26b** (1 g, yield: 70.6%).

MS m/z (ESI): 246.2 [M+1].

### Step 2

### Tert-butyl

### (R)-3-((4-((1-(3-(difluoromethyl)-2-fluorophenyl)ethyl)amino)-2-methyl-7-oxo-7,8-dihy dropyrido[2,3-d]pyrimidin-6-yl)oxy)azetidine-1-carboxylate 26c

The title compound **26c** (200 mg, yield: 39.2%) was obtained by following the synthetic route in Example 9 with the starting compound **9b** in the step 2 replaced by compound **26b,** and with the starting compound **1e** in the step 3 replaced by compound **2a.**

MS m/z (ESI): 520.2 [M+1].

### Step 3

### (R)-6-(azetidin-3-yloxy)-4-((1-(3-(difluoromethyl)-2-fluorophenyl)ethyl)amino)-2-meth ylpyrido[2,3-d]pyrimidin-7(8H)-one 26d

Compound **26c** (53 mg, 102 µmol) was dissolved in 2 mL of dichloromethane solvent, and 1 mL of trifluoroacetic acid was added. The mixture was stirred for 2 h. The reaction solution was concentrated under reduced pressure to give a crude product **26d** (40 mg, yield: 93.5%), which was directly used in the next step without purification.

MS m/z (ESI): 420.2 [M+1].

### Step 4

### rac-(R)-6-((1-acetylazetidin-3-yl)oxy)-4-((1-(3-(difhioromethyl)-2-fluorophenyl)ethyl)a mino)-2-methylpyrido[2,3-d]pyrimidin-7(8H)-one 26

Compound **26d** (20 mg, 47.68 µmol) and acetic anhydride (4.9 mg, 47.68 µmol) were dissolved in 2 mL of *N*,*N*-dimethylformamide, and 2-(7-azobenzotriazol)-*N*,*N*,*N'*,*N'*-tetramethyluronium hexafluorophosphate (35.9 mg, 152.6 mmol) and *N*,*N*-diisopropylethylamine (19.7 mg, 152.6 mmol) were added. The mixture was stirred for 2 h. The reaction solution was concentrated under reduced pressure, and the residue was purified by high performance liquid preparative chromatography to give the title compound **26** (10 mg, yield: 45.4%).

MS m/z (ESI): 462.2 [M+1].

¹H NMR (500 MHz, CDCl₃): δ 7.54 (q, 1H), 7.51-7.46 (m, 1H), 7.24-7.13 (m, 2H), 6.63 (t, 1H), 5.78 (tt, 1H), 5.10 (dddd, 1H), 4.52 (dt, 1H), 4.43 (ddd, 2H), 4.07 (d, 1H), 2.49 (s, 3H), 1.91 (d, 3H), 1.64 (t, 3H).

### Example 27

### (R)-4-((1-(3-(difluoromethyl)-2-fluorophenyl)ethyl)amino)-6-((1-(2-fluoroacetyl)azetidi n-3-yl)oxy)-2-methylpyrido[2,3-d]pyrimidin-7(8H)-one 27

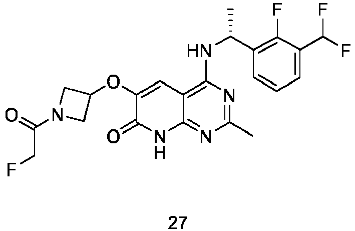

The title compound **27** (10 mg, yield: 27.3%) was obtained by following the synthetic route in Example **26** with the starting compound acetic anhydride in the step 4 replaced by 2-fluoroacetic acid.

MS m/z (ESI): 480.2 [M+1].

¹H NMR (500 MHz, CDCl₃): δ 7.57-7.46 (m, 2H), 7.25-7.13 (m, 1H), 7.10-6.80 ( m, 2H), 6.16-5.94 (m, 1H), 5.84-5.67 (m, 1H), 5.22-5.10 (m, 1H), 4.97-4.74 (m, 2H), 4.74-4.58 (m, 1H), 4.56-4.35 (m, 2H), 4.25-4.10 (m, 1H), 2.47 (s, 3H).1.7-1.62 (m,3H).

### Example 28

### 4-(((R)-1-(3-(difluoromethyl)-2-fluorophenyl)ethyl)amino)-6-((1s,4S)-1,4-dihydroxycyc lohexyl)-2,8-dimethylpyrido[2,3-d]pyrimidin-7(8H)-one 28-p1

### 4-(((R)-1-(3-(difluoromethyl)-2-fluorophenyl)ethyl)amino)-6-((1r,4R)-1,4-dihydroxycyc lohexyl)-2,8-dimethylpyrido[2,3-d]pyrimidin-7(8H)-one 28-p2

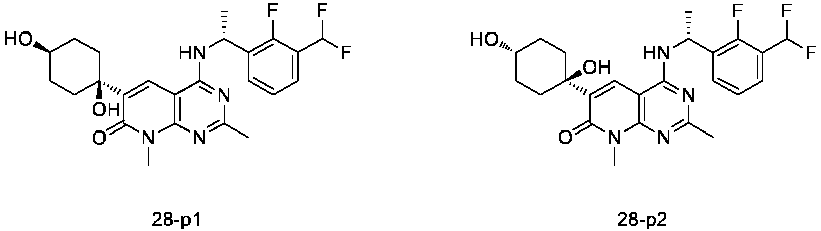

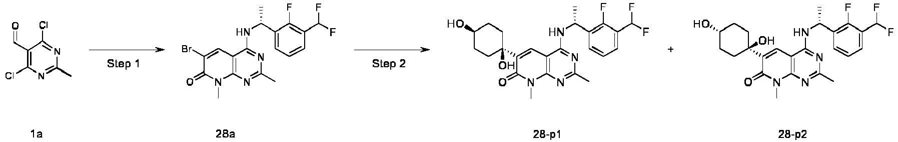

### Step 1

### (R)-6-bromo-4-((1-(3-(difluoromethyl)-2-fluorophenyl)ethyl)amino)-2,8-dimethylpyrid o[2,3-d]pyrimidin-7(8H)-one 28a

The title compound **28a** (440 mg, yield: 95.9%) was obtained by following the synthetic route in Example 1 with the starting compound 4-methoxybenzylamine in the step 1 replaced by methylamine hydrochloride, and with the starting compound **1e** in the step 4 replaced by compound **2a.**

MS m/z (ESI): 441.2 [M+1].

### Step 2

### 4-(((R)-1-(3-(difluoromethyl)-2-fluorophenyl)ethyl)amino)-6-((1s,4S)-1,4-dihydroxycyc lohexyl)-2,8-dimethylpyrido[2,3-d]pyrimidin-7(8H)-one 28-p1

### 4-(((R)-1-(3-(difluoromethyl)-2-fluorophenyl)ethyl)amino)-6-((1r,4R)-1,4-dihydroxycyc lohexyl)-2,8-dimethylpyrido[2,3-d]pyrimidin-7(8H)-one 28-p2

The title compounds **28-p1** (17 mg, yield: 4.8%) and **28-p2** (10 mg, yield: 2.8%) were obtained by following the synthetic routes for the steps 5 and 6 in Example 18, with the starting compound **2c** in the step 5 replaced by **28a.**

### Single conformation compound 28-p1 (shorter retention time) (17 mg, 4.8%)

MS m/z (ESI): 477.2 [M+1].

HPLC analysis: retention time 11.3 min, purity: 98.5% (chromatographic column: SharpSil-T, Prep 30×150 mm; 5 µm; mobile phase: A-water (10 mM ammonium bicarbonate) B-acetonitrile, gradient ratio: A 20%-55%).

¹H NMR (500 MHz, Methanol-*d*₄): δ 8.22 (s, 1 H), 7.50 (t, 1 H), 7.38 (t, 1 H), 7.14 (t, 1 H), 6.92 (t, 1 H), 5.71 (q, 1 H), 3.66-3.51 (m,4 H), 2.30 (s, 3 H), 2.11-2.07 (m, 2 H), 1.89-1.70 (m, 6 H), 1.56 (d, 3 H).

### Single conformation compound 28-p2 (longer retention time) (10 mg, 2.8%)

MS m/z (ESI): 477.2 [M+1].

HPLC analysis: retention time: 13.1 min, purity: 97.2% (column: SharpSil-T, Prep 30×150 mm; 5 µm; mobile phase: A-water (10 mM ammonium bicarbonate) B-acetonitrile, gradient ratio: A 20%-55%).

¹H NMR (500 MHz, Methanol-*d*₄): δ 8.28 (s, 1 H), 7.60 (t, 1 H), 7.49 (t, 1 H), 7.25 (t, 1 H), 7.02 (t, 1 H), 5.82 (q, 1 H), 4.07 (t, 1 H), 3.72 (s, 3 H), 2.41 (s, 3 H), 2.32 (qd, 4 H), 2.15-2.01 (m, 4 H), 1.67 (d, 3 H).

### Example 29

### (R)-4-(4-((1-(3-(difluoromethyl)-2-fluorophenyl)ethyl)amino)-2-methyl-7-oxo-7,8-dihy dropyrido[2,3-d]pyrimidin-6-yl)-4-hydroxy-N-methylcyclohexane-1-carboxamide 29

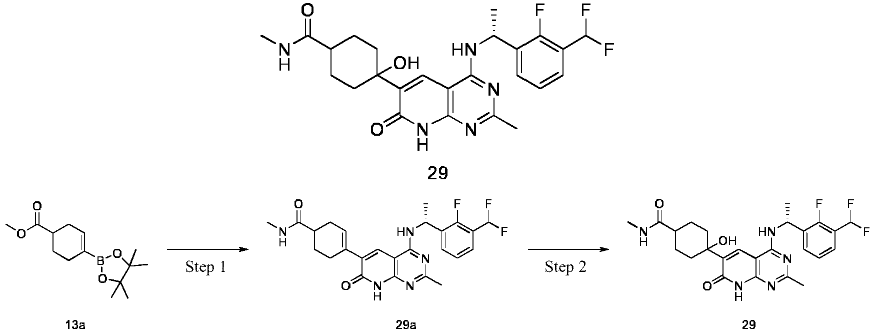

### Step 1

### 4-(4-(((R)-1-(3-(difluoromethyl)-2-fluorophenyl)ethyl)amino)-2-methyl-7-oxo-7,8-dihy dropyrido[2,3-d]pyrimidin-6-yl)-N-methylcyclohex-3-ene-1-carboxamide 29a

The title compound **29a** (40 mg, yield: 35.1%) by following the synthetic route in Example **13** with the starting compound dimethylamine hydrochloride in the second step replaced by the compound methylamine hydrochloride.

MS m/z (ESI): 486.2 [M+1].

### Step 2

### (R)-4-(4-((1-(3-(difluoromethyl)-2-fluorophenyl)ethyl)amino)-2-methyl-7-oxo-7,8-dihy dropyrido[2,3-d]pyrimidin-6-yl)-4-hydroxy-N-methylcyclohexane-1-carboxamide 29

Compound **29a** (40 mg, 82.3 µmol) was dissolved in dichloromethane (0.5 mL) and isopropanol (5 mL), and tris(2,2,6,6-tetramethyl-3,5-heptanedionato)manganese) (15 mg, 24.7 µmol) and phenylsilane (26.7 mg, 247.1 µmol) were added. The mixture was purged with oxygen three times and stirred for 16 h. The reaction solution was concentrated, and the residue was purified by high performance liquid preparative chromatography to give the title compound **29** (3 mg, yield: 7.2%).

MS m/z (ESI): 504.2 [M+1].

¹H NMR (500 MHz, Methanol-*d*₄): δ 8.28 (s, 1H), 7.61 (t, 1H), 7.49 (t, 1H), 7.26 (t, 1H), 7.19-6.84 (t, 1H), 5.81 (q, 1H), 2.75 (s, 3H), 2.54-2.40 (m, 2H), 2.36 (s, 3H), 2.20 (d, 1H), 2.13-2.02 (m, 2H), 1.92 (d, 1H), 1.84 (dd, 2H), 1.74 (d, 1H), 1.67 (d, 3H).

### Test Examples:

### Biological Evaluation

Test Example 1: Inhibitory Ability of Compounds of the Present Disclosure to the Interaction Between the KRAS Protein Subtype G12D or G12V and the SOS1 Protein. The inhibitory ability of the compounds of the present disclosure to the interaction between the KRAS protein subtype G12D or G12V and the SOS1 protein was determined by the following method. The experimental method was briefly described as follows:
I. Materials and instruments
   1. Biotin labeling kit (Dojindo, LK03)
   2. GDP (SIGMA, G7127)
   3. AlphaLISA glutathione acceptor beads (PerkinElmer, AL109C)
   4. AlphaScreen streptavidin donor beads (PerkinElmer, 6760002S)
   5. 384-well microplate (PerkinElmer, 6007290)
   6. BSA (Sangon Biotech, A600332-0100)
   7. Tween-20 (Diamond, A100777-0500)
   8. GST-TEV-SOS1(564-1049) (Viva Biotech, SOS1-191010)
   9. KRas G12D and KrasG12V (provided by Shanghai Panchao Biotechnology Co., Ltd.)
   10. Phosphate buffered saline (PBS) pH 7.4 (Shanghai BasalMedia Technologies Co., LTD., B320)
   11. Multi-mode microplate reader (PerkinElmer, Envision)
II. Procedures
   Preparation:
      1. The experimental buffer was prepared prior to the start of the experiment: 1×PBS + 0.1% BSA + 0.05% tween 20.
      2. KRAS G12D and KRAS-G12V proteins were labeled with biotin using the biotin labeling kit.
   Procedures:
      1. Firstly, the biotin-labeled KRAS G12D or KRAS G12V protein was mixed and incubated with the SOS1 protein and GDP for later use.
      2. AlphaLISA glutathione acceptor beads and AlphaScreen streptavidin donor beads were mixed at a ratio of 1:1 to make the concentration of 40 µg/mL for later use.
      3. The compound was formulated with the experimental buffer to obtain 10 concentration points by 5-fold gradient dilution from an initial concentration of 40 µM.
      4. In the 384-well microplate, 10 µL of a mixture of KRAS G12D or KRAS G12V protein and SOS1 and GDP and 5 µL of the diluted compound were added to each well and incubated at room temperature for 30 min away from the light.
      5. Then, 5 µL of a mixture of AlphaLISA glutathione acceptor beads and AlphaScreen streptavidin donor beads was added to each well and incubated at room temperature for 60 min away from the light.
      6. The fluorescence values were read on a multi-mode microplate reader.
      7. IC₅₀ values of the compounds were calculated using Graphpad Prism.
III. Experimental data The IC₅₀ values measured for the inhibitory ability of the compounds of the present disclosure to the interaction between the KRAS protein subtype G12D or G12V and SOS1 are shown in Table 1.

Table 1. IC₅₀ values for the inhibitory ability of the compounds of the present disclosure to the interaction between the KRAS protein subtype G12D or G12V and the SOS1 protein.

| Example No. | SOS1-G12D IC₅₀/nM | SOS1-G12V IC₅₀/nM |
|---|---|---|
| 1 | 22 | 54 |
| 4 | 17 | 67 |
| 5 | 42 | 79 |
| 6 | 30 | 56 |
| 9 | 8 | 41 |
| 10 | 34 | 80 |
| Shorter retention time in 11-p 1 and 11-p2 | 10 | 38 |
| Longer retention time in 11-p1 and 11-p2 | 21 | 23 |
| 12 | 14 | 50 |
| 13 | 21.5 | 79 |
| Shorter retention time in 14-p1 and 14-p2 | 14 | 47 |
| Longer retention time in 14-p1 and 14-p2 | 107 | 411 |
| 16 | 39 | 136 |
| 17 | 28 | 30 |
| 18-p1 | 20 | 67 |
| 18-p2 | 40 | 112 |
| 19 | 47 | 154 |
| 20 | 19 | 60 |
| 21 | 15 | 54 |
| 22 | 14 | 27 |
| 23 | 10 | 89 |
| 24 | 49 | 133 |
| 25-p1 | 31 | 35 |
| 25-p2 | 37 | 65 |
| 26 | 44 | 65 |
| 27 | 20 | 52 |
| 28-p1 | 100 | 97 |
| 28-p2 | 175 | 187 |
| 29 | 91 | 101 |

Conclusion: the compounds of the present disclosure are able to well inhibit the interaction between the KRAS protein subtype G12D or G12V and the SOS1 protein.

### Test Example 2: Biological Evaluation of Inhibition Experiment for ERK Phosphorylation of H358 Cells

### I. Purpose

This experiment is intended to evaluate the inhibition effect of the compounds of the present disclosure on the KRAS target (containing the G12C mutation) according to IC₅₀ by determining the inhibition effect of the compounds on ERK phosphorylation of the cells.

### II. Method

H358 cells (ATCC, CRL-5807) were cultured in RPMI1640 (Hyclone, SH30809.01) complete medium containing 10% fetal bovine serum. On the first day of the experiment, the H358 cells were seeded into a 96-well plate at a density of 25,000 cells/well with a complete medium to form 190 µL of cell suspension per well. The plate was incubated overnight in a cell incubator at 37 °C with 5% CO₂. The next day, 10 µL of test compound diluted in a gradient prepared with the complete medium was added to each well. The final concentrations of the compound were 9 concentration points obtained by 5-fold gradient dilution from 10 µM. A blank control containing 0.1% DMSO was set. The plate was incubated in a cell incubator at 37 °C with 5% CO₂ for 1 h. 1 h later, the 96-well cell culture plate was taken out, the medium was removed by pipetting, 200 µL PBS (Shanghai BasalMedia Technologies Co., Ltd., B320) was added to each well, and the cells were washed once. PBS was removed by pipetting, 50 µL of lysis buffer (Cisbio, 64KL1FDF) containing a blocking reagent (Cisbio, 64KB1AAC) was added to each well, and the plate was shaken on a shaker at room temperature for 40 min for lysis. After lysis, the lysate was pipetted and mixed well, 16 µL of lysate was transferred from each well to each of two HTRF 96-well assay plates (Cisbio, 66PL96100), and then 4 µL of premixed phospho-ERK1/2 antibody solution (Cisbio, 64AERPEG) or 4 µL of premixed total-ERK1/2 antibody solution (Cisbio, 64NRKPEG) was added to each of the two plates. The microplate was sealed with a sealing membrane, centrifuged for 1 min in a microplate centrifuge, and incubated overnight at room temperature away from the light. On the third day, the fluorescence values at an excitation wavelength of 337 nm and at emission wavelengths of 665 nm and 620 nm were read using a PHERAstar multi-mode microplate reader (BMG Labtech, S/N 471-0361).

### III. Data analysis

IC₅₀ values for inhibitory activity of the compounds were calculated using Graphpad Prism software based on compound concentrations and pERK/total ERK ratio. The results are shown in Table 2 below.

**Table 2. Inhibitory activity data for ERK phosphorylation of H358 cells**

| Example No. | IC₅₀ (nM) |
|---|---|
| 1 | 59 |
| 3 | 55 |
| 4 | 41 |
| 12 | 48 |
| 16 | 218 |
| 17 | 79 |
| 18-p1 | 51 |
| 18-p2 | 222 |
| 20 | 78 |
| 21 | 87 |
| 25-p1 | 101 |
| 25-p2 | 489 |
| 27 | 258 |
| 28-p1 | 231 |
| 28-p2 | 937 |

**Conclusion:** the compounds of the present disclosure have good inhibitory activity on ERK phosphorylation of H358 cells.

### Test Example 3: Biological Evaluation of Inhibition Experiment for Proliferation of H358 Cells

### I. Purpose

This example is intended to evaluate the inhibition effect of the compounds of the present disclosure on the KRAS target (containing the G12C mutation) by testing the inhibition effect of the compounds of the present disclosure on the proliferation of H358 cells.

### II. Method

H358 cells (ATCC, CRL-5807) were cultured in a complete medium, namely RPMI1640 medium (Hyclone, SH30809.01) containing 10% fetal bovine serum (Corning, 35-076-CV). On the first day of the experiment, the H358 cells were seeded into a 96-well low adsorption plate (Corning, CLS7007-24EA) at a density of 1500 cells/well with a complete medium to form 90 µL of cell suspension per well. The plate was centrifuged at 2000 rpm for 5 min at room temperature and then incubated overnight in a cell incubator at 37 °C with 5% CO₂. The next day, 10 µL of test compound diluted in a gradient prepared with the complete medium was added to each well. The final concentrations of the compound were 9 concentration points obtained by 5-fold gradient dilution from 10 µM. A blank control containing 0.1% DMSO was set. The plate was incubated in a cell incubator at 37 °C with 5% CO₂ for 120 h. On the seventh day, the 96-well cell culture plate was taken out and 50 µL of CellTiter-Glo^{®} 3D reagent (Promega, G9682) was added into each well. The plate was shaken at room temperature for 25 min, pipetted and mixed well, from which 50 µL of the mixture was transferred into a white impermeable 96-well plate (PE, 6005290). The luminescence signal values were read using a multi-mode microplate reader (PerkinElmer, VICTOR 3).

### III. Data analysis

IC₅₀ values for the inhibitory activity of the compounds were calculated using Graphpad Prism software. The results are shown in Table 3 below.

**Table 3. Inhibitory activity data for proliferation of H358 cells**

| Example No. | IC₅₀ (nM) |
|---|---|
| 1 | 34 |
| 4 | 97 |
| 5 | 461 |
| 6 | 345 |
| 9 | 106 |
| Shorter retention time in 11-p1 and 11-p2 | 104 |
| Longer retention time in 11-p1 and 11-p2 | 72 |
| 12 | 55 |
| 13 | 137 |
| Shorter retention time in 14-p1 and 14-p2 | 34 |
| Longer retention time in 14-p1 and 14-p2 | 597 |
| 16 | 97 |
| 17 | 65 |
| 18-p1 | 72 |
| 18-p2 | 213 |
| 20 | 118 |
| 21 | 62 |
| 25-p1 | 120.4 |
| 25-p2 | 1020 |
| 26 | 346 |
| 27 | 202 |
| 28-p1 | 243 |
| 28-p2 | 716 |
| 29 | 545 |

Conclusion: the compounds of the present disclosure have a good inhibitory effect on the proliferation of H358 cells.

### Test Example 4: Inhibition Effect of Compounds of the Present Disclosure on Human Liver Microsome CYP450 Enzyme

The inhibition effect of the compounds of the present disclosure on the human liver microsome CYP450 enzyme was determined by the following method:
I. Materials and instruments
   1. Phosphate buffered saline (20× PBS, purchased from Sangon);
   2. NADPH (ACROS, A2646-71-1);
   3. Human liver microsome (Corning Gentest, Cat No, 452161, Lot No. 905002, Donor35);
   4. ABI QTrap 4000 LC-MS System (AB Sciex);
   5. ZORBAX Extend-C18, 3 × 50 mm, 3.5 µm (Agilent, USA);
   6. CYP probe substrate.
II. Procedures
   1. Preparation of solutions

1) Preparation of 100 mM phosphate buffered saline (PBS)
50 mL of PBS solution at the concentration of 2000 mM was diluted to 1000 mL with 950 mL of ultrapure water, mixed uniformly and adjusted to pH 7.4 by a pH meter to obtain the PBS solution of pH 7.4, which was stored in a refrigerator at 4 °C (for a period up to 6 months).
2) Preparation of NADPH solution
An appropriate amount of NADPH powder was precisely weighed, and dissolved in a PBS buffer solution to prepare a solution at the concentration of 5 mM for later use (prepared right before use).
3) Preparation of liver microsome solution
An appropriate amount of human liver microsome stock solution (20 mg/mL) was diluted to obtain a 0.25 mg/mL microsome solution with 7.5 mM MgCl₂ solution for later use (prepared right before use).
4) Preparation of MgCl₂ solution
An appropriate amount of MgCl₂ powder was weighed, prepared into a 300 mM stock solution with a PBS solution, which was stored in a 4 °C refrigerator for later use. The solution was precisely measured, and diluted to obtain a 7.5 mM working solution with 100 mM PBS solution (prepared right before use).
5) Preparation of test compound solutions
a. An appropriate amount of test compound standard was precisely weighed, and prepared into a stock solution at the concentration of 30 mM with DMSO, which was stored in a refrigerator at 4 °C.
b. An appropriate amount of the stock solution was precisely pipetted, and diluted into series solution I at the concentrations of 10 mM, 3 mM, 1 mM, 0.3 mM, 0.03 mM and 0.003 mM with an appropriate amount of DMSO solution. An appropriate amount of the above series solution I was precisely pipetted, and diluted to obtain series solution II at the concentrations of 3 mM, 1 mM, 0.3 mM, 0.1 mM, 0.03 mM, 0.003 mM and 0.0003 mM with an appropriate amount of acetonitrile. An appropriate amount of the above series solution II was precisely pipetted, and diluted with PBS to obtain a working solution at the concentrations of 150 µM, 50 µM, 15 µM, 5 µM, 1.5 µM, 0.15 µM and 0.015 µM for later use.

6) Selection of CYP probe substrates and selective inhibitors
a. Preparation of a probe substrate stock solution: an appropriate amount of each probe substrate was weighed and prepared into a stock solution with DMSO, with the concentrations shown in Table 4 below.
b. Preparation of a probe substrate working solution: an appropriate amount of probe substrate stock solution was pipetted, and subjected to a 200-fold dilution with a PBS solution to obtain the probe substrate working solution, with the concentration shown in Table 4 below.

**Table 4.**

| CYP | Probe substrate | Stock solution concentration (mM) | Working solution concentration (µM) |
|---|---|---|---|
| 2C19 | (S)-mephenytoin | 20 | 100 |
| 3A4M | Midazolam | 3 | 15 |

### 2. Liver microsome incubation and sample preparation

The concentrations of the proteins, substrates and inhibitors in the reaction system are shown in Table 5 below.

**Table 5.**

| CYP | Probe substrate | Substrate concentration (µM) | Protein concentration (mg/mL) | Test compounds (µM) |
|---|---|---|---|---|
| 2C19 | (S)-mephenytoin | 20 | 0.1 | 30, 10, 3, 1, 0.3, 0.03, 0.003, 0 |
| 3A4M | Midazolam | 3 | | |

### 3. Procedures

1) 40 µL of human liver microsome solution (0.25 mg/mL), 20 µL of probe substrate solution and 20 µL of test compound solution were precisely pipetted into a 96-well plate and pre-incubated for 5 min under a water bath at 37 °C.
2) After 5 min of pre-incubation, the mixture was taken out, 20 µL of 5 mM NADPH solution was added to initiate the reaction, and the resulting mixture was incubated for 30 min under a water bath at 37 °C. Each sample was run in duplicate.
3) After the incubation was completed, 250 µL of acetonitrile solution containing an internal standard was added to terminate the reaction, and the mixture was shaken at 800 rpm for 10 min and centrifuged at 3700 rpm for 10 min. 100 µL of supernatant was precisely pipetted, diluted with 80 µL of distilled water, and shaken at 800 rpm for 10 min. The supernatant was pipetted for LC-MS/MS analysis.

The values were calculated by Graphpad Prism to obtain the IC₅₀ values for the inhibition of drugs on CYP2C19 (S)-mephenytoin 4'-hydroxylation and CYP3A4M midazolam 1-hydroxylation metabolism in human liver microsomes, as shown in Table 6.

**Table 6. IC₅₀ values of the compounds of the present disclosure for CYP2C19 (S)-mephenytoin site of metabolism and CYP3A4M midazolam site of metabolism in human liver microsomes**

| Example No. | IC₅₀(µM)-CYP2C19 | IC₅₀(µM)-CYP3A4M |
|---|---|---|
| 18-p1 | >30 | >30 |

Conclusion: metabolic drug interactions based on CYP2C19 (S)-mephenytoin 4'-hydroxylation and CYP3A4M midazolam 1-hydroxylation sites of metabolism do not occur over the 30 µM concentration range of the compounds of the present disclosure.

### Test Example 5: Effect of Compound of the Present Disclosure on hERG Potassium Ion Channel

### I. Purpose

This example is intended to test the blocking effect of the compounds of the present disclosure on hERG potassium currents in a stable cell strain transfected with an hERG potassium channel by a manual patch-clamp assay.

### II. Method

### 1. Cell culture

The cells used in this experiment were from a CHO cell line (provided by Sophion Bioscience, Denmark) transfected with hERG cDNA and stably expressing hERG channel at cell passage number of P5. Cells were cultured in a medium containing the following components (all from Invitrogen): Ham's F12 medium, 10% (v/v) inactivated fetal bovine serum, 100 µg/mL hygromycin B, and 100 µg/mL geneticin.

CHO hERG cells were grown in a culture dish containing the above culture medium and cultured in an incubator at 37 °C with 5% CO₂. 24-48 h before the electrophysiological experiment, CHO hERG cells were transferred to round slides placed in the culture dish and grown in the same culture medium and culture conditions as above. The density of CHO hERG cells on each round slide was required to achieve the requirement that most cells were independent and individual.

### 2. Experimental solutions

**Table 7. Compositions of intracellular and extracellular fluids**

| Reagent | Extracellular fluid (mM) (EC 0.0.0 NaCl-Ringer's solution) | Intracellular fluid (mM) (IC 0.0.0 KCl-Ringer's solution) |
|---|---|---|
| CaCl₂ | 2 | 5.4 |
| MgCl₂ | 1 | 1.8 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.4 (adjusted with NaOH) | 7.25 (adjusted with NaOH) |
| Osmotic pressure | Osmotic pressure of about 305 mOsm | Osmotic pressure of about 295 mOsm |

**Table 8. Reagent details**

| Reagent | Cat. No. | Batch No. | Molecular weight | Supplier |
|---|---|---|---|---|
| NaCl | S1679-1KG | WXBC1368V | 58.44 | Sigma |
| KCl | 31248-100G | WXBC2571V | 74.55 | Sigma |
| CaCl₂ | 21114-1L | BCBM6063V | 110.98 | Sigma |
| MgCl₂•6H₂O | M7304-100G | V900020-500G | 203.30 | Sigma |
| HEPES | H3375-1KG | SLBP2246V | 238.30 | Sigma |
| Glucose | G8270-1KG | WXBC2393V | 180.16 | Sigma |
| EGTA | 03777-50G | SLBP2807V | 380.15 | Sigma |
| Na₂-ATP | A-7699-5G | SLBJ8915V | 551.14 | Sigma |
| NaOH | 35254-1L | BCBG6297V | 40.00 | Sigma |
| KOH | 232041-50G | SLBK9251V | 56.00 | Sigma |

### 3. Electrophysiological recording system

In this experiment, a manual patch-clamp system (HEKA EPC-10 signal amplifier and digital conversion system, purchased from HEKA Electronics, Germany) was used for whole cell current recording. Round slides with CHO hERG cells grown on the surface were placed in an electrophysiological recording chamber under an inverted microscope. The chamber was continuously perfused with extracellular fluid (approximately 1 mL/min). The experimental procedures were performed by conventional whole-cell patch-clamp current recording technology. Unless otherwise stated, the experiment was performed at room temperature (-25 °C). The cells were clamped at a voltage of -80 mV. The cell clamp voltage was depolarized to +20 mV to activate the hERG potassium channel, and then clamped to -50 mV 5 s later to eliminate inactivation and generate tail currents. The tail current peak was used as a value for the numeral value of the hERG current. After the hERG potassium currents recorded in the above step were stable under the continuous extracellular fluid perfusion in the recording chamber, the drug to be tested could be added for perfusion until the inhibition effect of the drug on the hERG current reached a stable state. Generally, the last superposition of 3 consecutive current recording lines is used as a criterion for determine whether a stable state is reached. After reaching a stable state, the chamber was flushed by perfusion with extracellular fluid until the hERG currents returned to the level before dosing. One or more drugs, or multiple concentrations of the same drug, can be tested on one cell, but extracellular fluid flushes are required between different drugs. Cisapride (purchased from Sigma) was used as a positive control in the experiment to ensure that the cells used were of normal quality.

### 4. Procedures

To obtain IC₅₀ of the compounds, the following concentrations (30 µM, 10 µM, 3 µM, 1 µM, 0.3 µM and 0.1 µM) were selected for testing. Prior to the test, the compound was formulated into a 10 mM DMSO stock solution with DMSO (Sigma), which was diluted in a gradient to obtain stock solutions at 3 mM, 1 mM, 0.3 mM and 0.1 mM. The stock solutions were then diluted with extracellular fluid to the final µM test concentrations. The final concentration of DMSO was 0.1% in each concentration of compound solution except for the 30 µM compound test solution in which the final concentration of DMSO was about 0.3%. The test concentration of the positive control cisapride was 0.1 µM. All compound solutions were routinely sonicated and shaken for 5 to 10 min to ensure complete dissolution of the compounds.

The experimental data were analyzed by data analysis software supplied by HEKA Patchmaster(V2x73.2), Microsoft Excel, and Graphpad Prism 5.0.

### 5. Test results

The blocking effect of the compound of the present disclosure on hERG potassium currents was determined by the above test. The IC₅₀ values obtained are shown in Table 9.

**Table 9. IC₅₀ for the blocking effect of the compound of the present disclosure on the hERG potassium ion channel**

| Example No. | IC₅₀(µM) |
|---|---|
| 18-p1 | >30 |

Conclusion: the compound of the present disclosure has a weak inhibitory effect on hERG and can reduce side effects caused by the hERG pathway.

Test Example 6: Pharmacokinetic Evaluation of Compound of the Present Disclosure in Mice

### 1. Abstract

The drug concentration in the plasma of the test animals (mice) at different time points after intragastric administration (i.g.) of compound **18-p1** was determined by using an LC/MS/MS method. The pharmacokinetic behavior of the compound of the present disclosure in mice was studied and its pharmacokinetic profile was evaluated.

### 2. Test protocol

### 2.1. Test drug

### Compound 18-p1

### 2.2. Test animals

Nine C57 mice, female, purchased from Beijing Vital River Laboratory Animal Technology Co., Ltd., with animal production license number of SCXK (Shanghai) 2017-0005.

### 2.3. Drug formulation

An amount of compound **18-p1** was weighed, dissolved with 5% by volume of DMSO and 5% tween 80 (Shanghai Titan Scientific Co., Ltd.), and then prepared into a 0.1 mg/mL clear solution with 90% normal saline.

### 2.4. Administration

Intragastric administration: the mice were intragastrically administered at a dose of 2.0 mg/kg and a volume of 20.0 mL/kg.

### 3. Procedures

The compound **18-p1** was administered intragastrically to mice, and 0.1 mL of blood was collected from the orbit before administration and 0.25 h, 0.5 h, 1.0 h, 2.0 h, 4.0 h, 6.0 h, 8.0 h, 11.0 h and 24.0 h after administration. The blood was placed in an EDTA-K2 anticoagulation tube and centrifuged at 10,000 rpm for 1 min (4 °C), and plasma was separated out within 1 h and then stored at -20 °C for testing. The process from blood sampling to centrifugation was performed under an ice bath.

The content of the test compounds at different concentrations in plasma of mice after administration was determined: 10 µL of plasma of mice at each time point after administration was taken, and 200 µL of methanol (containing an internal standard solution camptothecin, 100 ng/mL) was added; the mixture was vortexed for 1 min, and centrifuged for 10 min (18,000 r/min). 3 µL of supernatant was taken from the plasma sample for LC/MS/MS analysis.

### 4. Pharmacokinetic parameters

**Table 10. Pharmacokinetic parameters of the compound of the present disclosure in mice**

| Route of administration | Example No. | Dose of administration | Plasma concentration | Area under curve | Half-life | Clearance | Apparent distribution volume |
|---|---|---|---|---|---|---|---|
| | | (mg/kg) | Cₘₐₓ (ng /mL) | AUC_{0- t} (ng /mL*h) | T1/2 (h) | CLz/F (ml/min/kg) | Vz/F (ml/kg) |
| Intragastric administration | Compound **18-p1** | 2 | 56.7 | 146 | 1.2 | 225.2 | 23384.3 |

Conclusion: the compound of the present disclosure has good pharmacokinetic absorption activity in mice, and has pharmacokinetic advantages.

### Test Example 7: Pharmacokinetic Evaluation of Compound of the Present Disclosure in Nude Mice

### 1. Abstract

The drug concentration in the plasma of the test animals (nude mice) at different time points after intragastric administration (i.g.) of compound **18-p1** was determined by using an LC/MS/MS method. The pharmacokinetic behavior of the compound of the present disclosure in nude mice was studied and its pharmacokinetic profile was evaluated.

### 2. Test protocol

### 2.1. Test drug

### Compound 18-p1

### 2.2. Test animals

Nine BALB/C nude mice, female, intragastric administration, purchased from Beijing Vital River Laboratory Animal Technology Co., Ltd., with animal production license number of SCXK (Shanghai) 2017-0005.

### 2.3. Drug formulation

An amount of compound **18-p1** was weighed, dissolved in 0.5% by volume of CMCNa, and then prepared into a 2.5 mg/mL clear solution with 99.5% normal saline.

### 2.4. Administration

The nude mice were intragastrically administered at a dose of 50.0 mg/kg and a volume of 20.0 mL/kg.

### 3. Procedures

The compound **18-p1** was administered intragastrically to nude mice, and 0.1 mL of blood was collected from the orbit before administration and 0.25 h, 0.5 h, 1.0 h, 2.0 h, 4.0 h, 6.0 h, 8.0 h, 11.0 h and 24.0 h after administration. The blood was placed in an EDTA-K2 anticoagulation tube and centrifuged at 10,000 rpm for 1 min (4 °C), and plasma was separated out within 1 h and then stored at -20 °C for testing. The process from blood sampling to centrifugation was performed under an ice bath.

The content of the test compounds at different concentrations in plasma of nude mice after administration was determined: 25 µL of plasma of nude mice at each time point after administration was taken, and 200 µL of acetonitrile (containing 50 µL of internal standard solution camptothecin, 100 ng/mL) was added; the mixture was vortexed for 5 min, and centrifuged for 10 min (4000 r/min). 0.5 µL of supernatant was taken from the plasma sample for LC/MS/MS analysis.

### 4. Pharmacokinetic parameters

**Table 11. Pharmacokinetic parameters of the compound of the present disclosure in nude mice**

| Route of administration | Example No. | Dose of administration | Plasma concentration | Area under curve | Half-life | Clearance | Apparent distribution volume |
|---|---|---|---|---|---|---|---|
| | | (mg/kg) | Cₘₐₓ (ng /mL) | AUC₀₋ₜ (ng /mL^{∗}h) | T1/2 (h) | CLz/F (ml/min/kg) | Vz/F (ml/kg) |
| Intragastric administration | Compound **18-p1** | 50 | 1555 | 5146 | 1.2 | 160 | 16587 |

Conclusion: the compound of the present disclosure has good pharmacokinetic absorption activity in nude mice, and has pharmacokinetic advantages.

Test Example 8: Pharmacokinetic Evaluation of Compound of the Present Disclosure in Dogs

### 1. Abstract

The drug concentration in the plasma of the test animals (dogs) at different time points after intragastric administration (i.g.) of compound **18-p1** was determined by using an LC/MS/MS method. The pharmacokinetic behavior in dogs of the compound of the present disclosure was studied and its pharmacokinetic profile was evaluated.

### 2. Test protocol

### 2.1. Test drug

### Compound 18-p1

### 2.2. Test animals

Four beagle dogs, half male and half female, fasted overnight, supplied by animal reserve bank (999M-004). All animals were beagle dogs qualified for physical examination and healthy without abnormalities.

### 2.3. Drug formulation

An amount of compound **18-p1** was weighed and dissolved in 5% by volume of DMSO, 20% by volume of PG, and 20% by volume of PEG400, and then prepared into a 0.4 mg/mL clear solution with 55% normal saline.

### 2.4. Administration

The administration was performed at a dose of 2.0 mg/kg and a volume of 5.0 mL/kg.

### 3. Procedures

The compound **18-p1** was administered intragastrically to dogs, and 1.0 mL of blood was collected from jugular vein or forelimb vein before administration and 0.25 h, 0.5 h, 1.0 h, 2.0 h, 4.0 h, 6.0 h, 8.0 h, 12.0 h and 24.0 h after administration. The blood was placed in an EDTA-K2 anticoagulation tube and centrifuged at 10,000 rpm for 5 min (4 °C), and plasma was separated out within 1 h and then stored at -80 °C for testing. The process from blood sampling to centrifugation was performed under an ice bath. Food intake was resumed 3 h after administration.

The content of the test compounds at different concentrations in plasma of dogs after administration was determined: 25 µL of plasma of dogs at each time point after administration was taken, and 200 µL of acetonitrile (containing 50 µL of internal standard solution camptothecin, 100 ng/mL) was added; the mixture was vortexed for 5 min, and centrifuged for 10 min (4000 r/min). 0.3 µL of supernatant was taken from the plasma sample for LC/MS/MS analysis.

### 4. Pharmacokinetic parameters

**Table 12. Pharmacokinetic parameters of the compound of the present disclosure in dogs**

| Route of administration | Example No. | Dose of administration | Plasma concentration | Area under curve | Half-life | Clearance | Apparent distribution volume |
|---|---|---|---|---|---|---|---|
| | | (mg/kg) | Cₘₐₓ (ng /mL) | AUC₀₋ₜ (ng /mL*h) | T1/2 (h) | CLz/F (ml/min/kg) | Vz/F (ml/kg) |
| Intragastric administration | Compound **18-p1** | 2 | 710 | 4597 | 3.91 | 7.64 | 2631 |

Conclusion: the compound of the present disclosure has good pharmacokinetic absorption activity in dogs, and has pharmacokinetic advantages.

## Claims

1. A compound of general formula (I) or a tautomer, mesomer, racemate, enantiomer or diastereomer thereof or a mixture thereof, or a pharmaceutically acceptable salt thereof: 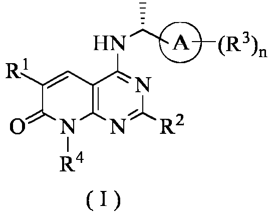 wherein:
ring A is aryl or heteroaryl;
R¹ is selected from the group consisting of hydrogen, alkyl, alkoxy, haloalkyl, haloalkoxy, hydroxyalkyl, cycloalkyloxy, heterocyclyloxy, alkenyl, alkynyl, hydroxy, cyano, amino, -NR⁵R⁶, nitro, cycloalkyl, heterocyclyl, aryloxy, heteroaryloxy, aryl and
heteroaryl, wherein the alkyl, alkoxy, cycloalkyloxy, heterocyclyloxy, cycloalkyl, heterocyclyl, aryloxy, heteroaryloxy, aryl and heteroaryl are each independently and optionally substituted with one or more substituents selected from the group consisting of halogen, alkyl, alkoxy, haloalkoxy, hydroxy, amino, oxo, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
R² is selected from the group consisting of hydrogen, halogen, alkyl, haloalkyl, alkoxy, haloalkoxy, hydroxyalkyl, cyano, amino and cycloalkyl;
R³ are identical or different and are each independently selected from the group consisting of hydrogen, halogen, alkyl, alkoxy, haloalkyl, haloalkoxy, hydroxyalkyl, alkenyl, alkynyl, hydroxy, cyano, amino, -(CH₂)ᵣNR⁵R⁶, cycloalkyl, heterocyclyl, aryl and heteroaryl, wherein the alkyl, haloalkyl, cycloalkyl, heterocyclyl, aryl and heteroaryl are each independently and optionally substituted with one or more substituents selected from the group consisting of halogen, alkyl, alkoxy, haloalkyl, haloalkoxy, hydroxy, nitro, amino, -(CH₂)ₛNR⁹R¹⁰, cyano, cycloalkyl, heterocyclyl, aryl and heteroaryl;
R⁴ is selected from the group consisting of hydrogen, alkyl and cycloalkyl, wherein the alkyl and cycloalkyl are each independently and optionally substituted with one or more substituents selected from the group consisting of halogen, alkyl, alkoxy, haloalkyl, haloalkoxy, hydroxy, nitro, amino, cyano, cycloalkyl, heterocyclyl, aryl and heteroaryl;
R⁵ and R⁶ are identical or different and are each independently selected from the group consisting of hydrogen, alkyl, haloalkyl, hydroxyalkyl, hydroxy, amino, cycloalkyl and heterocyclyl;
R⁷ is selected from the group consisting of hydrogen, alkyl, haloalkyl, hydroxyalkyl, cycloalkyl and heterocyclyl;
R⁸ are identical or different and are each independently selected from the group consisting of hydrogen, alkyl, haloalkyl, hydroxyalkyl, hydroxy, amino, cycloalkyl and
heterocyclyl, wherein the alkyl, haloalkyl, cycloalkyl and heterocyclyl are each independently and optionally substituted with one or more substituents selected from the group consisting of halogen, alkyl, alkoxy, haloalkyl, haloalkoxy, hydroxy, nitro, amino, cycano, cycloalkyl, heterocyclyl, aryl and heteroaryl;
R⁹ and R¹⁰ are identical or different and are each independently selected from the group consisting of hydrogen, alkyl, haloalkyl, hydroxyalkyl, hydroxy, amino, cycloalkyl and heterocyclyl;
n is 1, 2, 3 or 4;
p is 0, 1, 2 or 3;
q is 0, 1, 2 or 3;
r is 0, 1, 2 or 3; and
s is 0, 1, 2 or 3.

2. The compound of general formula (I) or the tautomer, mesomer, racemate, enantiomer or diastereomer thereof or the mixture thereof, or the pharmaceutically acceptable salt thereof according to claim 1, wherein R¹ is selected from the group consisting of hydrogen, alkyl, alkoxy, haloalkyl, haloalkoxy, hydroxyalkyl, cycloalkyloxy, heterocyclyloxy, alkenyl, alkynyl, hydroxy, cyano, amino, -NR⁵R⁶, nitro, cycloalkyl, heterocyclyl, aryl and heteroaryl, wherein the alkyl, alkoxy, cycloalkyloxy, heterocyclyloxy, cycloalkyl, heterocyclyl, aryl and heteroaryl are each independently and optionally substituted with one or more substituents selected from the group consisting of halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl; R⁴ is selected from the group consisting of hydrogen, alkyl and cycloalkyl; R⁸ is selected from the group consisting of hydrogen, alkyl, haloalkyl, hydroxyalkyl, hydroxy, amino, cycloalkyl and heterocyclyl; R⁵, R⁶, R⁷, R⁹, R¹⁰, p and q are as defined in claim 1.

3. The compound of general formula (I) or the tautomer, mesomer, racemate, enantiomer or diastereomer thereof or the mixture thereof, or the pharmaceutically acceptable salt thereof according to claim 1 or 2, wherein ring A is 6- to 10-membered aryl, preferably phenyl or

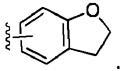

4. The compound of general formula (I) or the tautomer, mesomer, racemate, enantiomer or diastereomer thereof or the mixture thereof, or the pharmaceutically acceptable salt thereof according to any one of claims 1 to 3, being a compound of general formula (II) or a tautomer, mesomer, racemate, enantiomer or diastereomer thereof or a mixture thereof, or a pharmaceutically acceptable salt thereof: 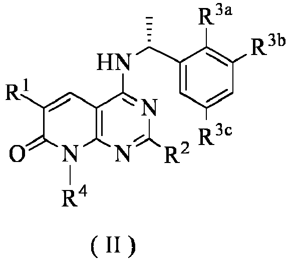 wherein:
R^{3a}, R^{3b} and R^{3c} are identical or different and are each independently selected from the group consisting of hydrogen, halogen, alkyl, alkoxy, haloalkyl, haloalkoxy, hydroxyalkyl, alkenyl, alkynyl, hydroxy, cyano, amino, -(CH₂)ᵣNR⁵R⁶, cycloalkyl, heterocyclyl, aryl and heteroaryl, wherein the alkyl, haloalkyl, cycloalkyl, heterocyclyl,
aryl and heteroaryl are each independently and optionally substituted with one or more substituents selected from the group consisting of halogen, alkyl, alkoxy, haloalkyl, haloalkoxy, hydroxy, nitro, amino, -(CH₂)ₛNR⁹R¹⁰, cyano, cycloalkyl, heterocyclyl, aryl and heteroaryl; preferably, R^{3a}, R^{3b} and R^{3c} are identical or different and are each independently selected from the group consisting of hydrogen, halogen, C₁₋₆ alkyl, C₁₋₆ haloalkyl, hydroxy, cyano and amino, wherein the C₁₋₆ alkyl and C₁₋₆ haloalkyl are optionally substituted with one or more substituents selected from the group consisting of halogen, hydroxy and C₁₋₆ alkoxy;
R¹, R², R⁴, R⁵, R⁶, R⁹, R¹⁰, s and r are as defined in claim 1.

5. The compound of general formula (I) or the tautomer, mesomer, racemate, enantiomer or diastereomer thereof or the mixture thereof, or the pharmaceutically acceptable salt thereof according to any one of claims 1 to 4, wherein R⁴ is hydrogen or C₁₋₆ alkyl, preferably, R⁴ is hydrogen.

6. The compound of general formula (I) or the tautomer, mesomer, racemate, enantiomer or diastereomer thereof or the mixture thereof, or the pharmaceutically acceptable salt thereof according to any one of claims 1 to 5, wherein R¹ is selected from the group consisting of C₁₋₆ alkyl, C₁₋₆ alkoxy, C₁₋₆ haloalkyl, C₁₋₆ haloalkoxy, C₁₋₆ hydroxyalkyl, 3- to 10-membered cycloalkyloxy, 3- to 10-membered heterocyclyloxy, 3- to 10-membered cycloalkyl, 3- to 10-membered heterocyclyl, 6- to 10-membered aryl, and 5- to 10-membered heteroaryl, wherein the C₁₋₆ alkyl, C₁₋₆ alkoxy, 3- to 10-membered cycloalkyloxy, 3- to 10-membered heterocyclyloxy, 3- to 10-membered cycloalkyl, 3- to 10-membered heterocyclyl, 6- to 10-membered aryl, and 5- to 10-membered heteroaryl are each independently and optionally substituted with one or more substituents selected from the group consisting of halogen, C₁₋₆ alkyl, C₁₋₆ alkoxy, C₁₋₆ haloalkoxy, hydroxy, amino, cyano, -C(O)(CH₂)_{q}OR⁷, -NHC(O)R⁸, -C(O)R⁸, -NR⁹R¹⁰, and -C(O)(CH₂)_{P}NR⁹R¹⁰; R⁷ to R¹⁰, q and p are as defined in claim 1.

7. The compound of general formula (I) or the tautomer, mesomer, racemate, enantiomer or diastereomer thereof or the mixture thereof, or the pharmaceutically acceptable salt thereof according to any one of claims 1 to 5, wherein R¹ is 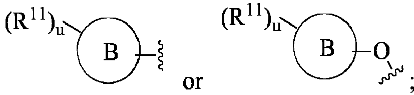
ring B is selected from the group consisting of cycloalkyl, heterocyclyl, aryl and heteroaryl; preferably, R¹ is selected from the group consisting of 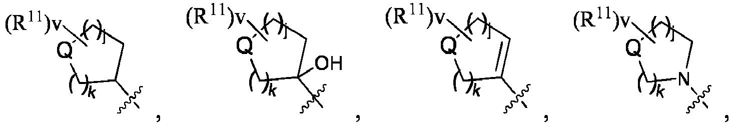 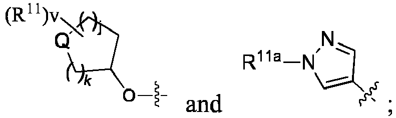
Q is selected from the group consisting of oxygen, sulfur, 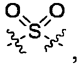 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
u is 0, 1, 2, 3, 4 or 5;
v is 0, 1, 2 or 3;
R⁷ to R¹⁰, p and q are as defined in claim 1.

8. The compound of general formula (I) or the tautomer, mesomer, racemate, enantiomer or diastereomer thereof or the mixture thereof, or the pharmaceutically acceptable salt thereof according to any one of claims 1 to 7, wherein R² is C₁₋₆ alkyl.

9. The compound of general formula (I) or the tautomer, mesomer, racemate, enantiomer or diastereomer thereof or the mixture thereof, or the pharmaceutically acceptable salt thereof according to any one of claims 1 to 3 and claims 5 to 8, wherein R³ are identical or different and are each independently selected from the group consisting of hydrogen, halogen, C₁₋₆ alkyl, C₁₋₆ haloalkyl, hydroxy, cyano and amino, wherein the C₁₋₆ alkyl and C₁₋₆ haloalkyl are optionally substituted with one or more substituents selected from the group consisting of halogen, hydroxy and C₁₋₆ alkoxy.

10. The compound of general formula (I) or the tautomer, mesomer, racemate, enantiomer or diastereomer thereof or the mixture thereof, or the pharmaceutically acceptable salt thereof according to any one of claims 1 to 9, selected from the group consisting of any one of the following compounds:

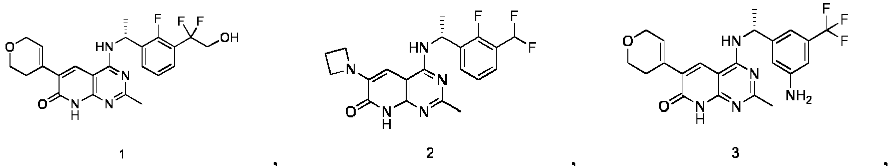

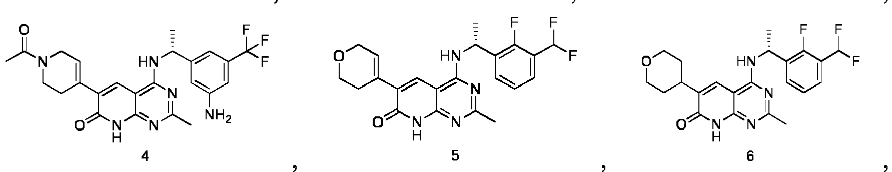

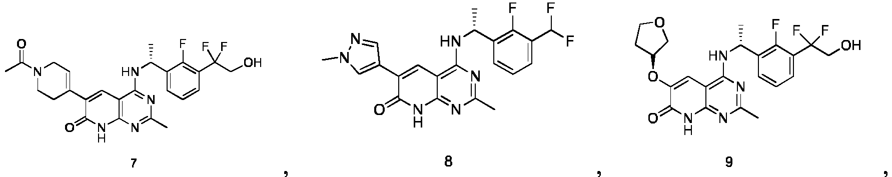

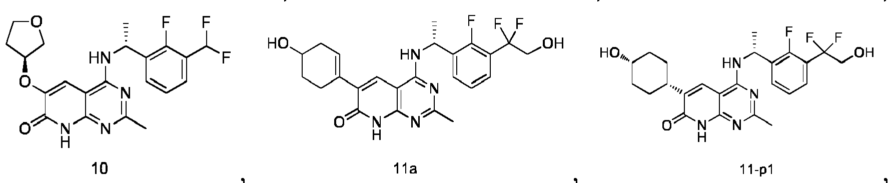

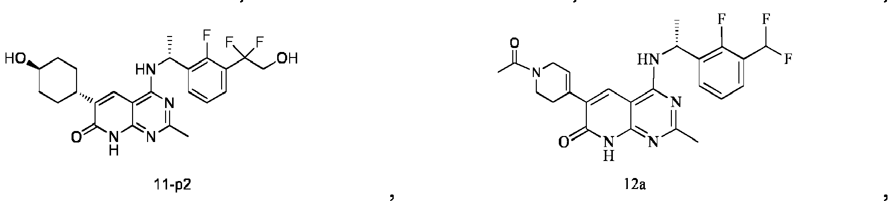

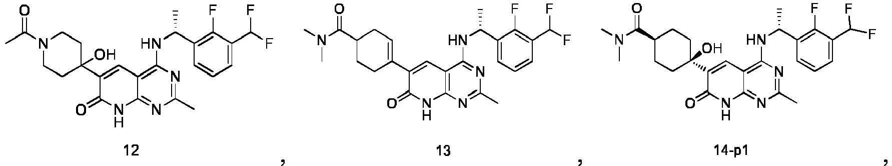

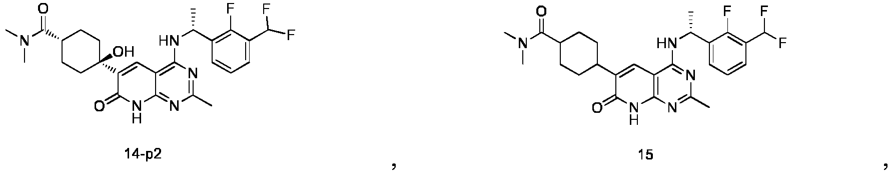

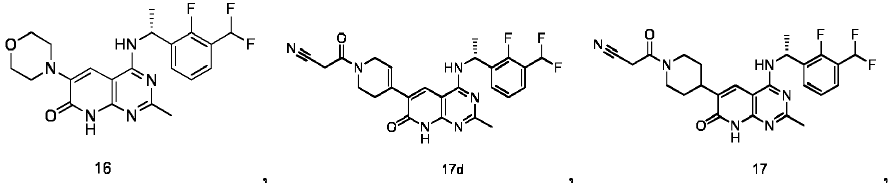

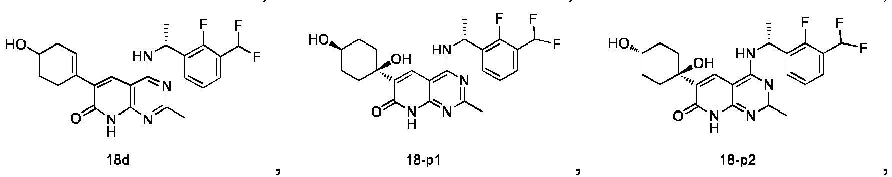

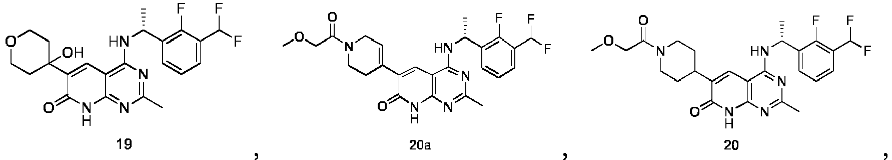

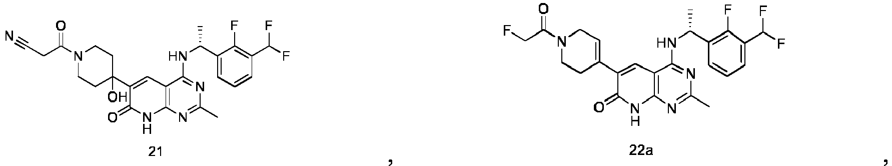

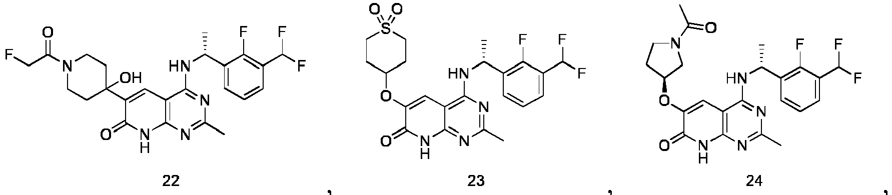

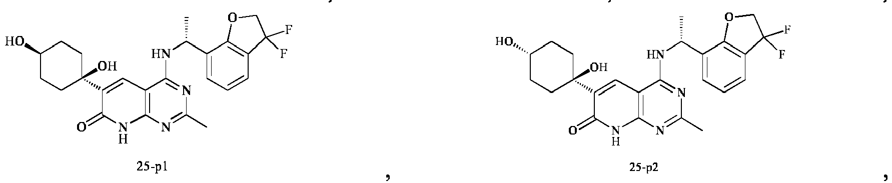

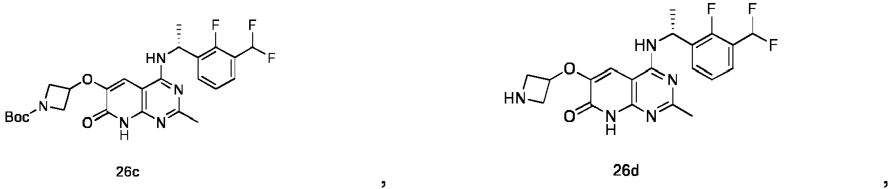

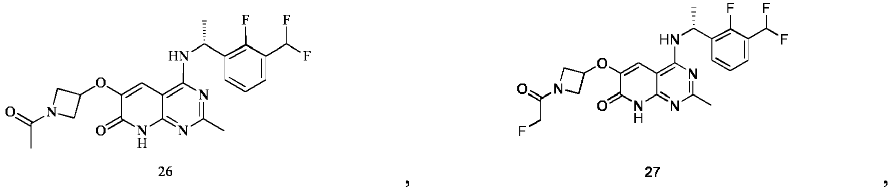

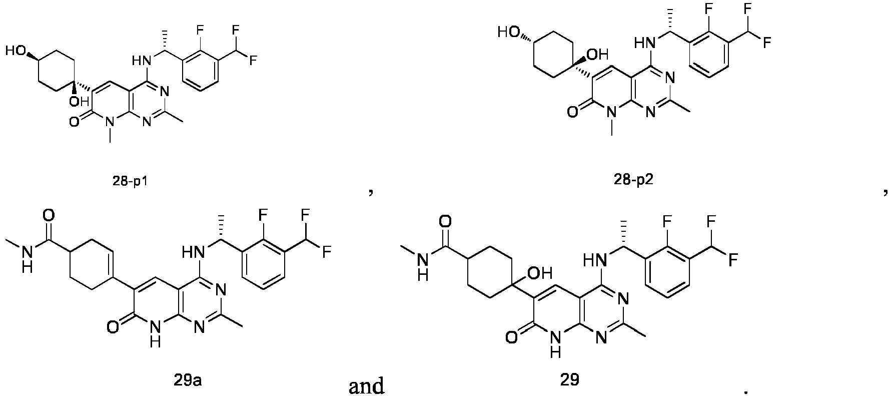

11. A compound of general formula (IA) or a tautomer, mesomer, racemate, enantiomer or diastereomer thereof or a mixture thereof, or a pharmaceutically acceptable salt thereof: 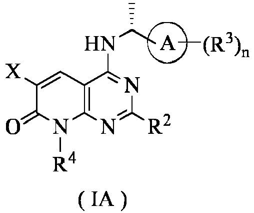 wherein:
X is halogen, preferably Br;
ring A, R² to R⁴ and n are as defined in claim 1.

12. The compound of general formula (IA) or the tautomer, mesomer, racemate, enantiomer or diastereomer thereof or the mixture thereof, or the pharmaceutically acceptable salt thereof according to claim 11, selected from the group consisting of any one of the following compounds: 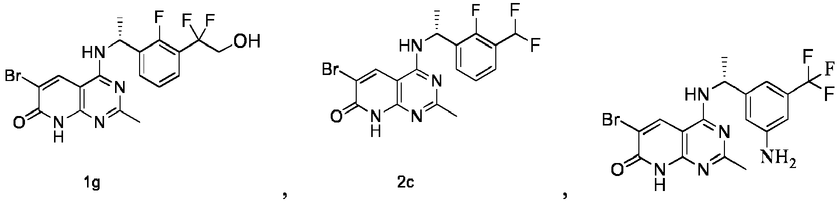 and

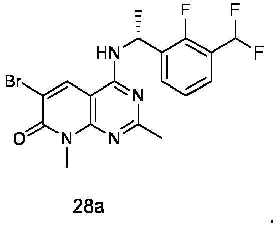

13. A compound of general formula (IB) or a tautomer, mesomer, racemate, enantiomer or diastereomer thereof or a mixture thereof, or a pharmaceutically acceptable salt thereof: 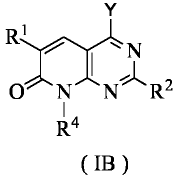 wherein: y is halogen, preferably Cl;
R² is selected from the group consisting of halogen, alkyl, haloalkyl, alkoxy, haloalkoxy, hydroxyalkyl, cyano and cycloalkyl;
R¹ and R⁴ are as defined in claim 1.

14. The compound of general formula (IB) or the tautomer, mesomer, racemate, enantiomer or diastereomer thereof or the mixture thereof, or the pharmaceutically acceptable salt thereof according to claim 13, being:

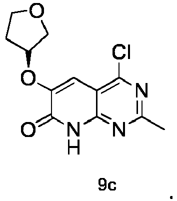

15. A method for preparing a compound of general formula (I) or a tautomer, mesomer, racemate, enantiomer or diastereomer thereof or a mixture thereof, or a pharmaceutically acceptable salt thereof, comprising the following step: 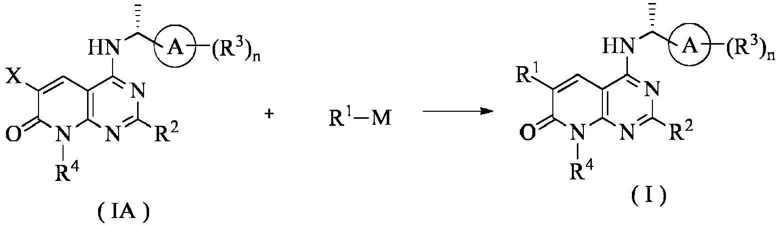 subjecting a compound of general formula (IA) or a tautomer, mesomer, racemate, enantiomer or diastereomer thereof or a mixture thereof, or a pharmaceutically acceptable salt thereof to a reaction with R¹-M to give a compound of general formula (I) or a tautomer, mesomer, racemate, enantiomer or diastereomer thereof or a mixture thereof, or a pharmaceutically acceptable salt thereof;
wherein:
X is halogen, preferably Br;
M is selected from the group consisting of 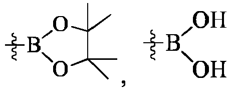 and hydrogen;
ring A, R¹ to R⁴ and n are as defined in claim 1.

16. A method for preparing a compound of general formula (I) or a tautomer, mesomer, racemate, enantiomer or diastereomer thereof or a mixture thereof, or a pharmaceutically acceptable salt thereof, comprising the following step: 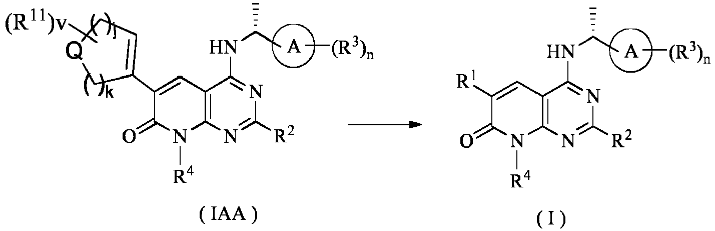 subjecting a compound of general formula (IAA) or a tautomer, mesomer, racemate, enantiomer or diastereomer thereof or a mixture thereof, or a pharmaceutically acceptable salt thereof to an oxidation reaction to give a compound of general formula (I) or a tautomer, mesomer, racemate, enantiomer or diastereomer thereof or a mixture thereof, or a pharmaceutically acceptable salt thereof;
wherein:
R¹ is 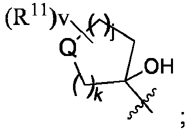
Q is selected from the group consisting of oxygen, sulfur, 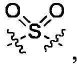 NR^{11a} and CR^{11b}R^{11c};
R^{11a}, R^{11b}, R^{11c} and R¹¹ are identical or different and are each independently selected from the group consisting of hydrogen, halogen, alkyl, alkoxy, haloalkoxy, hydroxy, amino, -C(O)(CH₂)_{q}OR⁷, -NHC(O)R⁸, -C(O)R⁸, -NR⁹R¹⁰, -C(O)(CH₂)ₚNR⁹R¹⁰, nitro, cyano, cycloalkyl, heterocyclyl, aryl and heteroaryl;
j is 0, 1 or 2;
k is 1 or 2;
v is 0, 1, 2 or 3;
ring A, R² to R⁴, R⁷ to R¹⁰, p, q and n are as defined in claim 1.

17. A method for preparing a compound of general formula (I) or a tautomer, mesomer, racemate, enantiomer or diastereomer thereof or a mixture thereof, or a pharmaceutically acceptable salt thereof, comprising the following step: 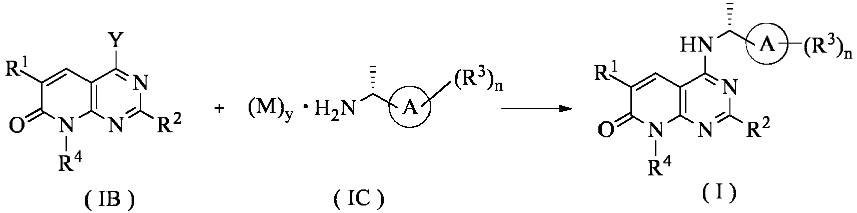 subjecting a compound of general formula (IB) or a tautomer, mesomer, racemate, enantiomer or diastereomer thereof or a mixture thereof, or a pharmaceutically acceptable salt thereof to a reaction with a compound of general formula (IC) to give a compound of general formula (I) or a tautomer, mesomer, racemate, enantiomer or diastereomer thereof or a mixture thereof, or a pharmaceutically acceptable salt thereof; wherein:
M is HCl;
y is 0 or 1;
Y is halogen, preferably Cl;
ring A, R¹ to R⁴ and n are as defined in claim 1.

18. A pharmaceutical composition, comprising a therapeutically effective amount of the compound of general formula (I) or the tautomer, mesomer, racemate, enantiomer or diastereomer thereof or the mixture thereof, or the pharmaceutically acceptable salt thereof according to any one of claims 1 to 10, and one or more pharmaceutically acceptable carriers, diluents or excipients.

19. Use of the compound of general formula (I) or the tautomer, mesomer, racemate, enantiomer or diastereomer thereof or the mixture thereof, or the pharmaceutically acceptable salt thereof according to any one of claims 1 to 10, or the pharmaceutical composition according to claim 18 in preparing a medicament for the inhibition of SOS1.

20. Use of the compound of general formula (I) or the tautomer, mesomer, racemate, enantiomer or diastereomer thereof or the mixture thereof, or the pharmaceutically acceptable salt thereof according to any one of claims 1 to 10, or the pharmaceutical composition according to claim 18 in preparing a medicament for the treatment and/or prevention of a cancer, an inflammation, an RAS disease, Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome, hereditary gingival fibromatosis, or other proliferative diseases, preferably in preparing a medicament for the treatment and/or prevention of a cancer.

21. The use according to claim 20, wherein the cancer is selected from the group consisting of melanoma, skin cancer, liver cancer, kidney cancer, lung cancer, nasopharyngeal cancer, stomach cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, brain tumor, myeloma, astrocytoma, glioblastoma and glioma; wherein the liver cancer is preferably hepatocellular carcinoma, the head and neck tumor is preferably head and neck squamous cell carcinoma, the sarcoma is preferably osteosarcoma, and the colorectal cancer is preferably colon cancer or rectal cancer.
